# EUROPEAN PATENT SPECIFICATION

(11) **EP 2 960 341 B1**
(45) Date of publication and mention of the grant of the patent: **27.06.2018**
(21) Application number: 15000987.6
(22) Date of filing: 28.11.2008
(51) Int. Cl.: C12Q 1/68, G01N 33/50, G01N 33/74, G01N 33/574

(54) **NOVEL TISSUE PROTECTIVE ERYTHROPOIETIN RECEPTOR (NEPOR) AND METHODS OF USE**
NEUARTIGER GEWEBESCHÜTZENDER ERYTHROPOIETIN-REZEPTOR (NEPOR) UND VERFAHREN ZUR VERWENDUNG
NOUVEAU RÉCEPTEUR D'ERYTHROPOIETINE PROTECTEUR DE TISSU (NEPOR) ET PROCÉDÉS D'UTILISATION

(30) Priority: 29.11.2007 US 991042 P
(43) Date of publication of application: 30.12.2015
(62) Divisional of application: 11004689.3
(73) Proprietor: Molecular Health GmbH, 69115 Heidelberg (DE)
(72) Inventor: JACKSON, David B., 69117 Heidelberg (DE); STEIN, Martin, 68307 Mannheim (DE); VOSS, Hartmut, 69198 Schriesheim (DE); BROCK, Stephan, 69469 Weinheim (DE)
(74) Representative: Lahrtz, Fritz

(56) References cited:
- WO-A2-2004/080418
- WO-A2-2006/034456
- XIA GUANGBIN ET AL: "Up-regulation of EphB4 in mesothelioma and its biological significance.", CLINICAL CANCER RESEARCH : AN OFFICIAL JOURNAL OF THE AMERICAN ASSOCIATION FOR CANCER RESEARCH 15 JUN 2005 LNKD- PUBMED:15958611, vol. 11, no. 12, 15 June 2005 (2005-06-15) , pages 4305-4315, XP055033638, ISSN: 1078-0432
- HEROULT M ET AL: "Eph receptor and ephrin ligand-mediated interactions during angiogenesis and tumor progression", EXPERIMENTAL CELL RESEARCH, ACADEMIC PRESS, US, vol. 312, no. 5, 10 March 2006 (2006-03-10), pages 642-650, XP024944931, ISSN: 0014-4827 [retrieved on 2006-03-10]
- ACS GEZA ET AL: "Prognostic significance of erythropoietin expression in human endometrial carcinoma", CANCER, vol. 100, no. 11, 1 June 2004 (2004-06-01) , pages 2376-2386, XP002517815, ISSN: 0008-543X
- HENKE MICHAEL ET AL: "Do erythropoietin receptors on cancer cells explain unexpected clinical findings?", JOURNAL OF CLINICAL ONCOLOGY, AMERICAN SOCIETY OF CLINICAL ONCOLOGY, US, vol. 24, no. 29, 10 October 2006 (2006-10-10), pages 4708-4713, XP002543703, ISSN: 0732-183X, DOI: 10.1200/JCO.2006.06.2737

## Description

### Technical Field

The present disclosure provides a novel tissue protective erythropoietin (EPO) binding receptor protein complex, here termed NEPOR. NEPOR is proposed to mediate EPO's tissue protective properties. Thus, presence of NEPOR on a tumour allows EPO to impinge on the survival of associated cells thereby enhancing tumour progression and negatively effecting patient survival. In this circumstance, presence of NEPOR represents a prognostic biomarker for poorer patient outcome, while also representing a unique clinically tractable anti-cancer target protein, Likewise, NEPOR mediates EPO's neuroprotective activity and thus represents a unique target for treatment of diseases associated with neuronal insults, such as stroke.

In the first instance, the present disclosure provides a method for assessing a tumour for expression of this protein. The present disclosure further provides a method to stratify patients having a tumour as suitable (*i.e*. NEPOR not present) or non-suitable (*i.e*., NEPOR present) for EPO treatment. The method disclosed comprises: (a) isolating a tissue sample from an individual who is receiving or is a candidate for receiving erythropoietin, (b) determining the level of expression of the NEPOR gene(s) (mRNA) and/or the presence of the NEPOR gene product (protein) from the isolated tissue, and (c) correlating the presence of an NEPOR gene expression product or the presence of NEPOR protein to a physiological response to the treatment with erythropoietin. In a second instance, the present disclosure provides a method for treating patients with NEPOR positive tumors. Furthermore, the present disclosure provides a method for treating stroke. Finally, the present disclosure provides a method for screening for NEPOR directed therapeutics (both antagonistic therapeutics for cancer, and agonistic therapeutics for treatment of hypoxia associated tissue damage, such as stroke).

### Background

Approved by the FDA in 1993 for treatment of anemia, Erythropoietin (EPO) is a 193 amino acid glycoprotein hormone, produced by the kidneys to regulate red blood cell (RBC) production; a process commonly termed erythropoiesis. EPO was originally identified as a cytokine that promotes erythrocyte progenitor survival and differentiation, but has also been shown to possess neuroprotective functions, particularly in response to ischemic injury in the central nervous system, (CNS). Clinical use of EPO has been prevalent in the treatment of anemic cancer patients, while ongoing studies are exploring EPO's potential in the treatment of neurological diseases (*e.g*. stroke). Notwithstanding, recent clinical studies in cancer patients have begun to uncover highly orrying adverse events, suggesting that administration of recombinant human EPO (rHuEPO) can adversely effect overall patient survival. An urgent need thus exists in medical oncology to better understand and predict the prevalence or susceptibility to this effect, so that administration of rHuEPO can be contra-indicated, continued or stopped.

### EPO: Biological function

Erythropoietin (EPO) is a 193 amino acid type I cytokine, produced by cells of the renal cortex to regulate red blood cell (RBC) production in a process termed erythropoiesis. Erythropoiesis is multistage in nature, involving the differentiation of pluripotent hematopoietic stem cells through the lineage-committed burst-forming unit-erythroid (BFU-E) and colony-forming unit-erythroid (CFU-E) progenitor cells, which give rise to a series of early and late erythroblasts, eventually leading to the formation of reticulocytes and mature erythrocytes. During this process, the sequential formation of pro-erythroblasts, basophilic, polychromatophilic, and orthochromatic erythroblasts is positively regulated by EPO. EPO induces multiple positive effects on early erythroblasts, including increased proliferation, progression through maturation, and protection from programmed cell death.

In terms of molecular mechanism, EPO binds to two identical receptors (EpoR), an event which activates several intracellular signaling pathways. These include Janus kinase 2-signal transducer and activator of transcription 5 (JAK2-STAT5), phosphatidylinositol 3-kinase (PI3K), protein kinase C (PKC), and Ras-Raf-MEK (mitogen-activated or extracellular signal-regulated protein kinase kinase)-ERK (extracellular signal-regulated protein kinase). The JAK2-STAT5 and RAS-RAF-MEK-ERK pathways are thought to be associated with Epo's mitogenic action, while the PI3K pathway, acting through Akt (PI3K-Akt), is viewed as a mediator of EPO's anti-apoptotic activities.

### EPO: Clinical use

Anemia (AmE) or anæmia/anaemia (BrE), from the Greek ('vα 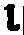 µíα)(an-haîma) meaning "without blood", is a deficiency of red blood cells (RBCs) and/or hemoglobin. The condition is commonly observed in patients with chronic diseases, and is particularly common in cancer where about 50% of patients are anaemic at presentation and some 70-90% developing the condition during the course of treatment (typically termed chemotherapy induced anemia (CIA)). In a recent review of the European Cancer Anemia Survey (ECAS), Ludwig et al. cited a 50% baseline anemia rate (hemoglobin [Hb] < 12 g/dL) among 3010 patients with hematological malignancies and a 41% baseline anemia rate among 11,453 patients with solid tumours (*Blood,* 2002;100:234a-235a. Abstract 884). Further longitudinal analysis revealed that 72% of 2780 patients with haematologic malignancies and 66% of 10,067 patients with solid tumours succumbed to CIA. Other published studies have reported varying high rates in patients at different phases and with different types of treatment (Table 1). Notwithstanding, all studies demonstrate the extremely high prevalence of anemia amongst cancer patients,

**Table 1. Prevalence of Anemia in Cancer Patients Undergoing Treatmen**

| **Type of Cancer** | **Prevalence of Anemia (Hb < 12 g/dL)** |
|---|---|
| Cervical cancer^{[3]} | 82% |
| Solid tumors^{[1]} | 66% |
| Colorectal cancer^{[3]} | 67% |
| Lung cancer^{[3]} | 63% |
| Haematological malignancies | 72% |

A number of factors contribute to the high incidence of anemia among cancer patients, including not only chemotherapy and radiation-induced myelosuppression, but also cytokine-mediated anemia of chronic disease, bleeding, marrow infiltration by tumour, hemolysis, and nutritional deficiencies. Whatever the source, anemia results in a reduced ability of blood to transfer oxygen to the tissues, leading to tissue hypoxia and an associated range of clinical consequences, affecting all realms of patient health: physiologic status, psychosocial well-being and quality of life. Not surprising, anemia can negatively affect a patient's response to cancer therapy, a fact which highlights the important supportive role of rHuEPO in restoring normal RBC counts.

### EPO: Clinical Safety signals

ESA's were for many years considered to be extremely safe in their labelled indications of chronic kidney disease and chemotherapy-induced anemia. The first hints of safety issues came in 2003 when results from a pair of studies examining EPO's potentiation of radiation and chemotherapy prompted an FDA meeting in May 2004. This first study (the ENHANCE study: Lancet 2003;362:1255-1260) suggested the relative risk of progression-free survival was worse for patients who received radiotherapy plus NeoRecormon epoetin beta from Roche than for patients receiving placebo plus radiotherapy. A randomized, double-blind, multi-institutional trial that included a study population of 351 patients who were receiving radiotherapy was performed. The patients were treated 3 times per week with either placebo or EPO in the form of epoetin beta starting 10 to 14 days before and continuing through radiation therapy. Although haemoglobin levels increased in 82% of patients receiving EPO, compared with 15% in patients receiving placebo, the rate of loco-regional progression-free survival was significantly lower. In addition, the EPO group had a higher relative risk for loco-regional progression and death.

In the second trial involving 939 breast cancer patients receiving chemotherapy (the BEST study: J. Clin. Oncol. 2005;23:5960-5972; see table 2), those given Eprex epoetin alfa from Johnson & Johnson had a higher 4-month mortality rate and a lower 12-month survival rate than those on placebo. Both studies attempted to push the limits of hemoglobin levels beyond that permitted for marketing by the FDA - the recommended haemoglobin target for Aranesp was at the time up to 12 g/dL, while the labels for Epogen and Procrit recommended 10-12 g/dL. Henke treated men to target levels of at least 15 g/dL, while women were treated to at least 14 g/dL. The target level in the BEST study was 12-14 g/dL.

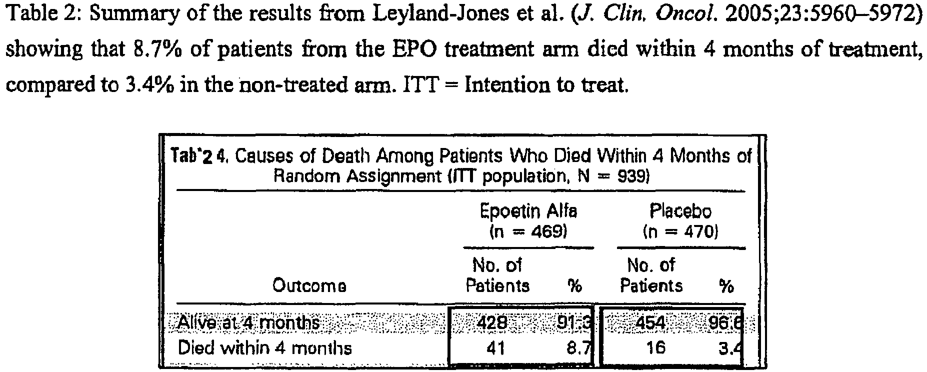

Johnson & Johnson (JNJ, New Brunswick, N.J.) have since reported data from the Phase IV CHOIR trial (N. Engl. J. Med. 2006 Nov 16;355(20):2085-98.) that tested whether using Procrit epoetin alfa to get hemoglobin levels to 13.5 g/dL would improve outcomes vs. treating to 11.3 g/dL (within the 10-12 g/dL range on the drug's label). Patients in the higher haemoglobin group had a significantly increased incidence of mortality and cardiovascular events. While this study was carried out in the renal disease space, the safety implications were further emphasized in a more recent study - DAHANCA10. In February 2007, Amgen disclosed that this independent study had been halted three months earlier after interim data showed that Aranesp plus radiation missed the primary endpoint of 3-year loco-regional control vs. radiation alone. The study also showed a non-significant increase in death in the Aranesp ann. DAHANCA10 explored whether the use of Aranesp to maintain a hemoglobin level of 14-15.5 g/dL during radiotherapy could improve loco-regional disease control in patients with primary head and neck squamous cell carcinoma (HNSCC).

Safety signals also emerged from the use of Aranesp in the AoC space (study 103). In January 2007, Amgen reported that the risk/benefit profile of Aranesp was "at best neutral" in a Phase III trial in patients who had AoC and who were not receiving chemo- or radio-therapy. Here the data revealed significantly more deaths in Aranesp patients than in placebo patients. The trial, which treated patients to a haemoglobin level of 12-13 g/dL, also missed its primary endpoint of a significant reduction in transfusion frequency at 16 weeks. Study 103 enrolled patients with various cancers, including non-small cell lung cancer (NSCLC), breast cancer and prostate cancer. Canadian researchers have published similar findings (J. Clin. Oncol. 2007 Mar 20; 25(9):1027-32). Here the authors showed that of the 70 advanced NSCLC patients with AoC, those receiving Procrit, had a significantly higher mortality rate than those receiving placebo. A synopsis of each of these studies is provided in Table 3 below:

**Table 3: Summary of results from EPO safety studies highlighting survival issues.**

| **STUDY** | **EPO type** | **POPULATION** | **DESIGN** | **STATUS** |
|---|---|---|---|---|
| **DAHANCA (**SE20029001**)** | ***Aranesp*** | HNSCC; Baseline Hb <= 14.5 | Multicenter, open-label trial of radiotherapy +/- Aranesp | Terminated early by DMC (after 522 of 600 planned patients enrolled) based on lower LRC rates and Increased deaths In the ESA am at planned Interlm analysis: 522 of 600 planned pts; summary results 12/06; CSR anticipated 9/08 |
| **EPO-CAN-20** | ***Eprex*/*Procrit*** | NSCLC not receiving chemo; baseline Hb <=12 | Double-bllnd, placebo controlled, randomized (1:1) +/- Eprex | Terminated early by OSMB for Increased deaths In ESA am; 70 of 300 patients enrolled; results published In abstract in 2004 and In the Journal of clinical oncology 3/07 |
| **BEST (EPO-INT-76)** | ***Eprex*/*Procrit*** | Metastatic breast cancer | Randomised, double blind, placebo controlled | Terminated In April 2002, after review of data In the first 938 pts by the DMC, due to evidence of excess mortality In the Eprex arm |
| **RTOG 9903** | ***Eprex*/*Procrit*** | HNSCC; baseline Hb 9-12.5 (female), 9-13.5 (male) | Open-label, randomized (1:1). chemo/radiation +/procrit | Terminated early by DSMB for trend to poorer of OS in EPO am. 14B of 372 patients enrolled. Results published in abstract 2004 |
| **Study 103 (Amgen)** | ***Aranesp*** | NSCLC, prostate, breast cancer | | |

### EPO clinical safety; a role for EPOR?

These clinical findings have led many investigators to suggest a possible role for ESA's in promoting tumour growth through stimulation of EPO receptor survival signalling in tumour cells, and via the stimulation of angiogenesis. Implicit in these proposed activities is the notion that the EPO receptor can somehow confer survival advantage to cancer cells, a negative side effect. This, in turn, suggests that EPO receptor is both present and activated by EPO binding in such cells. Using real-time, quantitative RT-PCR, the EPOR gene has not only been shown to be strongly expressed in bone marrow (containing the EPO-responsive erythroid progenitors), but also at significant levels in normal tissues (*e.g.* kidney, heart, brain, endothelium, and smooth muscle). Moreover, EPOR transcript levels in breast, colon, ovary, prostate, lung, lymphoma, ileum, stomach, and kidney tumour tissues and tumour cell lines were no higher than those levels observed in normal tissue counterparts. These findings are in concordance other reports which demonstrated that EPOR transcript levels are basically equivalent in matched tumour and non-tumour samples from patients with lung, colon and prostate cancer. From the perspective of these data, it is questionable whether the EPOR gene might somehow provide selective advantage to tumour cells, at least via abnormal expression levels.

Therefore, there is a possible role for EPOR in mediating tumour cell survival in response to EPO. From a molecular perspective, the ability of cancer cells to subvert the EPO/EPOR system would not be surprising. A number of preclinical studies have demonstrated EPO-mediated activation of the mitogen-activated protein kinase (MAPK), phosphatidylinositol 3-kinase (PI₃K)-Akt, JAK-STAT (Janus kinase-Signal Transducer and Activator of Transcription), and nuclear factor-kappa B (NFκB) signalling pathways in a variety of human cancers. Each of these signalling cascades has been associated with cellular functions that promote tumour progression. EPO stimulated not only chemotaxis of endothelial cells, together with migration and invasion of breast cancer and HNSCC cells, but also appears to induce cancer cell proliferation and inhibit apoptosis. Moreover, pretreatment with rHuEPO protects some cancer cell lines from the cytotoxic effects of the chemotherapeutic agent, cisplatin. Thus, EPO/EPOR signalling appears to contribute to a wide variety of tumour-promoting functions in different cancer types.

Despite this evidence, the possible contribution of EPO/EPOR signalling to cancer progression is anything but straightforward. The influence of EPO/EPOR on different cancer types appears to be quite variable and remains incompletely understood. Studies have shown that EPO does not influence the proliferation of cancer cell lines. Rosti et al. (Haematologica 1993 Jul-Aug; 78(4):208-12.), for example, investigated the proliferative potential of rHuEPO by testing the effects of this factor on clonogenic growth and DNA synthesis in 10 different cell lines derived from haematologic malignancies and solid tumours. The cell lines K-562 and HEL were included in this study, both of which express EPO receptors. Results showed that rHuEPO had no effect on either colony growth or DNA synthesis (see Table 4).

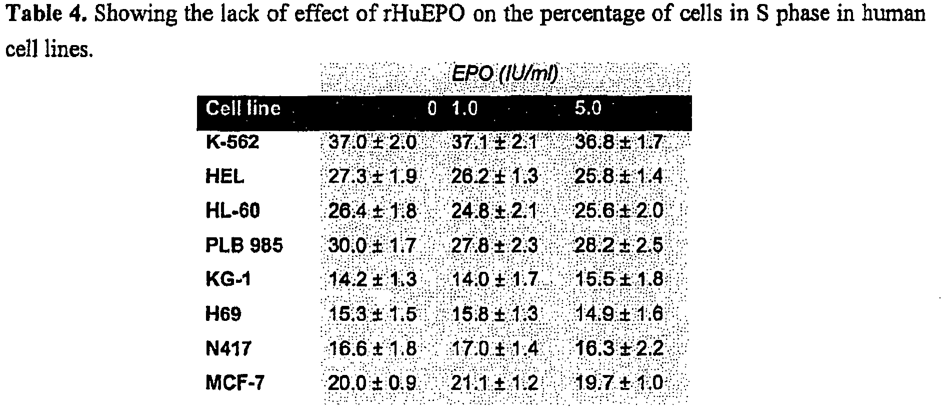

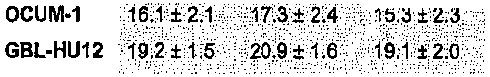

In a similar study, Westphal et al. (Tumori 2002 Mar-Apr; 88(2):150-9.) investigated the effects of EPO on more than 25 different benign and malignant human cell lines. Expression of EPO receptor mRNA and protein was analyzed with RT-PCR, Western blot, and immunocytochemistry. Cellular responses to various concentrations of EPO were evaluated using tritiated thymidine uptake, Northern blot analysis of c-fos expression, and tyrosine-kinase activity assay. EPO receptor mRNA and protein were identified in the majority of the tumour cell lines evaluated. Despite these findings, treatment with rHuEPO did not significantly influence the proliferation rate of EPO-receptor-positive tumour cell lines. Moreover, treatment with EPO neither affected the gene c-*fos* mRNA of those cell lines nor stimulated tyrosine-kinase activation. Based on their findings, the authors concluded that expression of the EPO receptor in tumour cells does not appear to be essential for growth and therefore should not have a deleterious effect in cancer patients.

### Might a novel EPO receptor explain these clinical and molecular findings?

Results by Lu et al. (J. Biol. Chem., Vol. 281, Issue 11, 7002-7011, 2006) establish that receptor activation is not simply accomplished by bringing two receptors into close proximity through disulfide linkages in the transmembrane or extracellular domains. Instead, the relative orientation of the two transmembrane domains of an EpoR dimer, rather than their proximity, determines the extent of receptor activation. More specifically, these authors propose that Epo binding to the inactive, symmetric EpoR dimer causes the repositioning of the two fibronectinIII domains to an asymmetric 120° relative orientation, which in turn changes the orientation of the transmembrane domains and intracellular domains, and juxtaposes the appended JAK2s to initiate the phosphorylation cascade, EPO mutants would not necessarily be expected to be capable of initiating EPOR-signalling, due to their inability to induce the correct relative conformation of the fibronectinIII domains. Interestingly, it appears that certain aspects of EPO function can be decoupled from EPOR activity. Leist et al. (Science 305, 239-242.) have shown that the haematopoietic and tissue-protective activities of Epo are distinct and separate, demonstrating for example that carbamylated Epo (CEpo) does not stimulate erythropoiesis, yet prevents tissue injury in a wide variety *of in vivo* and *in vitro* models.

### EPO and neuronal protection:

EPO's efficacy in treating nervous system disease has been demonstrated in several experimental models of brain and spinal cord injury. As such, EPO has become a candidate for therapeutic neuro-protection. Notwithstanding, the use of EPO as a neuro-protectant raises several safety issues. Although recombinant EPO seems to be potentially safe at neuroprotective proven doses, cardiovascular or cerebrovascular events can occur as a result of its bone marrow stimulating activities. Interestingly, as highlighted above, EPO's neuronal protective function appears molecularly separable from the haematopoietic activity, as carbamylated EPO and certain EPO mutants are neuroprotective but fail to induce haematopoiesis. Such mutants fail to bind EPOR (Leist et al. Science 305, 239-242).

### Synopsis:

EPO was for a long time considered to act solely on haematopoietic cells, a fact which led to its emergence as a leading treatment for chemotherapy-induced anemia. However, emerging evidence has shown that EPO is expressed in a variety of tissue and cell types, including cancer, vascular endothelial, and neuronal cells. Expression of EPO is induced in response to hypoxia, an event mediated by the HIF-1 transcription factor. EPO is prototypically thought to exert its biological effects via binding to its cell surface receptor EPOR, resulting in tyrosine phosphorylation of the receptor and other intracellular proteins, including JAK2 and STAT5. The JAK/STAT pathway is utilized both in haematopoietic and non-haematopoietic cells (including brain cells) following binding of EPO to the EPO receptor. The recent findings of EPO-receptor expression in human breast and renal cancer cells, as well as in several tumour cell lines, have raised important questions in the oncology setting about a possible tumour-growth-promoting effect of rHuEPO on EPO-receptor-bearing tumours. This possibility has been borne out in several clinical trials. Interestingly, other studies have shown that certain EPO mutants which are cytoprotective but not longer able to induce haematopoiesis, function independently of EPOR. This suggests that another EPO receptor may exist which lacks EPOR's strict binding conformation requirements.

In view of the disclosure provided herein and its related discovery, a brief description of ephrin receptors and ephrin biology is provided as follows.

### Ephrin and Ephrin Receptor Biology

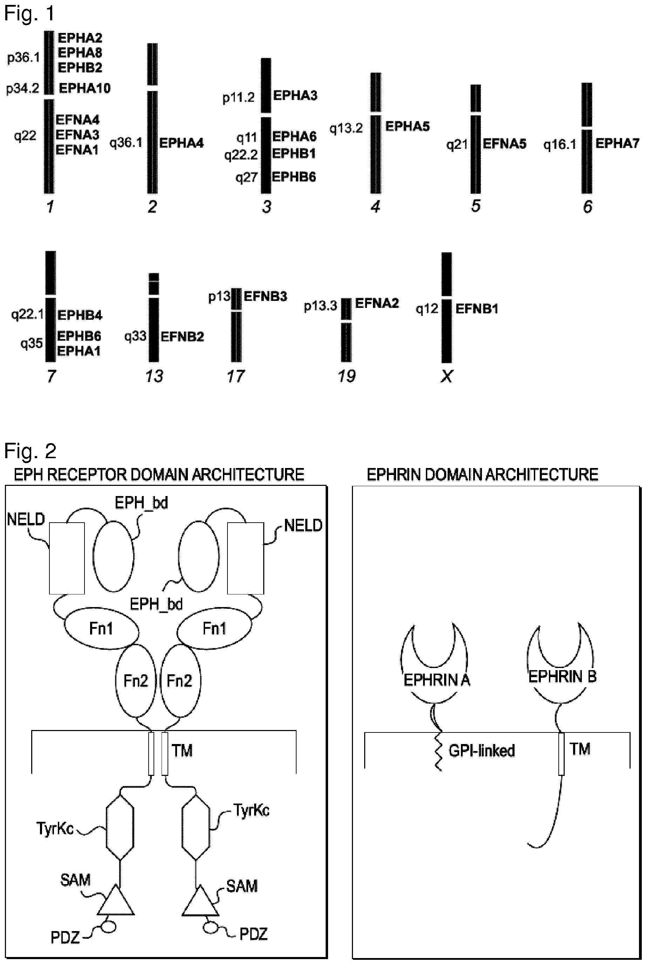

Erythropoietin-producing hepatocellular carcinoma (Eph) receptors form the largest family of receptor tyrosine kinases. Eph receptors are divided into two groups (Eph-A's and Eph-B's) based on the similarity of their extracellular domain sequences and the distinct structural properties of the ephrin ligands (Eph Nomenclature Committee, 1997). About 16 ephrin receptor genes (EphA1-10, EphB1-6) have been identified in the vertebrate genome (Pasquale, Nat. Rev., Mol. Cell Biol. 6 (2005), pp. 462-475.), 14 of which are present in humans (Figure 1) and other mammals (EphA1-8, EphA10, EphB1-4, EphB6).

Eph receptors are single-pass transmembrane proteins with highly conserved extracellular and intracellular domains. The former domains consists of an N-terminal ligand binding domain, a cysteine-rich EGF-like region and two fibronectin type III repeats (Yamaguchi and Pasquale, Curr. Opin. Neurobiol. 14 (2004), pp. 288-296.). Intracellularly, the juxtamembrane region is followed by a tyrosine kinase domain, followed by a sterile-α-motif (SAM), and a type-II PSD-95/Disc large/ZO-1 (PDZ) binding motif at the carboxyl terminus (Kullander and Klein, Nat. Rev., Mol. Cell Biol. 3 (2002), pp. 475-486.). The tyrosine kinase domain of one receptor from each class (EphA10 and EphB6) lacks residues that are essential for catalytic activity. Eph receptor variants are generated by alternative splicing and their structures differ from the prototypical domain structure. The domain architecture of Eph receptors and Ephrins (A and B subclasses) are shown in Figure 2.

Eph receptors can undergo cis-oriented homo- as well as heterodimerization (Freywald et al., J. Biol. Chem. 277 (2002), pp. 3823-3828.), which is mediated directly by the extracellular cysteine-rich region, the fibronectin type III repeats (Lackmann et al., J. Biol. Chem. 273 (1998), pp. 20228-20237.) and the SAM motif (Stapleton et al., Nat. Struct. Biol. 6 (1999), pp. 44-49. and Thanos et al., Science 283 (1999), pp. 833-836.) or indirectly through PDZ protein interactions (Fanning and Anderson, J. Clin. Invest. 103 (1999), pp. 767-772). *Trans*-oriented interactions typically occur with select ephrin molecules on opposing cells. In common with their receptors, the ephrins (named derived from Eph family receptor interacting proteins or *ephoros)* are divided into two distinct subclasses A and B. Ephrin-A ligands are GPI-anchored peripheral membrane molecules. In contrast, ephrin-B ligands are transmembrane molecules whose short cytoplasmic domain is capable of participating in various signalling events. The ephrin-A and ephrin-B molecules were initially described as selectively interacting with EphA and EphB receptors, respectively. However, there may be crosstalk between A and B family members. For example, ephrin-A5 is capable of binding EphB2, while EphA4 binds to ephrin-A and ephrin-B family members. Although interactions across classes are limited, within a class they are promiscuous, with multiple EphA receptors binding to a given ephrinA and vice versa

While neither class of ephrins possesses a catalytic activity, both can activate signal transduction pathways after interaction with Eph receptors (reverse signalling). Reverse signalling activated by transmembrane ephrins includes tyrosine phosphorylation of their cytoplasmic tail and interaction with various signalling molecules. The mechanism by which GPI-linked ephrins stimulate downstream signalling is still unclear.

Signalling sometimes involves formation of signalling assemblies, a process that begins with a monovalent interaction (nanomolar affinity) between an Eph receptor and an ephrin on a juxtaposed cell. Crystallographic work has shown that the globular ephrin-binding domain of EphB2 contains a cavity that accommodates a hydrophobic protrusion from the ephrins. Structural changes occur upon binding. For example, EphB2 undergoes different structural rearrangements upon binding to ephrin-B2 or ephrin-A5.

A lower affinity binding interface is also present on the opposite side of the EphB2 ligand binding domain (Eph_1b), with complementary interfaces also present in the Eph-receptor-binding domain of ephrin-B2. While only of micromolar binding affinity, the second interface can mediate the dimerization of two Eph-ephrin dimers into a tetramer that comprises two receptor and two ephrin molecules extending from adjacent cell surfaces. The lower-affinity interface contains important determinants of subclass specificity and is not engaged in the EphB2-ephrin-A5 complex.

Signalling is initiated upon transphosphorylation via correctly orientated kinase domains. Eph receptors become extensively phosphorylated upon activation by ephrins and via src-kinase association. Phosphorylation promotes conformational order on the activation segment of the kinase domain that favours substrate binding and also disrupts intra-molecular inhibitory interactions that occur between the juxtamembrane segment and the kinase domain. Src-family mediated phosphorylation of Eph receptors has also been shown to act in a similar manner.

### Summary

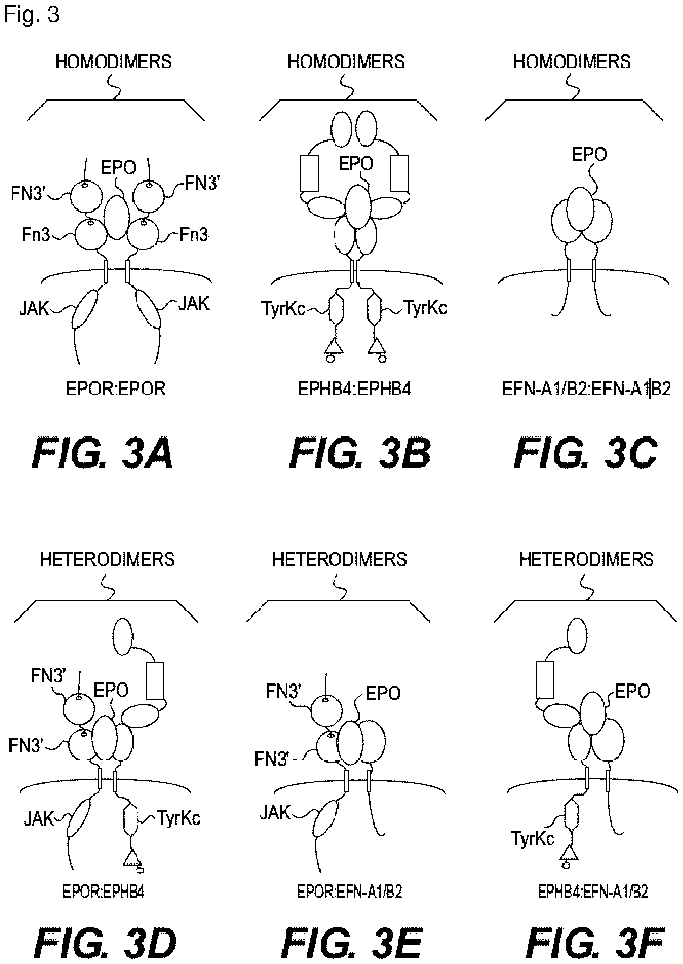

The present invention discloses members of the ephrin family (ephrinA1 and EPH-B4) as mediators of cytoprotective EPO signalling, either as homodimers and/or as heterodimeric partners of EPOR and/or each other. Our data emphasize the importance of EPH-B4 and EphrinA1 in mediating this function. As such, NEPOR represents a novel EPO receptor derived from a unique combination *(i.e.* via homo- and/or hetero-dimerization) of components derived from ephrin biology and possibly the EPO receptor. See figure 3 for summary.

The present disclosure is based upon the data that EPH-B4 and EphrinA1 are the components of a novel EPO receptor (NEPOR). We are able to show that EPO stimulates enhanced tumor growth in a mouse tumor model system. EPO stimulates the Akt signalling pathway in cell lines lacking EPO receptor expression. These cells express EPH-B4 which is a receptor that stimulates signalling via the Akt pathway. Furthermore in a mouse tumor model it can be shown that EPO is capable of stimulating significant tumor growth. Such activity is inhibited via kno-down of the EPH-B4 receptor highlighting the EPH-B4 dependant nature of a EPO mediated tumor genesis. As such, NEPOR is primarily composed of EPH-B4 as a homodimer and/or in heterodimeric association with EPOR or an Ephrin. Furthermore, *in silico* analyses points to structural complementarity between EPO and Ephrin molecules, particularly Ephrin A1. Thus, NEPOR may also be composed of EphrinA1 as a homodimer and/or in heterodimeric association EPH-B4. A summary of these putative NEPOR species is provided in Figure 3 and Table 5.

The present disclosure provides a method for assessing a tissue for expression of the tissue protective NEPOR receptor complex and/or EPH-B4 and/or Ephrin A1. In so doing, the present disclosure provides a prognostic method to stratify patients having a tumour as suitable (NEPOR not present on the tumour; NEPOR-) or non-suitable (NEPOR present on the tumour; NEPOR+) for EPO treatment. Specifically, the method for assessing tumour tissue NEPOR and/or gene expression components comprises:
(a) isolating a tissue sample from an individual who is receiving or shall receive erythropoietin,
(b) determining the level of expression of the NEPOR gene transcript(s) (*i.e.* EPH-B4, and/or Ephrin A1 mRNA) and/or the presence of the NEPOR gene products (*i.e*. EPH-B4, and/or Ephrin A1 proteins) from the isolated tissue, and
(c) correlating the presence of these NEPOR component gene expression products to a negative physiological response to the treatment with erythropoietin.

Preferably, the expression of the NEPOR component genes (*i.e*. EPH-B4, and/or Ephrin A1 mRNA) is determined by a molecular biological technique selected from the group consisting of PCR, QPCR, R-PCR, gene expression microarray analysis, northern-blot analysis, reverse transcription and amplification, zymography, ligase-chain-reaction, NASBA, RNase Protection Assay (RPA), capillary electrophoresis with laser induced fluorescence (CE-LIF) and combinations thereof.

Preferably, the determination of the presence of the NEPOR gene products is done by detecting the respective proteins with an immunoassay procedure, where the immunoassay procedure is selected from the group of immunoprecipitation, enzyme immunoassay (EIA), radioimmunoassay (RIA) or fluorescent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a luciferase-assay. The immunoassay procedure is most preferably based on ELISA.

Preferably, the method for detection of NEPOR and/or EPH-B4, and/or Ephrin A1 on tumour tissue can also be an *in situ* imaging method, comprising administering an anti-NEPOR antibody or NEPOR binding peptide linked to a radio-ligand or other imaging agent, and measuring for tissue distribution and location of the radio-ligand or other imaging agent. Preferably, the tissue sample is selected from the cancerous tissue or circulating cells derived from same, or from a group of biological tissues and fluids such as blood, lymph, urine, cerebral fluid. Specifically, the individual is a cancer patient who is to be treated with erythropoietin or is being treated with erythropoietin. Preferably, the negative physiological effect is increased tumor progression and/or poorer patient survival. Preferably, the presence of NEPOR gene products and/or EPH-B4, and/or Ephrin A1 is indicative of increased tumor progression and/or poorer patient survival upon treatment with erythropoietin. Preferably the cancer is one of head and neck cancer, breast cancer, liver cancer, colorectal cancer, small intestine cancer, leukemia, prostate cancer, lung cancer, ovarian cancer, pancreatic cancer, endometrial cancer, stomach cancer, non-Hodgkin lymphoma, kidney cancer, Renal cell carcinoma (RCC), malignant melanoma, gallbladder cancer, bladder cancer, vulvar cancer, Penile cancer, testicular cancer, thymus cancer, Kaposi's sarcoma, eye cancer, adrenal gland cancer, brain cancer, cervical cancer, appendix cancer, adenoid cancer, bile duct cancer, urethral cancer, spinal cancer, Ewing's family of tumors, extragonal germ cell cancer, extra hepatic bile duct cancer, fallopian tube cancer, soft tissue cancers, bone cancer, Hodgkin's lymphoma, anal cancer, malignant mesothelioma, vaginal cancer skin cancer, central nervous system cancer (craniopharyngioma), pleuropulmonary blastoma, nasal cavity and paranasal sinus cancer transitional cell cancer of renal pelvis and ureter, pituitary gland cancer, sqamous cell carcinoma of the head and neck (HNSCC), prostate cancer, colorectal cancer, lung cancer, brain cancer, bladder cancer, and salivary gland cancer. It is particularly preferred that the cancer is selected from the group of squamous cell carcinoma of the head and neck (HNSCC), prostate cancer, colorectal cancer, lung cancer, kidney cancer, brain cancer, bladder cancer and breast cancer.

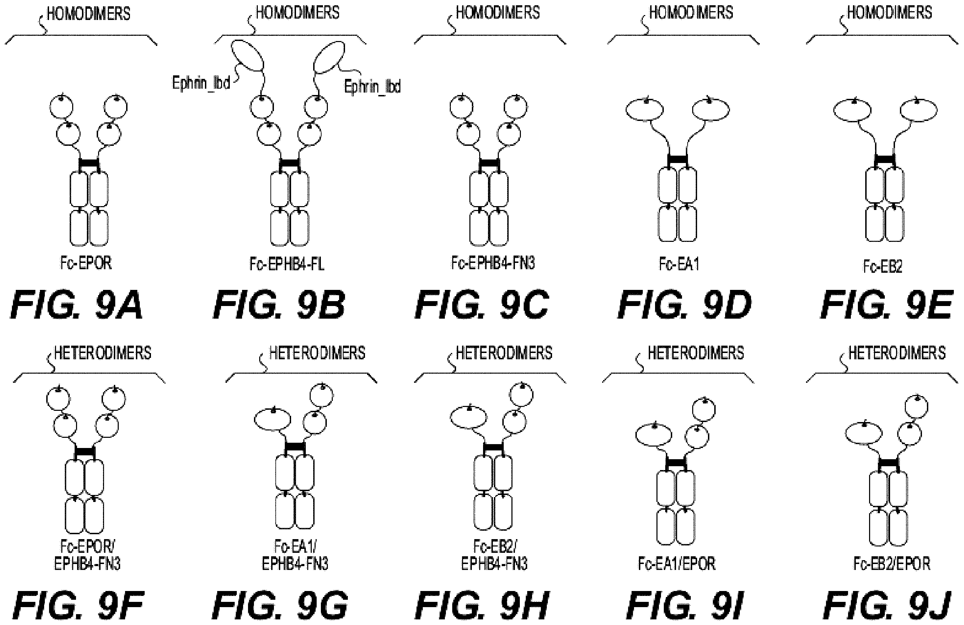

The present disclosure further provides a method for designing a therapy which modulates the activity of NEPOR and/or EPH-B4, and/or Ephrin A1, comprising:
1) performing an *in vitro* screening assay for NEPOR and/or EPH-B4, and/or Ephrin A1 specific therapies; by measuring the binding of test compounds to a tissue protective NEPOR receptor complex and/or EPH-B4, and/or Ephrin A1 (also in comparison to EPOR homodimer complexes), wherein the test compound is labelled (binding of the labelled test compound to the receptor complexes detailed in figure 9) and is measured by detecting the label attached to the test compound;
2) performing a label-free screening approach such as surface plasmon resonance. In this case the test compound is not labelled and its binding to NEPOR receptor complexes (as detailed in figure 9) is measured by a label independent (optical) method.
3) testing NEPOR and/or EPH-B4, and/or Ephrin A1 activity by (a) contacting a test compound with a tissue protective NEPOR receptor complex (N) or tissue protective NEPOR receptor complex-expressing cell; measuring the level of the activity of (N) in the cell; identifying a test compound that increases or decreases the level of activity of (N) as compared to the level of activity of (N) measured in the absence of the test compound; and assaying the identified test compound for tissue protective activity;
4) testing the modulation of NEPOR/ligand binding and/or EPH-B4, and/or Ephrin A1 ligand binding by (a) contacting (N) with a tissue protective NEPOR receptor complex ligand and/or EPH-B4, and/or Ephrin A1 ligand attached to a first label, and an equivalent amount of a test compound attached to a second label under conditions conducive to binding, removing unbound material from (N), and detecting the level of the first and second labels, where if the second label is present the compound binds (N) and if the level of the first label decreases relative to the level of the first label when the labelled ligand is contacted with (N) under conditions conducive to binding in the absence of a test compound after removal of unbound material, then a compound that binds to (N) is identified.
5) identifying a compound that modulates a tissue protective activity in a mammal, comprising: (a) administering the compound to a first animal immediately following infliction of an injury, wherein the first animal endogenously expresses a tissue protective NEPOR receptor complex; and (b) administering the compound to a second animal immediately following infliction of the same injury as in step (a), wherein the second animal is deficient in expression of a tissue protective NEPOR receptor complex and/or EPH-B4, and/or Ephrin A1 or components thereof; such that if recovery from the injury differs in the animal of step (a) as compared to the animal of step (b), a compound that modulates a tissue protective activity is identified.

The present disclosure further provides methods for treating or preventing a disease or disorder in a human comprising administering a therapeutically effective amount of a compound that modulates the activity of a tissue protective NEPOR receptor complex to a human in need of such treatment or prevention, with the proviso that the compound is not EPO. The compound is selected from the group consisting of an antibody specific for the tissue protective NEPOR receptor complex, an antibody is specific for a tissue protective NEPOR receptor complex ligand, a small molecule, a peptide, an EPO mutant, an EPO:Ephrin_ligand_binding domain chimera, a member of a library, and a combination thereof. Preferably, such compounds negatively modulate the tissue protective function of the NEPOR receptor complex in the aforementioned mentioned cancers. Preferably such compounds positively modulate the tissue protective function of the NEPOR receptor complex wherein the disease or disorder is caused by hypoxia, seizure disorders, neurodegenerative diseases, neurotoxin poisoning, multiple sclerosis, hypotension, cardiac arrest, radiation, or hypoglycemia.

The present disclosure further provides a method for identifying compounds that modulate NEPOR's tissue protective signalling activity, comprising (a) contacting a test compound with the NEPOR receptor complex expressing cell; (b) measuring the level of tissue protective activity initiated by NEPOR activation in the cell; (c) identifying a test compound which increases or decreases the level of tissue protective NEPOR complex activity in a cell; (d) assaying the identified compounds for tissue protective activity mediated via NEPOR; and (e) assaying the identified therapeutics for NEPOR inhibitory activity. Preferably, the assay in step (d) is a tissue protective NEPOR receptor complex activity is measured by a cell proliferation/differentiation assay. More preferably, the cells in the cell proliferentiation/differentiation assay are recombinantly engineered to express EPH-B4, and/or EPOR, and/or Ephrin A1. More preferably, the cells endogenously express an EPO receptor and are transformed with a nucleic acid comprising a nucleotide sequence that (i) is operably linked to a promoter, and (ii) encodes either EPH-B4 and/or Ephrin A1. Most preferably, the cells endogenously express EPH-B4 and/or Ephrin A1 and are transformed with a nucleic acid comprising a nucleotide sequence that (i) is operably linked to a promoter, and (ii) encodes an EPO receptor polypeptide.

The present disclosure further provides a method for identifying a compound that modulates the interaction between a tissue protective NEPOR receptor complex and a tissue protective NEPOR receptor complex ligand, comprising: (a) contacting a tissue protective NEPOR receptor complex with one or more test compounds; and (b) measuring the tissue protective NEPOR receptor complex activity, whereby if the activity measured in (b) differs from the tissue protective NEPOR receptor complex activity in the absence of the one or more test compounds, then a compound that modulates the interaction between the tissue protective NEPOR receptor complex and the tissue protective NEPOR receptor complex ligand is identified. Preferably, the tissue protective NEPOR receptor complex activity is measured by cell proliferation or cell differentiation. Preferably, the tissue protective NEPOR receptor complex activity measured is the ability of the tissue protective NEPOR receptor complex to interact with a tissue protective NEPOR receptor complex ligand. Preferably, the step of assaying the identified compound for tissue protective activity comprises detecting the presence of nucleolin in the cell. Preferably, the step of assaying the identified compound for tissue protective activity comprises detecting or measuring an increased level of activity of neuroglobin or cytoglobin in a cell. Preferably, the tissue protective NEPOR receptor complex is in solution. Preferably, the tissue protective NEPOR receptor complex is in a cell. Preferably, the compound inhibits the binding of a tissue protective NEPOR receptor complex ligand to a tissue protective NEPOR receptor complex. Preferably, the compound enhances the binding of a tissue protective NEPOR receptor complex ligand to a tissue protective NEPOR receptor complex. Preferably, the tissue protective NEPOR receptor complex contacted in step (a) is on a cell surface. Preferably, the tissue protective NEPOR receptor complex is on an isolated cell membrane. Preferably, the tissue protective NEPOR receptor complex activity is compared to EPOR receptor activation to identify NEPOR specific compounds. Preferably, the tissue protective NEPOR receptor complex is immobilized to a solid surface and more preferably, the solid surface is a microtiter dish or a chip.

The present disclosure further provides a method for identifying a compound that binds a tissue protective NEPOR receptor complex, comprising: (a) contacting a test compound with a ligand-binding tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support; and (b) contacting a test compound with a ligand-binding EPOR receptor complex fragment comprising at least two EPO receptor extracellular domains fused to an Fc fragment attached to a solid support (c) removing unbound test compounds from the solid supports; (d) identifying the compound attached to the tissue protective NEPOR receptor complex fragment, but not the EPOR receptor complex (and vice versa), whereby a compound bound to the solid support is identified as a compound that binds specifically to a tissue protective NEPOR receptor complex or a compound that binds specifically to an EPOR receptor complex.

The present disclosure further provides a method for identifying a compound that binds a tissue protective NEPOR receptor complex, comprising: (a) contacting a test compound with a ligand-binding tissue protective NEPOR receptor complex fragment comprising at least one EPO receptor or EPH-B4 receptor or Ephrin A1 receptor, extracellular domain fused to an Fc fragment attached to a solid support; (b) removing unbound test compounds from the solid supports; (c) identifying the compound attached to the tissue protective NEPOR receptor complex fragment, whereby a compound bound to the solid support is identified as a compound that binds specifically to a tissue protective NEPOR receptor complex.

The present disclosure further provides a method for identifying a compound that binds to a tissue protective NEPOR receptor complex, comprising: (a) contacting a tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support with (i) a tissue protective NEPOR receptor complex ligand attached to a first label and (ii) an equivalent amount of a test compound attached to a second label under conditions conducive to binding; (b) removing unbound material from the tissue protective NEPOR receptor complex; and (c) detecting the level of the first and second labels wherein if the second label is present the compound binds the complex and if the level of the first label decreases relative to the level of the first label where the labelled ligand is contacted with a tissue protective NEPOR receptor complex under conditions conducive to binding in the absence of a test compound after removal of unbound material, then a compound that binds to a tissue protective NEPOR receptor complex is identified.

The present disclosure further provides a method for identifying a compound that modulates the binding of a tissue protective NEPOR receptor complex ligand to a tissue protective NEPOR receptor complex, comprising: (a) contacting a tissue protective NEPOR receptor complex ligand with a tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support; in the presence of one or more test compounds under conditions conducive to binding; and (b) measuring the amount of tissue protective NEPOR receptor complex ligated bound to the tissue protective NEPOR receptor complex; whereby if the amount of bound tissue protective NEPOR receptor complex ligand measured in (b) differs from the amount of bound tissue protective NEPOR receptor complex ligand measured in the absence of the one or more test compounds, then a compound that modulates the binding of a tissue protective NEPOR receptor complex ligand to the tissue protective NEPOR receptor complex is identified.

Preferably, the amount of bound tissue protective NEPOR receptor complex ligand is measured using a tissue protective NEPOR receptor complex ligand-specific antibody. Preferably, the tissue protective NEPOR receptor complex ligand is labelled and binding of the tissue protective NEPOR receptor complex ligand to the tissue protective NEPOR receptor complex is measured by detecting the label attached to the tissue protective NEPOR receptor complex ligand. Preferably, the tissue protective NEPOR receptor complex ligand is labelled and binding of the labelled ligand to the tissue protective NEPOR receptor complex is measured by detecting the label attached to the tissue protective NEPOR receptor complex ligand. Preferably, the label is fluorescent. Preferably, the test compound is an antibody specific for the tissue protective NEPOR receptor complex. Preferably, the test compound is a small molecule. Preferably, the test compound is a peptide or a member of a library. Preferably, the tissue protective NEPOR receptor complex ligand is EPO, or derivatives thereof. Preferably, the compound binds the tissue protective NEPOR receptor complex or ligand thereof. Preferably, the tissue protective NEPOR receptor complex activity is compared to EPOR receptor activation to identify NEPOR specific compounds.

The present disclosure further provides a method for identifying a compound that modulates a tissue protective activity in a mammal, comprising: (a) administering the compound to a first animal immediately following infliction of an injury, wherein the first animal endogenously expresses a tissue protective NEPOR receptor complex; and (b) administering the compound to a second animal immediately following infliction of the same injury as in step (a), wherein the second animal is deficient in expression of a tissue protective NEPOR receptor complex or components thereof; such that if recovery from the injury differs in the animal of step (a) as compared to the animal of step (b), a compound that modulates a tissue protective activity is identified.

The present disclosure further provides a method for designing a compound which interferes with NEPOR's survival promoting activity, comprising:
(a) providing the molecular makeup of the NEPOR species and providing amino acid sequences of a component NEPOR polypeptides;
(b) using software comprised by the digital computer to design a chemical compound/protein construct which is predicted to bind to NEPOR; and
(c) optionally designing protein constructs which mimic NEPOR in its dimcrised/multimerised state (*e.g*. Fc constructs).

The present disclosure further provides a method for identifying compounds that modulate NEPOR's tissue protective signalling activity, comprising (a) contacting a test compound with the NEPOR receptor complex; (b) measuring the level of tissue protective activity initiated by NEPOR activation; (c) identifying a test compound which increases or decreases the level of tissue protective NEPOR complex activity; (d) assaying the identified therapeutics for tissue protective activity mediated via NEPOR; and (e) assaying the identified therapeutics for NEPOR inhibitory activity. Preferably, the tissue protective NEPOR receptor complex activity is measured by measuring the binding of the test compound to the NEPOR receptor complex. More preferably, the test compound is labelled and binding of the labelled test compound to the tissue protective NEPOR receptor complex is measured by detecting the label attached to the test compound. Most preferably, the tissue protective NEPOR receptor complex activity is measured by measuring the binding of the test compound to the tissue protective NEPOR receptor complex.

The present disclosure further provides a method for imaging tumour tissue that is susceptible to enhanced survival in response to EPO treatment, comprising administering an anti-NEPOR antibody or NEPOR binding peptide linked to a radio-ligand or other imaging agent, and measuring for tissue distribution and location of the radio-ligand or other imaging agent. Preferably, the anti-NEPOR antibody is a monoclonal or polyclonal antibody selected from the group of antibodies listed in Table 6.

The present disclosure further provides a method for modulating cell survival in NEPOR positive tissue comprising administering an EPO mutants and peptides selected from the group consisting of peptides from SEQ ID NO. 17 through SEQ ID NO. 212.

The present disclosure further provides a method for modulating cell survival in NEPOR positive tissue comprising administering an effective amount of an EPO chimera, comprising an ephrin receptor ligand binding domain selected from the group consisting of SEQ ID NO. 215, and SEQ ID NO. 216.

### Brief Description of the Figures

Figure 1 shows the genomic localization of human Eph receptor (EPH) and ephrin (EFN) genes on human chromosomes.
Figure 2 shows the domain architecture of Eph receptors and Ephrins (A and B subclasses).
Figure 3 shows a process for identifying putative EPO binding transmembrane receptors. All proteins containing two membrane proximal FN3 domains were extracted (84 in all) and assessed for evidence of response to hypoxia. EPH-B4 was amongst one of four possible proteins extracted. Moreover, it is the only member of the Ephrin receptor family which is embryonic lethal, with death in embryo's preceding that of EPOR knock-outs.

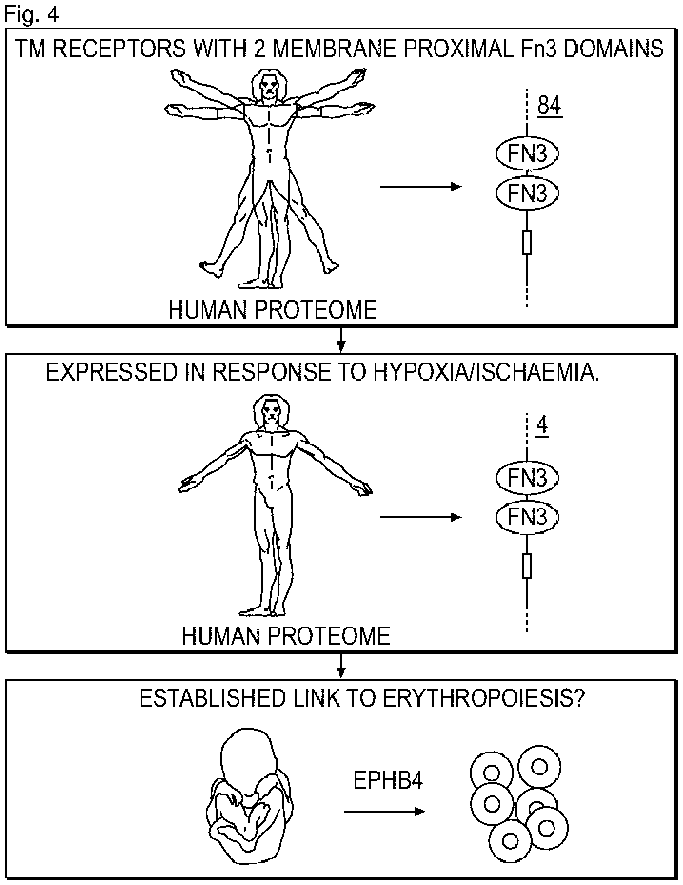

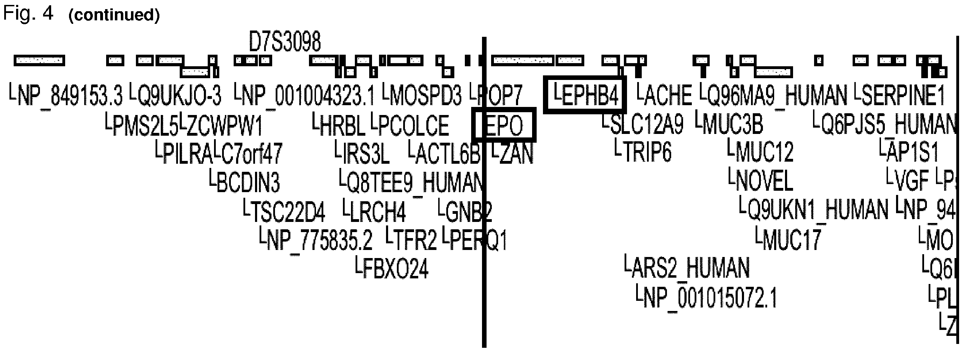

Figure 4 shows the human EPO locus showing the neighbouring EPH-B4 gene.

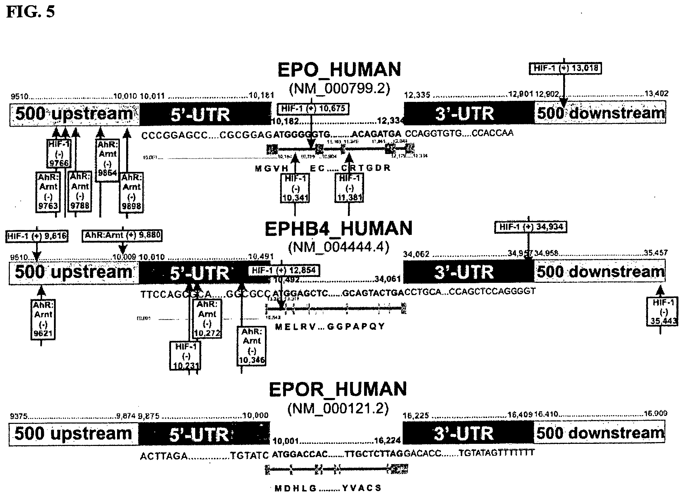

Figure 5 shows a schematic of the results from analysis of the 5' and 3' UTR's (and an additional 500bp on either side) of the EPO, EPOR and EPH-B4 genes for the presence of hypoxia inducible transcription factor binding sites. This study was performed employing the "Match" algorithm from TRANSFAC *(*Nucleic Acids Res. 2003 Jan 1, 31(1):374-8) to analyse the composition of HIF1 binding sites. Strikingly, only the EPO and EPH-B4 genes were found to contain such sites, supporting the hypothesis that EPH-B4 is indeed hypoxia inducible.

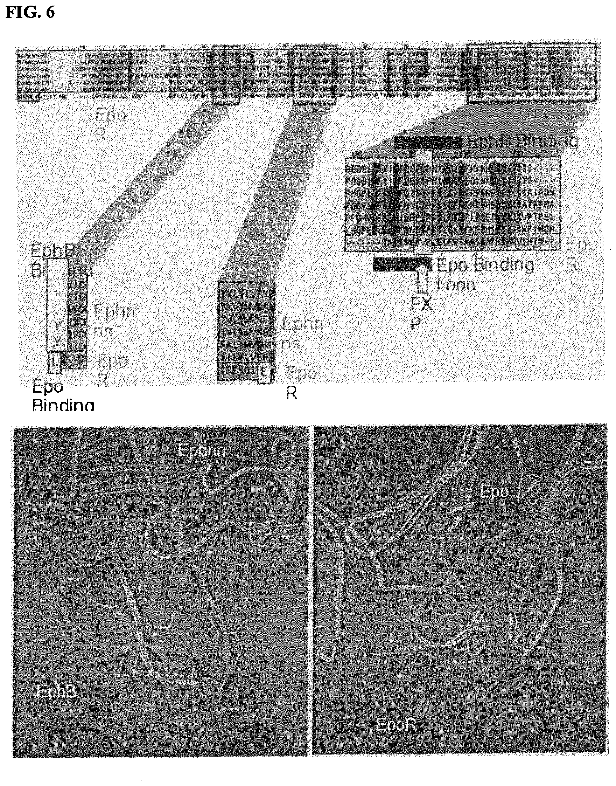

Figure 6 shows a structural analysis of EphrinA5:EphB2 association in comparison with that of EPO:EPOR. This structural analysis reveals several commonalities consistent with a propensity for Ephrin A1 to bind EPO. The top panel shows homology of the EPO binding region of EPOR to the human Ephrin A molecules. The two lower panels compare the structural aspects of Ephrin A with EPOR.

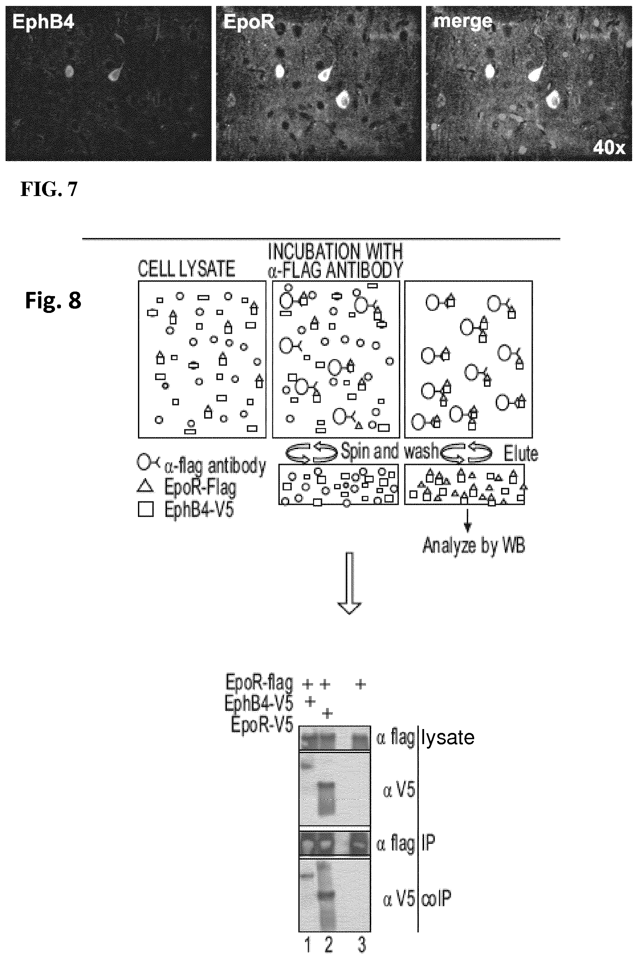

Figure 7 shows staining of hippocampus with anti-EPH-B4 and anti-EpoR antibodies. It should be noted that there is a striking co-expression of both proteins restricted to certain cells only. These data suggest functional coupling of EPH-B4 and EPOR activity.
Figure 8 shows co-immunoprecipitation of EPH-B4 using flag-tagged EpoR. This finding is consistent with the notion that EPH-B4 and EPOR might heterodimerize.
Figure 9 shows the possible various species of NEPOR (without being bound by theory). In this representation, NEPOR homo/heterodimer species are shown as Fc constructs. This mimics the dimerization of separate receptor monomers. Any method which allows the production of such NEPOR dimers can be employed in screening for NEPOR specific agonists and antagonists, including small molecules, peptides, proteins and EPO variants.

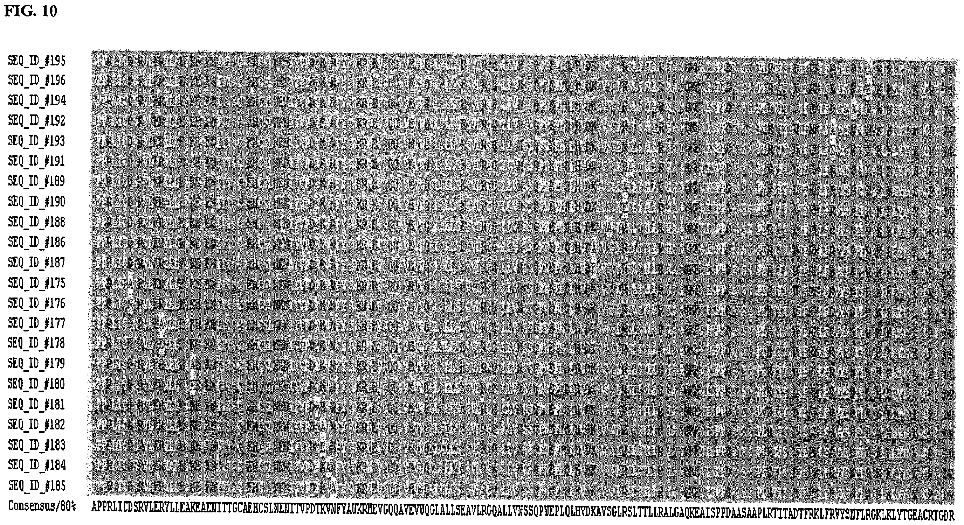

Figure 10 shows an alignment of EPO protein mutants which are predicted to bind NEPOR more favourably than EPOR. Such mutants are predicted to be primarily tissue protective as opposed to haematopoietic, particularly those versions combining the described mutations.

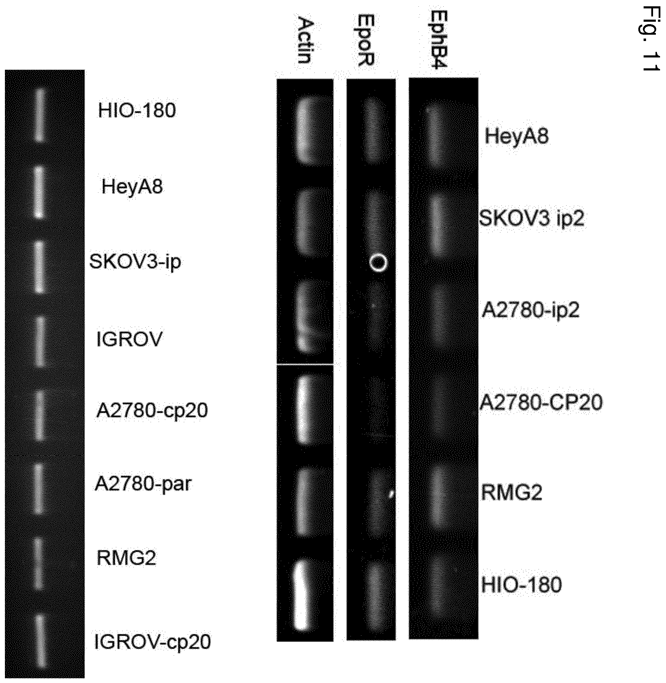

Figure 11 shows mRNA levels of ovarian cancer cell lines. RNA was isolated from a panel ovarian cancer cell lines and was reverse transcribed into cDNA. PCR was done using primers specific for EPO receptor, EPH-B4, Ephrin A1 and actin.

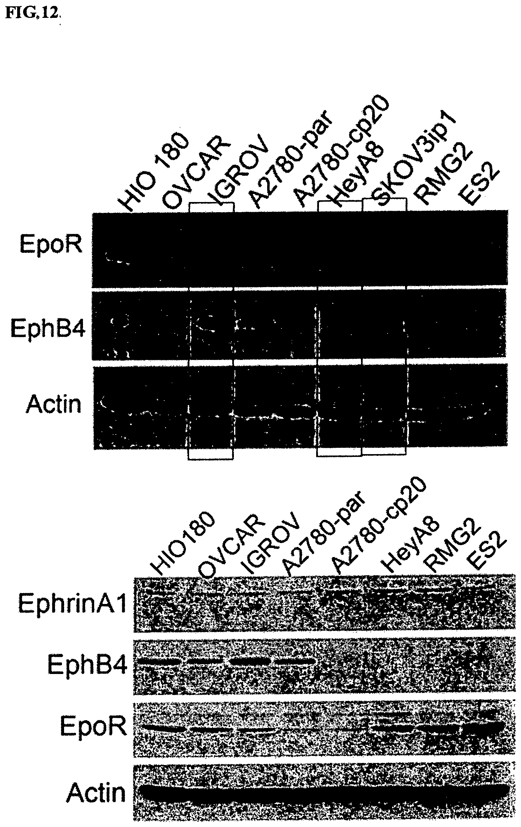

Figure 12 shows protein expression in ovarian cancer cell lines. Protein extracts were isolated from a panel ovarian cancer cell lines. Samples were separated using SDS-Page gel electrophoresis. Immunoblots using antibodies for EPO Receptor (R&D biosystems), EPH-B4 (a gift from Prakash Gil), Ephrin A1 and acting (Sigma Aldrich) were used to compare protein expression.

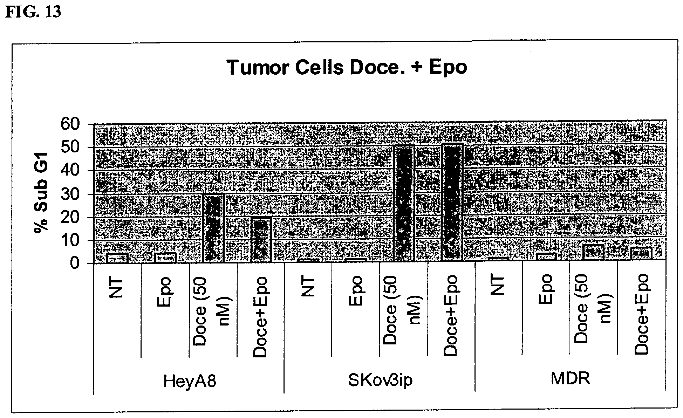

Figure 13 shows ESA protection from chemotherapy induced apoptosis. Ovarian cancer cell lines Hey A8, SkoV3 ipl, and HeyA8-MDR (chemoresistant) were treated with 50 U erythropoietin (EPO), 50 nM docetaxel, or a combination of EPO and docetaxes for 48 hours. Cells were then fixed and DNA stained with propidium iodide. Percentage of sub G1 cells were then quantified using flow cytometer (BD).

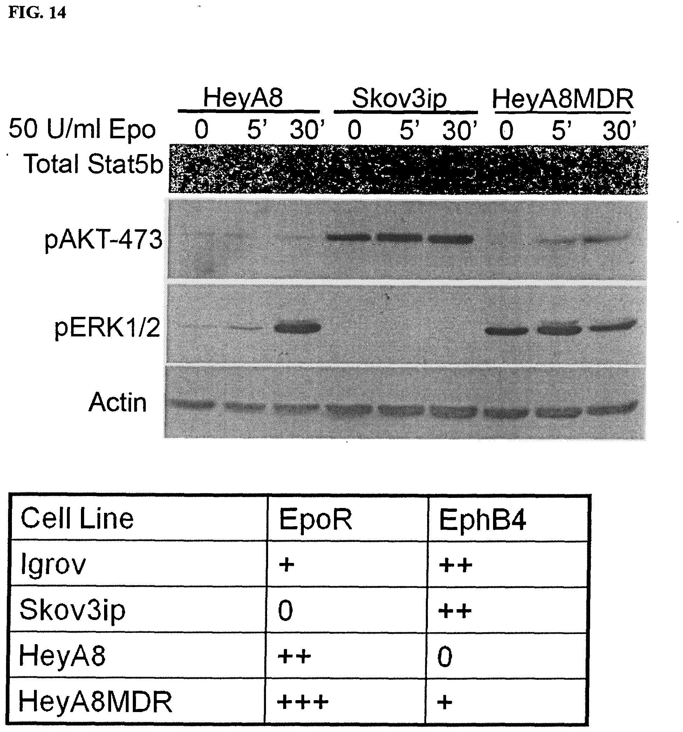

Figure 14 shows signalling pathways activated in response to EPO in ovarian cancer cell lines. Cell lines previously characterized for expression levels of EPOR, EPH-B4, and Ephrin A1 were washed and grown in serum free media for two hours. Cells were then treated with 50 U EPO and collected and designated time points (0, 5 and 30 minutes). Protein extracts were isolated and analyzed by immunoblots using antibodies for phosphor-STAT5 (Invitrogen), phosphor-AKT, phosphor-ERK (Cell Signaling) and acting (Sigma Aldrich).

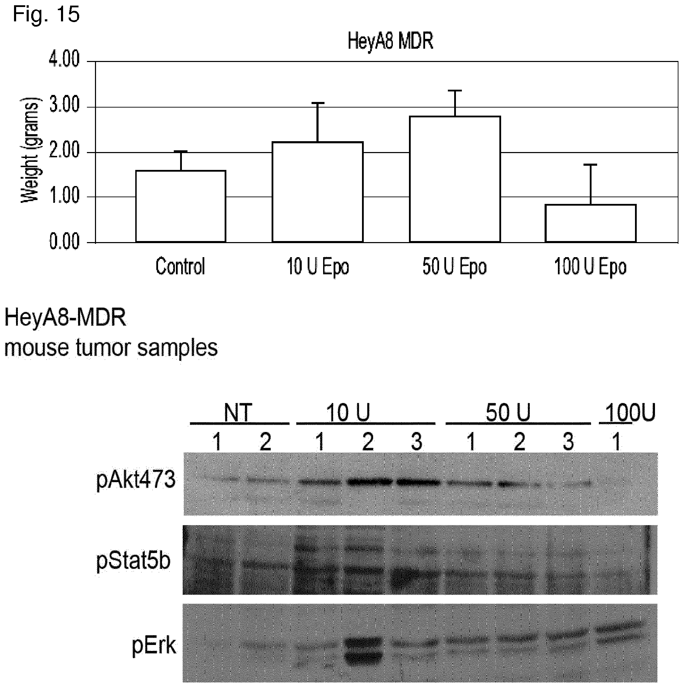

Figure 15 shows erythropoietin induced tumor growth in nude mice. Mice were injected i.p. with 1x10⁶ Hey MDR ovarian cancer cells. Day eight following injections mice were injected with designated amounts of EPO (10, 50, 100 U, three mice per group) every second day. A) Mice were sacrificed at day 26 and tumor weight was measured. B) Protein extracts were isolated from tumors and analyzed by immunoblot using antibodies specific for phosphor AKT ser 473, phosphor ERK (Cell Signaling) and pSTAT5b (Invitrogen).

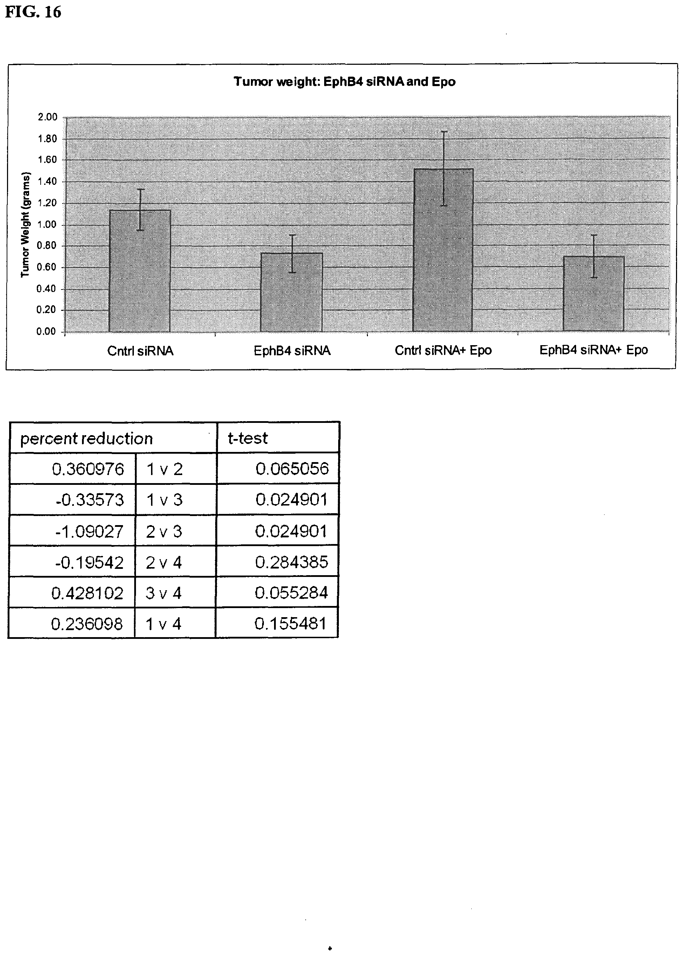

Figure 16 shows EPH-B4 expression effects tumor promoting effect of EPO. Female nude mice were injected i.p. with 1x10⁶ HeyA8-MDR cells. Day eight following injection the cells were treated with control siRNA - DOPC, EPH-B4 siRNA-DOPC, EPO, or in EPO + control or EPH-B4 siRNA-DOPC (10 per group). (50 U EPO given 3 x week, 5 µg siRNA 2 x week). Mice were sacrificed on day 25 and tumor weights were measured. Statistics were done using students T-test. B) Distribution of tumor weight per group.

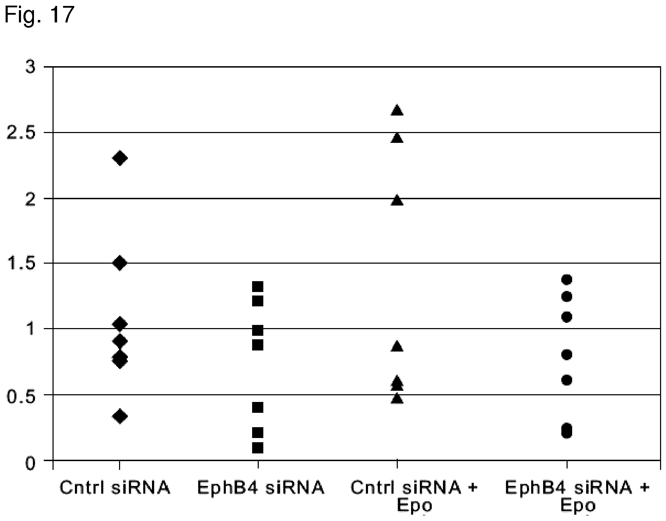

Figure 17 shows tumor weight distributions.

### Detailed Description

The present disclosure results from the identification of a novel EPO receptor, henceforth referred to as NEPOR. NEPOR was identified using a bioinformatics workflow encompassing both a functional and sequence based analysis of the human genome/proteome. Homology analysis involving an extracellular protein database (termed XtraCellDB) was used in conjunction text-mining and genome context analysis. These *in silico* predictions were subsequently verified in lab-based experiments. Thus, the present disclosure provides genomic, proteomic and experiment evidence that the protein EPH-B4 (Erythropoietin Producing Hepatoma protein B4) and/or Ephrin A1 act as EPO receptors.

Working on the theory that the adverse effects of EPO seen in many cancer patients may be mediated by a receptor complex distinct from the prototypical EPO receptor (EPOR) homodimer, we initiated an *in silico* discovery project to try to identify a novel EPO receptor. Should such a novel EPO receptor species exist, we hypothesized that it will be responsible for mediating EPO-induced cell survival activity, as opposed to EPO mediated haematopoietic activity. Thus, we proposed the existence of at least two species of EPO receptor; the prototypical EPOR homodimer which is primarily responsible for EPO's haematopoietic activity, and a novel EPO receptor, termed NEPOR, which is primarily responsible for EPO's cytoprotective activities. The existence of such a novel EPO receptor is compelling for three main reasons. Firstly it allows the prediction of a cancer patients response to EPO. Presence of NEPOR on a tumour cell would imply a negative response to EPO, since binding of EPO by NEPOR would induce a cascade of survival signals within tumour cells and tissues, thus contributing to cancer progression and poorer patient survival. Thus, detection of NEPOR expression in a tumour provides a novel biomarker for stratify cancer patients as suitable (*i.e*. NEPOR not present) or unsuitable (*i.e*. NEPOR present) for EPO treatment. A corollary of this model is a second interesting perspective. If NEPOR is capable of initiating survival signals on cancer cells, then it represents an excellent therapeutic target for treatment of cancers expressing this receptor. Thus, therapeutic molecules targeting and antagonizing the tissue protective function of this receptor should be efficacious anti-cancer agents. Finally, under conditions where induction of cell survival is favourable, such as in response to ischemic stroke, therapeutic molecules capable of activating NEPOR-mediated survival signals provide an efficacious path to treating a variety of neurological diseases. Definition of NEPOR's molecular composition therefore provides the molecular basis for designing such therapies.

It had previously been proposed that rHuEPO can promote tumour growth through stimulation of Epo receptor (EPOR) signalling in tumour cells, and via the stimulation of angiogenesis. Binding of EPO to EPOR homodimers was assumed to somehow confer survival advantage to cancer cells, leading to increased loco-regional progression and poorer survival rates in patients having a form of cancer. However, aware of the binding promiscuity of exogenously administered therapeutics, we were anxious to address the possibility as to whether another receptor might be responsible for the observed negative outcomes, either alone or in functional interaction with EPOR.

In an effort to identify such a novel cytoprotective EPO receptor, we developed an *in silico* based analysis approach specifically designed to mine the human proteome for candidate molecules. Combining the power of text-mining and in-depth bioinformatics analysis, this multi-evidence based approach successfully identified a putative novel EPO receptor. Subsequent lab-based validation supports these findings. Given its established physiological role, we propose that by impinging on this receptors activity, EPO can confer survival advantage to certain cells, including cancer cells and neurons. As a consequence, the expression of this protein on cancer cells can be used to stratify the suitability of cancer patients for EPO treatment. Patients with cancer associated NEPOR expression should be contraindicated for EPO treatment. However, a corollary of this finding is that these same individuals represent excellent candidates for treatment with antagonistic anti-NEPOR therapies. In addition, we also propose that by mediating EPO's cyto-protective activity, NEPOR represents an excellent therapeutic target for a variety of diseases involving tissue ischaemia (*e.g*. stroke).

Thus, in the first instance, the present disclosure provides a method for assessing a tumour for expression of NEPOR. The disclosure provides a method to stratify patients having a tumour as suitable (*i.e*. NEPOR not present) or non-suitable (i.e., NEPOR present) for EPO treatment. The method disclosed comprises: (a) isolating a tissue sample from an individual who is receiving or shall receive erythropoietin, (b) determining the level of expression of the NEPOR gene(s) (mRNA) and/or the presence of the NEPOR gene product (protein) from the isolated tissue, and (c) correlating the presence of an NEPOR gene expression product or the presence of NEPOR protein to a physiological response to the treatment with erythropoietin. In a second instance, the present disclosure provides a method for treating patients possessing NEPOR positive tumors. Furthermore, the present disclosure provides a method for treating stroke. Finally, by providing a means of comparing binding affinities of putative therapeutics to both NEPOR and EPOR, the present disclosure provides a method for screening for NEPOR specific therapeutics (both antagonistic therapeutics for cancer, and agonistic therapeutics for treatment of hypoxia associated disease such as stroke). Such therapeutics will lack the haematopoietic activity associated with EPOR binding and signaling.

### NEPOR - Molecular definition

We have identified a novel multimeric EPO receptor, which we term NEPOR. NEPOR comprises EPHB4 and/or Ephrin A1 molecules either as homodimers or heterodimers. Without being bound by theory, these components may also heterodimerize with the EPO receptor. A synopsis of the possible molecular compositions of NEPOR is provided in Figure 3. Despite the room for molecular promiscuity involving other components from ephrin biology, EPH-B4 and/or EphrinA1 are components of a novel EPO receptor (NEPOR). As such NEPOR is primarily composed of EPH-B4 and Ephrin A1, either as a homodimers and/or in heterodimeric association with each other, or the EPO receptor. Without being bound by theory, given the strong functional association between EPH-B4 and Ephrin B2, NEPOR may also comprise Ephrin B2 disclosed herein as SEQ ID NO. 4 (amino acid sequence), SEQ ID NO. 8 (mRNA sequence), and SEQ ID NO. 12 (binding region).

Table 5 shows, without being bound by theory, the possible molecular composition of dimeric EPO receptors. The prototypical haematopoietic EPO receptor (EPOR) represents a homodimer of two EPOR (SEQ ID NO.1) monomers (1). Our results suggest that a novel tissue protective EPO receptor dimer is comprised of Ephrin A1 (SEQ ID NO.3) and EPH-B4 (SEQ ID NO.2). Possible scenarios are shown in Table 5.

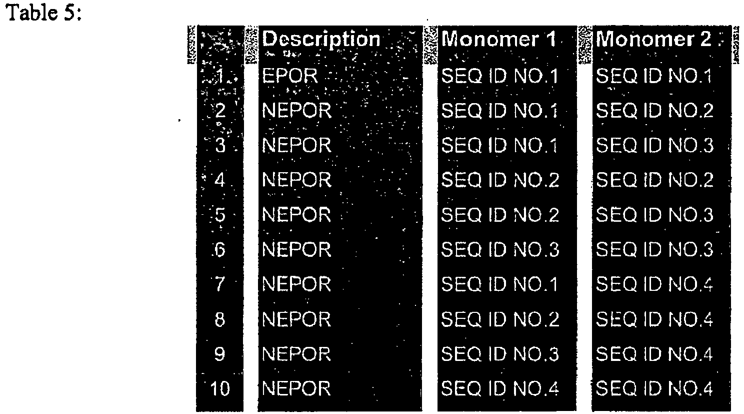

SEQ ID NO.1
   >EPOR
SEQ ID NO.2
   >EPH-B4
SEQ ID NO.3
   >EphrinAl
SEQ ID NO.4
   > EphrinB2

The present disclosure includes any splice variant of the polypeptides of SEQ ID NOS 1-4 components possessing the extracellular EPO binding region (for EPH-B4 this region of proposed to encompass the two fibronectinIII domains; the pink oval structures in Figure 3B, D and F) and the intracellular signalling part, is also capable of mediating EPO's (and derivatives thereof) cyto-protective effect.

### NEPOR: Prognostic implications

The type 1 cytokine, Erythropoietin (EPO), possesses both haematopoietic and tissue protective activities. The present disclosure provides that the latter functionality is mediated via interactions of EPO with a novel EPO receptor, termed NEPOR. The model provides that binding of EPO to NEPOR receptor complexes, on NEPOR positive cancer cells, confers survival advantage to such cells. The implicit physiological outcome for patients possessing NEPOR positive cancers is therefore increased loco-regional cancer progression and poorer overall survival.

Thus, the present invention provides a diagnostic or prognostic test that can predict whether or not cancer patients administered EPO will respond negatively in terms of survival outcome. The prognostic test comprises determining NEPOR (*i.e*. EPH-B4, and/or Ephrin A1) in tumour tissue, or more particularly cancer cells. In another embodiment NEPOR component gene expression levels in tumour cells can be compared to baseline levels or levels in surrounding normal cells or tissue. Therefore, a comparative analysis looking at elevated or normal baseline expression levels of NEPOR component expression, using standard gene expression analysis methods (such as q-PCR and DNA microarray analyses) provides a diagnostic test that can determine whether or not administration of EPO to cancer patients will unwittingly enhance tumour cell survival (a negative outcome).

As stated, one method that can be used for comparing levels of gene expression of components of NEPOR and/or EPH-B4, and/or Ephrin A1 is Quantitative polymerase chain reaction (qPCR). This is a modification of PCR or polymerase chain reaction used to rapidly measure the quantity of DNA present in a tissue sample. Like other forms of polymerase chain reaction, the process is used to amplify nucleic acid samples, via the temperature-mediated enzyme DNA polymerase. PCR amplifies DNA exponentially, doubling the number of molecules present with each amplification cycle. The number of amplification cycles and the amount of PCR end-product should allow one to calculate the initial quantity of NEPOR-specific genetic material and/or EPH-B4 and/or Ephrin A1 genetic material in particular mRNA molecules using NEPOR-specific component sequences in particular and/or EPH-B4, and/or Ephrin A1 sequences for the two primers used for amplification.

In addition, gene expression analysis of NEPOR components and/or EPH-B4, and/or Ephrin A1 can be done with a microarray analysis containing a plurality of capture probes specific for sequences of the NEPOR complex in particular and/or EPH-B4, and/or Ephrin A1. As EPO is proposed to stimulate survival of NEPOR positive cancer cells and/or EPH-B4, and/or Ephrin A1 positive cells, it is important to test all cancer patients for NEPOR status and/or and/or EPH-B4, and/or Ephrin A1 status prior to and during EPO administration. This is best done with a microarray analysis for expression status of NEPOR component genes in tumour tissue and with mRNA samples taken from tumour tissue. Ascertaining the levels of endogenous tumour associated NEPOR (*i.e*. EPH-B4, and/or EphrinAl) expression, provide correlations as to patient prognosis/survival rate.

The present disclosure thus provides a method to stratify patients having a tumour as suitable *(i.e.* NEPOR not present and/or EPH-B4, and/or Ephrin A1 present) or non-suitable *(i.e.,* NEPOR present and/or and/or EPH-B4, and/or Ephrin A1 present) for EPO treatment. The method disclosed comprises: (a) isolating a tissue sample from an individual who is receiving or shall receive erythropoietin, (b) determining the level of expression of EPH-B4 and/or Ephrin A1 from the isolated tissue, and (c) correlating the presence of these component gene expression products to a negative physiological response to the treatment with erythropoietin.
SEQ ID NO.5
   >erythropoietin receptor (EPOR), mRNA
SEQ ID NO.6
   >EPH receptor B4 (EPHB4), mRNA
SEQ ID NO.7
   >Ephrin-Al (EFNA1) mRNA
**SEQ** ID NO.8
   >ephrin-B2 (EFNB2), mRNA

Detection of NEPOR component mRNA (SEQ ID NOs 5-8) should preferentially be performed using probes complementary to the sub-region of SEQ ID NO's 5-8, encoding the EPO binding domain and is particular SEQ Id NO. 6 and/or 7 encoding EPH-B4 and Ephrin A1. This implies for EPH-B4, probes complementary to SEQ ID NO. 10.; for Ephrin A1, probes complementary to SEQ ID NO. 11.
SEQ ID NO.9
   >epor_epobinding_coding region
SEQ ID NO.10
   >ephb4_epobinding_coding region
SEQ ID NO.11
   >ephrinA1_epobinding_coding region
SEQ ID NO.12
   >ephrinb2_epobinding_coding region

The determination of the presence of the Ephrin A1 and/or the determination of the presence of the EPH-B4 gene product (mRNA) may be done by using a hybridization technique or an amplification technique. It is preferred that the technique is selected from the group of, real-time-PCR, northem-blot analysis, reverse transcription and amplification, zymography, ligase-chain-reaction, NASBA, RNase Protection Assay (RPA), capillary electrophoresis with laser induced fluorescence (CE-LIF) and combinations thereof.

Specifically, the individual is a cancer patient who is to be treated with erythropoietin or is being treated with erythropoietin. Preferably, the negative physiological effect is poorer patient survival due to enhanced tumor progression. Preferably, the presence of a higher level of NEPOR component genes (mRNA) and/or the presence of NEPOR component gene expression products (proteins) and/or EPH-B4 and/or Ephrin A1 on tumor tissues is indicative of poorer survival prognosis upon treatment with erythropoietin.

Preferably, the determination of the presence of the NEPOR dimer complex is done by detecting the respective NEPOR proteins with an immunoassay. Also peptides thereof may be detected. The immunoassay is selected from the group of immunoprecipitation, a protein array or binding to a mass microbalance instrument (for example, Q-Sense or Attana), enzyme immunoassay (EIA), radioimmunoassay (RIA) or fluorescent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a luciferase-assay. Preferably, the immunoassay is an ELISA. Preferably, the anti-NEPOR antibody and/or EPH-B4 and/or Ephrin A1 antibody is a monoclonal or polyclonal antibody, for example selected from - or similar to - the antibodies listed in Table 6.

Preferably, detection of NEPOR component proteins should preferentially be performed using antibodies detecting the sub-regions of SEQ ID NOs 6 and 7, representing the EPO binding domain. This implies for EPH-B4, antibodies specific to SEQ ID NO. 14.; for Ephrin A1, antibodies specific to SEQ ID NO. 15.
SEQ ID NO.13
   >epor_epobinding_region
SEQ ID NO.14
   >ephb4_epobinding_region
SEQ ID NO.15
   >ephrinA1_epobinding_region
SEQ ID NO.16
   >ephrinb2_epobinding_region

Preferably, the individual is a cancer patient who is to be treated with erythropoietin or is being treated with erythropoietin. The tissue sample may be selected from the group of biological tissues and fluids such as blood, lymph, urine, cerebral fluid. The tissue sample may also be a tumor biopsy sample. It is preferred that the tissue sample is from the cancer tissue or circulating cells derived from same.

It is preferred that the cancer of the cancer patient is selected from the group of, head and neck cancer, breast cancer, liver cancer, colorectal cancer, small intestine cancer, leukemia, prostate cancer, lung cancer, ovarian cancer, pancreatic cancer, endometrial cancer, stomach cancer, non-Hodgkin lymphoma, kidney cancer, Renal cell carcinoma (RCC), malignant melanoma, gallbladder cancer, bladder cancer, vulvar cancer, Penile cancer, testicular cancer, thymus cancer, Kaposi's sarcoma, eye cancer, adrenal gland cancer, brain cancer, cervical cancer, appendix cancer, adenoid cancer, bile duct cancer, urethral cancer, spinal cancer, Ewing's family of tumors, extragonal germ cell cancer, extra hepatic bile duct cancer, fallopian tube cancer, soft tissue cancers, bone cancer, Hodgkin's lymphoma, anal cancer, malignant mesothelioma, vaginal cancer skin cancer, central nervous system cancer (craniopharyngioma), pleuropulmonary blastoma, nasal cavity and paranasal sinus cancer transitional cell cancer of renal pelvis and ureter, pituitary gland cancer, sqamous cell carcinoma of the head and neck (HNSCC), prostate cancer, colorectal cancer, lung cancer, brain cancer, bladder cancer, and salivary gland cancer. It is particularly preferred that the cancer is selected from the group of squamous cell carcinoma of the head and neck (HNSCC), prostate cancer, colorectal cancer, lung cancer, kidney cancer, brain cancer and bladder cancer.

### NEPOR and Disease Intervention and Therapy Design/Screening.

Without being bound by theory, NEPOR is proposed to mediate the cyto-protective effects of EPO and its variants. Thus, EPO and variants that have been shown to possess cyto-protective (but not haematopoietic) activity can affect NEPOR function. Therefore, the present disclosure provides knowledge of NEPOR's composition that can be used to optimize the structure and efficacy of such therapeutic molecules (that is, better manage the structure-activity relationship or SAR of the EPO pharmacophore). Moreover, the present disclosure provides knowledge of NEPOR's composition that can be used to identify novel NEPOR regulating compounds. For example, in diseases associated with hypoxic conditions (*e.g*., stroke, heart attack), NEPOR binding compounds of enhanced efficacy can be developed to mimic the effects of EPO on NEPOR. Similarly, NEPOR specific antagonists (such as those molecules that bind the active site of NEPOR yet do not transducer signal are antagonists of EPO function. Such EPO antagonist agents, when concomitantly administered with EPO, can allow for EPO effects to improve haematopoiesis (that is, treat the anaemia) yet prevent the side effect of promoting tumour cell growth, survival and angiogenesis in NEPOR positive cancers such as HNSCC. Moreover, contrasting the relative activity of compounds to the tissue protective NEPOR receptor complex in comparison to the EPOR receptor homodimer provides for generating NEPOR specific/directed therapies.

Definition of NEPOR provides methods for identifying therapeutic molecules that modulate NEPOR's tissue protective signalling activity. This comprises: (a) contacting a test compound with the NEPOR receptor complex and/or EPH-B4 and/or Ephrin A1 and an EPOR homodimer complex; (b) measuring and comparing the level of tissue protective activity initiated by NEPOR activation with the activation of EPOR homodimer signalling; (c) identifying a test compound which increases or decreases the level of tissue protective NEPOR complex activity as compared to the level of EPOR complex activation; and (d) assaying the identified therapeutics for tissue protective activity mediated via NEPOR, but lack of EPOR activation and (e) assaying the identified therapeutics for NEPOR inhibitory activity. The method is useful for identifying therapeutics that modulates the interaction between a tissue protective NEPOR complex and/or EPH-B4 and/or Ephrin A1 and the EPO ligand. The method is furthermore useful for identifying therapies for treating diseases of the central nervous system or peripheral nervous system which have primarily neurological or psychiatric symptoms, ophthalmic diseases, cardiovascular diseases, cardiopulmonary diseases, respiratory diseases, kidney, urinary and reproductive diseases, bone diseases, skin diseases, gastrointestinal diseases and endocrine and metabolic abnormalities and cancer.

More specifically, identification of NEPOR provides a method identifying (II) a compound that modulates the tissue protective activity of NEPOR, comprising:
(a) contacting a test compound with a tissue protective NEPOR receptor complex (N) and/or EPH-B4 and/or Ephrin A1 or tissue protective cytokine receptor complex-expressing cell; measuring the level of the activity of (N) in the cell; identifying a test compound that increases or decreases the level of activity of (N) as compared to the level of activity of (N) measured in the absence of the test compound; and assaying the identified test compound for tissue protective activity;
(b) contacting a test compound with a cell that is recombinantly engineered to express (N), where the cell or the recombinant cell is transformed with a nucleic acid comprising a nucleotide sequence that is functionally linked to a promoter and encodes EPH-B4 and/or Ephrin A1 polypeptides; measuring the level of activity of (N) in the cell; and
(c) contacting a test compound with a tissue protective NEPOR receptor complex-expressing cell, where the cell is transformed with a nucleic acid comprising a nucleotide sequence that encodes a reporter gene functionally linked to regulatory element associated with the activity of (N); identifying a test compound that increases or decreases the level of reporter gene expression relative to the level of reporter gene expression measured in the absence of the test compound; and assaying the identified test compound for a tissue protective activity.

The present disclosure further provides a method for identifying (12) a compound that binds to (N), comprising:
(a) contacting (N) with a tissue protective NEPOR receptor complex ligand and/or EPH-B4 and/or Ephrin A1 ligand attached to a first label, and an equivalent amount of a test compound attached to a second label under conditions conducive to binding, removing unbound material from (N), and detecting the level of the first and second labels, where if the second label is present the compound binds (N) and if the level of the first label decreases relative to the level of the first label when the labelled ligand is contacted with (N) under conditions conducive to binding in the absence of a test compound after removal of unbound material, then a compound that binds to (N) is identified; or
(b) contacting a test compound with a ligand-binding tissue protective receptor NEPOR complex fragment comprising at least one EPH-B4 receptor or Ephrin A1 receptor, extracellular domain fused to a Fc fragment attached to a solid support, removing unbound test compounds from the solid support, and identifying the compound attached to the tissue protective NEPOR receptor complex fragment, such that a compound bound to the solid support is identified as a compound that binds to a tissue protective NEPOR receptor complex; and identifying (13) a compound that modulates the binding of a tissue protective NEPOR receptor complex ligand to (N), or compound that modulates the interaction between (N) and tissue protective cytokine receptor complex ligand, involves (i) contacting a tissue protective NEPOR receptor complex ligand with (N) in the presence of one or more test compounds under conditions conducive to binding, and measuring the amount of tissue protective cytokine receptor complex ligand bound to (N).

The present disclosure further provides novel tissue protective NEPOR receptor complexes in particular EPH-B4 and/or Ephrin A1 containing complexes that can be used to provide an in *vitro* screening assay for NEPOR specific therapies; by measuring the binding of test compounds to the tissue protective NEPOR receptor complex in comparison to EPOR homodimer complexes. The test compound is labelled and binding of the labelled test compound to the receptor complexes detailed in Figure 9 is measured by detecting the label attached to the test compound. Alternatively, a label free detection approach such as surface plasmon resonance may be employed. Such an approach can provide for novel neuroprotective therapies (*i.e*. NEPOR agonists) which lack haematopoietic activity. Such an approach can also provide for novel oncotherapies *(i.e.* NEPOR antagonists i.e. at least a and/or EPH-B4 and/or Ephrin A1 agonist) which do not inhibit haematopoiesis. The nature of such screening arrays involving recombinant receptor constructs is demonstrated in Figure 9 (in the exemplified case as Fc constructs).

### Use

(I1) is useful for identifying a compound that modulates NEPOR's tissue protective activity. (I2) is useful for identifying a compound that binds to NEPOR. (I3) is useful for identifying a compound that modulates the binding of a tissue protective NEPOR receptor complex ligand to (N), or compound that modulates the interaction between (N) and tissue protective cytokine receptor complex ligand (claimed). The compounds identified using (I1)-(I3) are useful for treating various conditions of the central and peripheral nervous systems (*e.g*., hypoxia, and/or ischemia, epilepsy, chronic seizure disorders, neurotoxin poisoning, septic shock, anaphylactic shock), neuropsychologic disorders (senile dementia, Alzheimer's disease, Parkinson's disease, dermentia, multiple sclerosis, Creutzfeldt-Jakob disease, Huntington's disease), inflammatory diseases (*e.g*., chronic bronchitis, rheumatoid arthritis, glomerulonephritis, encephalitis, meningitis, polymyositis), opthalamic diseases (*e.g*., angiitis, retinal ischemia), cardiovascular diseases (*e.g.*, myocardial infraction, myocarditis), cardiopulmonary diseases (*e.g.*, asthma, pulmonary thrombosis), respiratory diseases, kidney, urinary, and reproductive diseases (*e.g*., myasthenia gravis, diabetes, autoimmune diseases), bone diseases (*e.g*., osteopenia, Paget's disease), gastrointestinal diseases and endocrine and metabolic abnormalities.

The compounds identified using (I1)-(I3) are also useful for treating NEPOR positive cancers in particular and/or EPH-B4 and/or Ephrin A1 positive cancers including, head and neck cancer, breast cancer, liver cancer, colorectal cancer, small intestine cancer, leukemia, prostate cancer, lung cancer, ovarian cancer, pancreatic cancer, endometrial cancer, stomach cancer, non-Hodgkin lymphoma, kidney cancer, Renal cell carcinoma (RCC), malignant melanoma, gallbladder cancer, bladder cancer, vulvar cancer, Penile cancer, testicular cancer, thymus cancer, Kaposi's sarcoma, eye cancer, adrenal gland cancer, brain cancer, cervical cancer, appendix cancer, adenoid cancer, bile duct cancer, urethral cancer, spinal cancer, Ewing's family of tumors, extragonal germ cell cancer, extra hepatic bile duct cancer, fallopian tube cancer, soft tissue cancers, bone cancer, Hodgkin's lymphoma, anal cancer, malignant mesothelioma, vaginal cancer skin cancer, central nervous system cancer (craniopharyngioma), pleuropulmonary blastoma, nasal cavity and paranasal sinus cancer transitional cell cancer of renal pelvis and ureter, pituitary gland cancer, sqamous cell carcinoma of the head and neck (HNSCC), prostate cancer, colorectal cancer, lung cancer, brain cancer, bladder cancer, and salivary gland cancer. It is particularly preferred that the cancer is selected from the group of squamous cell carcinoma of the head and neck (HNSCC), prostate cancer, colorectal cancer, lung cancer, kidney cancer, brain cancer and bladder cancer.

### NEPOR in Oncology Therapy

The hypothesis of the present disclosure is that EPO results in poorer survival outcomes (at least in some cancers) because of its effects on NEPOR activity i.e. in particular EPH-B4 and/or Ephrin A1 activity. Therefore, treatment of these NEPOR positive patients with a NEPOR targeted therapy is a prudent path to disease intervention. Specific approaches to antagonising NEPOR mediated survival signals include, for example:
a) NEPOR specific antagonistic antibodies. Such antibodies block and antagonise the extracellular regions of the molecule specifically associated with the mediation of NEPOR's cyto-protective activity.
b) NEPOR specific small-molecules. Such small molecules block and antagonise the extracellular regions of the molecule specifically associated with the mediation of NEPOR's cytoprotective activity.
c) high-affinity peptides which specifically target NEPOR to block and antagonise the mediation of EPO's cytoprotective activity.
d) Small molecules targeting EPH-B4's intracellular tyrosine kinase domain (*e.g.* Dasatinib), including:
   1: CID: 1095868, AKI-STT-00166305; ZINC00818264; BAS 09636496 IUPAC: N-[5-[(3-chlorophenyl)methyl]-1,3-thiazol-2-yl]-2-(4,6-dimethylpyrimidin-2-yl)sulfanylacetamide. MW: 404.93678 | MF: C18H17ClN4OS2. (MW is molecular weight and MF is molecular formula)
   2: CID: 1465558, IUPAC: 2-[(3-chlorobenzoyl)amino]-4-methyl-N-pyridin-3-yl-1,3-thiazole-5-carboxamide, MW: 372.82872 | MF: C17H13ClN4O2S.
   3: CID: 1468201, IUPAC: N-[5-[(2-chlorophenyl)carbamoyl]-4-methyl-1,3-thiazol-2-yl]pyridine-4-carboxamide, MW: 372.82872 | MF: C17H13ClN4O2S.
   4: CID: 3062316, Dasatinib; Sprycel; BMS Dasatinib, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 488.00554 | MF: C22H26ClN7O2S.
   5: CID: 3072360, 142287-40-9; Pyrimido(4,5-d)pyrimidin-4(1H)-one, 7-methyl-1-phenyl-2-((3-(4-(2-thiazolyl)-1-piperazinyl)propyl)thio)- IUPAC: 2-methyl-8-phenyl-7-[3-[4-(1,3-thiazol-2-yl)piperazin-1-yl]propylsulfanyl]pyrimido[6,5-d]pyrimidin-5-one, MW: 479.6209 | MF: C23H25N7OS2.
   6: CID: 5041467, STK154706; ZINC04687922, IUPAC: [2-[(2-methylphenyl)amino]-1,3-thiazol-4-yl]-(4-pyrimidin-2-ylpiperazin-1-yl)methanone, MW: 380.4667 | MF: C19H20N6OS. 7: CID: 9822929, IUPAC: N-(2-chloro-6-methylphenyl)-2-[(6-imidazol-1-ylpyridazin-3-yl)amino]-1,3-thiazole-5-carboxamide, MW: 411.869 | MF: C18H14ClN7OS.
   8: CID: 9927718, IUPAC: N-(2-chloro-6-methylphenyl)-2-(cyclopropanecarbonylamino)-1,3-thiazole-5-carboxamide, MW: 335.809 | MF: C15H14ClN3O2S.
   9: CID: 10006113, IUPAC: N-[4-chloro-2-[(5-chloropyridin-2-yl)carbamoyl]phenyl]-5-methyl-6,7-dihydro-4H-[1,3]thiazolo[5,4-c]pyridine-2-carboxamide hydrochloride, MW: 498.81322 | MF: C20H18Cl3N5O2S.
   10: CID: 10006114, IUPAC: N-[4-chloro-2-[(5-chloropyridin-2-yl)carbamoyl]phenyl]-5-methyl-6,7-dihydro-4H-[1,3]thiazolo[5,4-c]pyridine-2-carboxamide, MW: 462.35228 | MF: C20H17Cl2N5O2S.
   11: CID: 10052635, IUPAC: 2-[[2-methyl-5-[[6-[(4-methylpiperazin-1-yl)methyl]pyridin-2-yl]amino]phenyl]amino]-N-(2-methylphenyl)-1,3-thiazole-5-carboxamide, MW: 527.68362 | MF: C29H33N7OS.
   12: CID: 10195898, IUPAC: N-[(4-chlorophenyl)methyl]-2-[[[(2S)-2-hydroxy-2-pyrimidin-2-ylethyl]-methylamino]methyl]-4-methyl-7-oxothieno[2,3-e]pyridine-6-carboxamide, MW: 497.99706 | MF: C24H24ClN5O3S.
   13: CID: 10206276, IUPAC: N-[4-[(5-chloropyridin-2-yl)carbamoyl]-2-phenyl-1,3-thiazol-5-yl)-1-propan-2-ylpiperidine-4-carboxamide, MW: 484.01354 | MF: C24H26ClN5O2S.
   14: CID: 10252208, IUPAC: 2-[4-(5-amino-1,3-thiazol-2-yl)phenyl]-3-(5-chloropyridin-2-yl)quinazolin-4-one, MW: 431.89746 | MF: C22H14ClN5OS.
   15: CID: 10253695, IUPAC: 2-[4-[3-(5-chloropyridin-2-yl)-4-oxoquinazolin-2-yl]phenyl]-1,3-thiazole-5-carboxamide, MW: 459.90756 | MF: C23H14ClN5O2S.
   16: CID: 10301604, IUPAC: N-[4-[(5-chloropyridin-2-yl)carbarmoyl]-2-(3,4-difluorophenyl)-1,3-thiazol-5-yl]-1-propan-2-ylpiperidine-4-carboxamide, MW: 519.994466 | MF: C24H24ClF2N5O2S.
   17: CID: 10344807, IUPAC: N-[2-[4-[3-(5-chloropyridin-2-yl)-4-oxoquinazolin-2-yl]phenyl]-1,3-thiazol-4-yl]acetamide, MW: 473.93414 | MF: C24H16ClN5O2S.
   18: CID: 10368624, IUPAC: N-[(4-chlorophenyl)methyl]-2-[[(2-hydroxy-2-pyrimidin-2-ylethyl)-methylamino]methyl]-7-methyl-4-oxothieno[3,2-e]pyridine-5-carboxamide, MW: 497.99706 | MF: C24H24ClN5O3S.
   19: CID: 10370949, IUPAC: (3Z)-4-[[(2S)-2-(3-chlorophenyl)-2-hydroxyethyl]amino]-3-[6-methyl-2-[4-(1,3-thiazol-2-ylmethyl)piperazin-1-yl]-7,9-dihydropurin-8-ylidene]pyridin-2-one, MW: 578.08832| MF: C27H28ClN9O2S.
   20: CID: 10412586, IUPAC: N-[2-[4-[3-(5-chloropyridin-2-yl)-4-oxoquinazolin-2-yl]phenyl]-1,3-thiazol-5-yl]acetamide, MW: 473.93414 | MF: C24H16ClN5O2S.
   21: CID: 10413555, IUPAC: N-[(4-chlorophenyl)methyl]-2-[[[(2R)-2-hydroxy-2-pyrimidin-2-ylethyl]-methylamino]methyl]-7-methyl-4-oxothieno[3,2-e]pyridine-5-carboxamide, MW: 497.99706 | MF: C24H24ClN5O3S.
   22: CID: 10456156, IUPAC: 4-[(3-chlorothiophen-2-yl)methylamino]-2-[(4-morpholin-4-ylphenyl)amino]pyrimidine-5-carboxamide, MW: 444.93774 | MF: C20H21ClN6O2S.
   23: CID: 10458706, IUPAC: N-[5-[2-[(4-chlorophenyl)amino]pyrimidin-4-yl]-4-methyl-1,3-thiazol-2-yl]-3-(2-morpholin-4-ylethylamino)propanamide, MW: 502.03212 | MF: C23H28ClN7O2S.
   24: CID: 11153014, IUPAC: N-(2-chloro-6-methylphenyl)-2-[(2,6-dimethylpyrimidin-4-yl)amino]-1,3-thiazole-5-carboxamide, MW: 373.85984 | MF: C17H16ClN5OS.
   25: CID: 11167695, IUPAC: N-(2-chloro-6-methylphenyl)-2-[[2-methyl-6-(2-morpholin-4-ylethylamino)pyrimidin-4-yl]amino]-1,3-thiazole-5-carboxamide, MW: 488.00554 j MF: C22H26ClN7O2S.
   26: CID: 11168231, IUPAC: N-(2-chloro-6-methylphenyl)-2-[(6-chloro-2-methylpyrimidin-4-yl)amino]-N-[(4-methoxyphenyl)methyl]-1,3-thiazole-5-carboxamide, MW: 514.42684 | MF: C24H21Cl2N5O2S.
   27: CID: 11200510, IUPAC: N-(2-chloro-6-methylphenyl)-2-[[6-(2-hydroxyethylamino)pyridin-2-yl]amino]-1,3-thiazole-5-carboxamide, MW: 403.88582 | MF: C18H18ClN5O2S.
   28: CID: 11247793, IUPAC: N-(2-chloro-6-methylphenyl)-2-[[6-(methyl-(3-methylaminopropyl)amino)pyridin-2-yl]amino]-1,3-thiazole-5-carboxamide, MW: 444.9808 | MF: C21H25ClN6OS.
   29: CID: 11260009, IUPAC: N-(2-chloro-6-methylphenyl)-2-[[6-[4-(hydroxymethyl)piperidin-1-yl]-2-methylpyrimidin-4-yl]amino]-1,3-thiazole-5-carboxamide, MW: 472.9909 | MF: C22H25ClN6O2S.
   30: CID: 11269410, IUPAC: N-(2-chloro-6-methylphenyl)-2-[(6-chloro-2-methylpyrimidin-4-yl)amino]-1,3-thiazole-5-carboxamide, MW: 394.27832 | MF: C16H13Cl2N5OS.
   31: CID: 11282881, IUPAC: N-(2-chloro-6-methylphenyl)-2-[[6-(2-morpholin-4-ylethylamino)pyrimidin-4-yl]amino]-1,3-thiazole-5-carboxamide, MW: 473.97896 | MF: C21H24ClN7O2S.
   32: CID: 11283174, IUPAC: N-(2-chloro-6-methylphenyl)-2-[[6-(3-morpholin-4-ylpropylamino)pyridin-2-yl]amino]-1,3-thiazole-5-carboxamide, MW: 487.01748 | MF: C23H27ClN6O2S.
   33: CID: 11328827, IUPAC: N-(2-chloro-6-methylphenyl)-2-[[6-(3-imidazol-1-ylpropylamino)pyridin-2-yl]amino]-1,3-thiazole-5-carboxamide, MW: 467.97438 | MF: C22H22ClN7OS.
   34: CID: 11407465, IUPAC: N-(2-chloro-6-methylphenyl)-2-[[6-(2-hydroxyethylamino)-2-methylpyrimidin-4-yl]amino]-1,3-thiazole-5-carboxamide, MW: 418.90046 | MF: C18H19ClN6O2S.
   35: CID: 11466196, IUPAC: N-(2-chloro-6-methylphenyl)-2-[[2-methyl-6-(3-morpholin-4-ylpropylamino)pyrimidin-4-yl]amino]-1,3-thiazole-5-carboxamide. MW: 502.03212 | MF: C23H28ClN7O2S.
   36: CID: 11466607, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrochloride, MW: 524.46648 | MF: C22H27Cl2N7O2S.
   37: CID: 11487256, IUPAC: N-(2-chloro-6-methylphenyl)-2-[(6-morpholin-4-ylpyrimidin-4-yl)amino]-1,3-thiazole-5-carboxamide, MW: 430.91116 | MF: C19H19ClN6O2S.
   38: CID: 11505502, IUPAC: 2-[[6-[4-(2-hydroxyethyl)piperazin-1-yl]pyrimidin-4-yl)amino]-N-[2-methyl-5-[[3-(trifluoromethyl)benzoyl]amino]pheryl]-1,3-thiazole-5-carboxamide. MW: 626.65257 | MF: C29H29F3N8O3S.
   39: CID: 11512538, IUPAC: 2-[4-[6-[[5-[(2-chloro-6-methylphenyl)carbamoyl]-1,3-thiazol-2-yl]amino]-2-methylpyrimidin-4-yl]piperazin-1-yl]ethyl 2,2-dimethylpropanoate, MW: 572.12196 | MF: C27H34ClN7O3S.
   40: CID: 11539665, IUPAC: (3-chloro-2-fluorophenyl)-[4-[[6-[(5-fluoro-1,3-thiazol-2-yl)amino]pyridin-2-yl]methyl]piperazin-1-yl]methanone, MW: 449.904626 | MF: C20H18ClF2N5OS.
   41: CID: 11540687, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrate, MW: 506.02082 | MF: C22H28ClN7O3S.
   42: CID: 11569328, IUPAC: N-(2-chloro-6-methylphenyl)-2-[[3-[4-(2-hydroxyethyl)piperazin-1-yl]-5-methylphenyl]amino]-1,3-thiazole-5-carboxamide, MW: 486.02942 | MF: C24H28ClN5O2S.
   43: CID: 11570976, IUPAC: 2-[[6-[4-(2-hydroxyethyl)piperazin-1-yl]-2-methylpyrimidin-4-yl]amino]-N-[2-methyl-5-[[3-(trifluoromethyl)phenyl]carbamoyl]phenyl]-1 ,3-thiazole-5-carboxamide, MW: 640.67915 | MF: C30H31F3N8O3S.
   44: CID: 11577776, IUPAC: 2-[[6-(2-hydroxyethylamino)-2-methylpyrimidin-4-yl]amino]-N-[2-methyl-5-[[3-(trifluoromethyl)benzoyl]amino]phenyl]-1,3-thiazole-5-carboxamide, MW: 571.57407 | MF: C26H24F3N7O3S.
   45: CID: 11590089, IUPAC: (3-chloro-2-fluorophenyl)-[4-[5-methyl-6-(1,3-thiazol-2-ylamino)pyridin-2-yl]piperazin-1-yl]methanone, MW: 431.914163 | MF: C20H19ClFN5OS.
   46: CID: 11606973, IUPAC: N-[5-[[3-[4-(2-hydroxyethyl)piperazin-1-yl]-5-(trifluoromethyl)benzoyl]amino]-2-methylphenyl]-2-(pyridin-2-ylamino)-1,3-thiazole-5-carboxamide, MW: 625.66451 | MF: C30H30F3N7O3S.
   47: CID: 11650098, IUPAC: 2-1[6-(4-methylpiperazin-1-yl)pyrimidin-4-yl]amino]-N-[2-methyl-5-[[3-(trifluoromethyl)benzoyl]amino]phenyl]-1,3-thiazole-5-carboxamide,MW: 596.62659 | MF: C28H27F3N8O2S.
   48: CID: 11650132, IUPAC: pentyl N-[5-[(2-chloro-6-methylphenyl)carbamoyl]-1,3-thiazol-2-yl]-N-[6-[4-(2-hydroxyethyl)piperazin-1-yl]-2-methylpyrimidin-4-yl]carbamate, MW: 602.14794 | MF: C28H36ClN7O4S.
   49: CID: 11650511, IUPAC: N-[5-[[3-(4-ethylpiperazin-1-yl)-5-(trifluoromethyl)benzoyl]amino]-2-methylphenyl]-2-[[6-(2-hydroxyethylamino)-2-methylpyrimidm-4-yl]amino]-1,3-thiazole-5-carboxamide, MW: 683.74695 | MF: C32H36F3N9O3S.
   50: CID: 11664355, IUPAC: 2-[(2-methyl-6-morpholin-4-ylpyrimidin-4-yl)amino]-N-[2-methyl-5-[[3-(trifluoromethyl)benzoyl]amino]phenyl]-1,3-thiazole-5-carboxamide, MW: 597.61135 | MF: C28H26F3N7O3S.
   51: CID: 11664511, IUPAC: 2-[[4-[4-(2-hydroxyethyl)piperazin-1-yl]pyridin-2-yl]amino]-N-[2-methyl-5-[[3-(trifluoromethyl)benzoyl]amino]phenyl]-1,3-thiazole-5-carboxamide, MW: 625.66451 | MF: C30H30F3N7O3S.
   52: CID: 11669430, IUPAC: N-(2-chloro-6-methylphenyl)-2-[(2-methyl-6-piperazin-1-ylpyrimidin-4-yl)amino]-1,3-thiazole-5-carboxamide, MW: 443.95298 | MF: C20H22ClN7OS. 53: CID: 11676373, IUPAC: (3-chloro-2-fluorophenyl)-[4-[[6-(1,3-thiazol-2-ylamino)pyridin-2-yl]methyl]piperazin-1-yl]methanone, MW: 431.914163 | MF: C20H19ClFN5OS.
   54: CID: 11684148, IUPAC: (3-chloro-2-fluorophenyl)-[4-[[6-[(5-chloro-1,3-thiazol-2-yl)amino]pyridin-2-yl]methyl]piperazin-1-yl]methanone, MW: 466.359223 | MF: C20H18Cl2FN5OS.
   55: CID: 11700117, IUPAC: 2-[[6-(4-ethylpiperazin-1-yl)-2-methylpyrimidin-4-yl]amino]-N-[2-methyl-5-[[3-(trifluoromethyl)benzoyl]amino]phenyl]-1,3-thiazole-5-carboxamide, MW: 624.67975 | MF: C30H31F3N8O2S.
   56: CID: 11707091, IUPAC: 2-[[2-methyl-6-(4-methylpiperazin-1-yl)pyrimidin-4-yl]amino]-N-[2-methyl-5-[[3-(trifluoromethyl)benzoyl]amino]phenyl]-1,3-thiazole-5-carboxamide, MW: 610.65317 | MF: C29H29F3N8O2S.
   57: CID: 11714286, IUPAC: 2-[[5-[4-(2-hydroxyethyl)piperazin-1-yl]pyridin-2-yl]amino]-N-[2-methyl-5-[[3-(trifluoromethyl)benzoyl]amino]phenyl]-1,3-thiazole-5-carboxamide, MW: 625.66451 | MF: C30H30F3N7O3S.
   58: CID: 11714353, IUPAC: 2-[[6-[4-(2-hydroxyethyl)piperazin-1-yl]-2-methylpyrimidin-4-yl]amino]-N-[2-methyl-5-[[3-(trifluoromethyl)benzoyl]amino]phenyl]-1,3-thiazole-5-carboxamide, MW: 640.67915 | MF: C30H31F3N8O3S.
   59: CID: 11752136, IUPAC: N-(2-chloro-6-methylphenyl)-2-[[5-[4-(2-hydroxyethyl)piperazin-1-yl]-2-methylpyrimidin-4-yl]amino]-1,3-thiazole-5-carboxamide, MW: 488.00554 | MF: C22H26ClN7O2S.
   60: CID: 11772766, IUPAC: 4-[2-(3-chlorophenyl)ethylamino]-2-pyridin-4-yl-1,3-thiazole-5-carboxamide, MW: 358.8452 | MF: C17H15ClN4OS.
   61: CID: 11775143, IUPAC: N-(2-chloro-6-methylphenyl)-2-[(2-methyl-6-morpholin-4-ylpyrimidin-4-yl)amino]-1,3-thiazole-5-carboxamide, MW: 444.93774 | MF: C20H21ClN6O2S. 62: CID: 11854012, IUPAC: 2-[4-[6-[[5-[(2-chloro-6-methylphenyl)carbamoyl]-1,3-thiazol-2-yl]amino]-2-methylpyrimidin-4-yl)piperazin-1-yl]acetic acid, MW: 501.98906 | MF: C22H24ClN7O3S.
   63: CID: 11854269, IUPAC: 2-[4-[6-[[5-[(2-chloro-6-methylphenyl)carbamoyl]-1,3-thiazol-2-yl]amino]-2-methylpyrimidin-4-yl]piperazin-1-yl]ethyl hydrogen sulfate, MW: 568.06874 | MF: C22H26ClN7O5S2.
   64: CID: 11854270, IUPAC: N-(2-chloro-6-methylphenyl)-2-[[6-[2-(2-hydroxyethylamino)ethylamino]-2-methylpyrimidin-4-yl]amino]-1,3-thiazole-5-carboxamide, MW: 461.96826 | MF: C20H24ClN7O2S
   65: CID: 11854271, IUPAC: 2-[[6-(2-aminoethylamino)-2-methylpyrimidin-4-yl]amino]-N-(2-chloro-6-methylphenyl)-1,3-thiazole-5-carboxamide, MW: 417.9157 | MF: C18H20ClN7OS. 66: CID: 11854272, IUPAC: 2-[[2-[4-[6-[[5-[(2-chloro-6-methylphenyl)carbamoyl]-1,3-thiazol-2-yl]amino]-2-methylpyrimidin-4-yl]piperazin-1-yl]acetyl]amino]ethanesulfonic acid, MW: 609.12066 | MF: C24H29ClN8O5S2.
   67: CID: 11854533, IUPAC: N-(2-chloro-4-hydroxy-6-methylphenyl)-2-[[6-[4-(2-hydroxyethyl)piperazin-1-yl]-2-methylpyrimidin-4-yl]amino]-1,3-thiazole-5-carboxamide, MW: 504.00494 | MF: C22H26ClN7O3S.
   68: CID: 11854534, IUPAC: N-[2-chloro-6-(hydroxymethyl)phenyl]-2-[[6-[4-(2-hydroxyethyl)piperazin-1-yl]-2-methylpyrimidin-4-yl]amino]-1,3-thiazole-5-carboxamide, MW: 504.00494 | MF: C22H26ClN7O3S.
   69: CID: 11854535, IUPAC: N-(2-chloro-6-methylphenyl)-2-[[6-[4-(2-hydroxyethyl)-4-oxidopiperazin-4-ium-1-yl]-2-methylpyrimidin-4-yl]amino]-1,3-thiazole-5-carboxamide, MW: 504.00494 | MF: C22H26ClN7O3S.
   70: CID: 11854536, IUPAC: 2-[4-[6-[[5-[(2-chloro-6-methylphenyl)carbamoyl]-1,3-thiazol-2-yl]amino]-2-methylpyrimidin-4-yl]-1-oxidopiperazin-1-ium-1-yl]acetic acid, MW: 517.98846 | MF: C22H24ClN7O4S.
   71: CID: 11949914, IUPAC: 4-[[2-(5-chloro-2-fluorophenyl)-5-dimethylaminopyrimidin-4-yl]amino]-N-[2-(2-hydroxyethylamino)ethyl]pyridine-3-carboxamide, MW: 473.931003 | MF: C22H25ClFN7O2.
   72: CID: 11951866, IUPAC: 4-[[2-(5-chloro-2-fluorophenyl)-5-pyrrolidin-1-ylpyrimidin-4-yl]amino]-N-(2-hydroxyethyl)pyridine-3-carboxamide, MW: 456.900483 | MF: C22H22ClFN6O2.
   73: CID: 11952045, IUPAC: 4-[[2-(5-chloro-2-fluorophenyl)-5-pyrrolidin-1-ylpyrimidin-4-yl]amino]-N-[(2S)-2-hydroxypropyl]pyridine-3-carboxamide, MW: 470.927063 | MF: C23H24ClFN6O2.
   74: CID: 15979866, IUPAC: 5-[2-[[4-(4-acetylpiperazin-1-yl)pyridin-2-yl]amino]-1,3-thiazol-5-yl]-N-methylpyridine-3-carboxamide, MW: 437.51802 | MF: C21H23N7O2S.
   75: CID: 15980109, IUPAC: N-(2-aminoethyl)-5-[2-[(4-morpholin-4-ylpyridin-2-yl)amino]-1,3-thiazol-5-yl]pyridine-3-carboxamide, MW: 425.50732 | MF: C20H23N7O2S
   76: CID: 15980233, IUPAC: N-(2-hydroxyethyl)-5-[2-[(4-morpholin-4-ylpyridin-2-yl)amino]-1,3-thiazol-5-yl]pyridine-3-carboxamide, MW: 426.49208 | MF: C20H22N6O3S.
   77: CID: 15980347, IUPAC: N-(2-methylaminoethyl)-5-[2-[(4-morpholin-4-ylpyridin-2-yl)amino]-1,3-thiazol-5-yl]pyridine-3-carboxamide, MW: 439.5339 | MF: C21H25N7O2S.
   78: CID: 15980351, IUPAC: 5-[2-[[4-[4-(2-hydroxyacetyl)piperazin-1-yl]pyridin-2-yl]amino]-1,3-thiazol-5-yl]-N-(2,2,2-trifluoroethyl)pyridine-3-carboxamide, MW: 521.51539 | MF: C22H22F3N7O3S.
   79: CID: 15982537, IUPAC: (3-chloro-2-fluorophenyl)-[4-[6-[(5-fluoro-1,3-thiazol-2-yl)amino]-5-methylpyridin-2-yl]piperazin-1-yl]methanone, MW: 449.904626 | MF: C20H18ClF2N5OS.
   80: CID: 16034848, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 2,3-dihydroxybutanedioic acid, MW: 638.09238 | MF: C26H32ClN7O8S.
   81: CID: 16037977, IUPAC: N-(2-chloro-6-methylphenyl)-2-[[6-[4-(2-hydroxyethyl)piperazin-1-yl]-5-methylpyrimidin-4-yl]amino]-1,3-thiazole-5-carboxamide, MW: 488.00554 | MF: C22H26ClN7O2S.
   82: CID: 16061431, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 4-[(4-methylpiperazin-1-yl)methyl]-N-[4-methyl-3-[(4-pyridin-3-ylpyrimidin-2-yl)amino]phenyl]benzamide, MW: 981.60828 | MF: C51H57CIN14O3S.
   83: CID: 16223227, IUPAC: but-2-enedioic acid; N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 604.0777 | MF: C26H30CIN7O6S.
   84: CID: 16223228, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrobromide, MW: 568.91748 | MF: C22H27BrClN7O2S.
   85: CID: 16223229, IUPAC: but-2-enedioic acid; N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 604.0777 | MF: C26H30ClN7O6S.
   86: CID: 16223316, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; methanesulfonic acid, MW: 584.1112 | MF: C23H30ClN7O5S2.
   87: CID: 16223317, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; phosphoric acid, MW: 586.000721 | MF: C22H29ClN7O6PS.
   88: CID: 16223318, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 2-hydroxybenzoic acid, MW: 626.12628 | MF: C29H32ClN7O5S.
   89: CID: 16223319, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; sulfuric acid, MW: 586.08402 | MF: C22H28ClN7O6S2.
   90: CID: 16223320, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 4-methylbenzenesulfonic acid, MW: 660.20716 | MF: C29H34ClN7O5S2.
   91: CID: 16584134, AKE-PB223730486, IUPAC: N-(4-chlorophenyl)-2-[(4,5-dimethyl-1,3-thiazol-2-yl)amino]-4-methylpyrimidine-5-carboxamide, MW: 373.85984 | MF: C17H16ClN5OS.
   92: CID: 16584137, AKE-PB223730492, IUPAC: N-(3-chlorophenyl)-2-[(4,5-dimethyl-1,3-thiazol-2-yl)amino]-4-methylpyrimidine-5-carboxamide, MW: 373.85984 | MF: C17H16ClN5OS.
   93: CID: 16584139, AKE-PB223730496, IUPAC: 2-[(4,5-dimethyl-1,3-thiazol-2-yl)amino]-4-methyl-N-(2-methylphenyl)pyrimidine-5-carboxamide, MW: 353.44136 | MF: C18H19N5OS. 94: CID: 16655683, IUPAC: 2-[(6-chloro-2-methylpyrimidin-4-yl)amino]-N-(2,6-dichlorophenyl)-1,3-thiazole-5-carboxamide, MW: 414.6968 | MF: C15H10Cl3N5OS.
   95: CID: 16655839, IUPAC: N-(2,6-dichlorophenyl)-2-[[6-[4-(2-hydroxyethyl)piperazin-1-yl]-2-methylpyrimidin-4-yl]amino]-1,3-thiazole-5-carboxamide, MW: 508.42402 | MF: C21H23Cl2N7O2S.
   96: CID: 16660745, IUPAC: N-(4-fluorophenyl)-4-(2-hydroxyethylamino)-6-methylsulfanyl-2-pyridin-4-ylpyrimidine-5-carboxamide, MW: 399.441923 | MF: C19H18FN502S.
   97: CID: 16660747, IUPAC: N-(4-ethylphenyl)-4-(2-hydroxyethylamino)-6-methylsulfanyl-2-pyridin-4-ylpyrimidine-5-carboxamide, MW: 409.50462 | MF: C21H23N5O2S.
   98: CID: 16660907, IUPAC: 4-(2-hydroxyethylamino)-N-(4-methylphenyl)-6-methylsulfanyl-2-pyridin-4-ylpyrimidine-5-carboxamide, MW: 395.47804 | MF: C20H21N5O2S.
   99: CID: 16661063, IUPAC: N-(4-chlorophenyl)-4-(2-hydroxyethylamino)-6-methylsulfanyl-2-pyridin-4-ylpyrimidine-5-carboxamide, MW: 415.89652 | MF: C19H18ClN5O2S.
   100: CID: 16661212, IUPAC: N-(2,4-dimethylphenyl)-4-(2-hydroxyethylamino)-6-methylsulfanyl-2-pyridin-4-ylpyrimidine-5-carboxamide, MW: 409.50462 | MF: C21H23N5O2S.
   101: CID: 16661214, IUPAC: 4-(1-hydroxybutan-2-ylamino)-N-(4-methylphenyl)-6-methylsulfanyl-2-pyridin-4-ylpyrimidine-5-carboxamide, MW: 423.5312 | MF: C22H25N5O2S.

Herein, CID is the compound identifier as defined in Pubchem.
e) Small molecules targeting and antagonising downstream components of the NEPOR signalling pathway, particularly EPH-B4 tyrosine kinase inhibitors.
f) Combination therapies involving one or more of approaches a-e.
g) The present disclosure also provides a combination therapy. Co-administration of EPO with an intracellular inhibitor of NEPOR signalling (e.g. Dasatinib) is proposed to maintain EPO signalling via EPOR (and thus promote haematopoiesis) while inhibiting survival of NEPOR positive tumour cells.

### NEPOR Based Therapeutics to Treat Neuronal Insults

Without being bound by theory, the present disclosure provides that EPO is neuroprotective because of its effects on NEPOR activity, i.e. in particular and/or EPH-B4 and/or Ephrin A1 activity. Therefore, the present disclosure provides a method for treating ischemic stroke, trauma, epilepsy, neurodegenerative diseases, and cognitive dysfunction with an agonistic NEPOR targeted therapy. Specific approaches to positively enhance NEPOR mediated survival signals include:
a) NEPOR specific antibodies. Such antibodies bind and initiate/enhance the mediation of NEPOR's cyto-protective activity.
b) NEPOR specific small-molecules. Such small molecules bind and initiate/enhance the mediation of NEPOR's cytoprotective activity.
c) NEPOR-targeting EPO mutants and glycosylated versions thereof. Due to EPOR's strict conformational requirements for mediating signalling in response to EPO, the following EPOR mutants (SEQ ID NO. 17 - SEQ ID NO. 212.) favour binding to NEPOR as opposed to EPOR and thus primarily act as tissue protective.

SEQ ID NO.17
SEQ ID NO.18
SEQ ID NO.19
SEQ ID NO.20
SEQ ID NO.21
   APPRLICDSRVLERYLLEAKEAENITTGCAEHCSLNENITVPPGVGQLFPAVGAPAAACG
SEQ ID NO.22
   APPRLICDSRVLERYLLEAKEAENITTGCAEHCSLNENNHC
SEQ ID NO.23
   APPRLICDSRVLERYLLEAKEAENIT
SEQ ID NO.24
   APPRLICDSRVLEAYLLEAKEAENIT
SEQ ID NO.25
   APPRLICDSRVLEEYLLEAKEAENIT
SEQ ID NO.26
   APPRLICDSRVLERYL
SEQ ID NO.27
   APPRLI
SEQ ID NO.28

Deletions of hWT 4 EPOR interaction sites
SEQ ID NO.29
SEQ ID NO.30
SEQ ID NO.31
SEQ ID NO.32

C-term deletions beginning at the last Cysteine bridge C161
SEQ ID NO.33
SEQ ID NO.34
SEQ ID NO.35
SEQ ID NO.36
SEQ ID NO.37
SEQ ID NO.38
SEQ ID NO.39
SEQ ID NO.40
SEQ ID NO.41
SEQ ID NO.42
SEQ ID NO.43
SEQ ID NO.44
SEQ ID NO.45
SEQ ID NO.46
SEQ ID NO.47
SEQ ID NO.48
SEQ ID NO.49
SEQ ID NO.50
SEQ ID NO.51
SEQ ID NO.52
SEQ ID NO.53
SEQ ID NO.54
SEQ ID NO.55
SEQ ID NO.56
SEQ ID NO.57
SEQ ID NO.58
SEQ ID NO.59
SEQ ID NO.60
SEQ ID NO.61
SEQ ID NO.62
SEQ ID NO.63
SEQ ID NO.64
SEQ ID NO.65
SEQ ID NO.66
SEQ ID NO.67
SEQ ID NO.68
SEQ ID NO.69
SEQ ID NO.70
SEQ ID NO.71
SEQ ID NO.72
SEQ ID NO.73
SEQ ID NO.74
SEQ ID NO.75
SEQ ID NO.76
SEQ ID NO.77
SEQ ID NO.78
SEQ ID NO.79
SEQ ID NO.80
SEQ ID NO.81
SEQ ID NO.82
SEQ ID NO.83
SEQ ID NO.84
SEQ ID NO.85
SEQ ID NO.86
SEQ ID NO.87
SEQ ID NO.88
SEQ ID NO.89
SEQ ID NO.90
SEQ ID NO.91
SEQ ID NO.92
SEQ ID NO.93
SEQ ID NO.94
SEQ ID NO.95
SEQ ID NO.96
SEQ ID NO.97
SEQ ID NO.98
SEQ ID NO.99
SEQ ID NO.100
SEQ ID NO.101
SEQ ID NO.102
SEQ ID NO.103
SEQ ID NO.104
SEQ ID NO.105
SEQ ID NO.106
SEQ ID NO.107
SEQ ID NO.108
SEQ ID NO.109
SEQ ID NO.110
SEQ ID NO.111
SEQ ID NO.112
SEQ ID NO.113
SEQ ID NO.114
SEQ ID NO.115
SEQ ID NO.116
   APPRLICDSRVLERYLLEAKEAENITTGCAEHCSLNENITVPDTKVNFYAWKRMEVGQQAVEVWQGLALLSEAVLRG
SEQ ID NO.117
   APPRLICDSRVLERYLLEAKEAENITTGCAEHCSLNENITVPDTKVNFYAWKRMEVGQQAVEVWQGLALLSEAVLR
SEQ ID NO.118
   APPRLICDSRVLERYLLEAKEAENITTGCAEHCSLNENITVPDTKVNFYAWKRMEVGQQAVEVWQGLALLSEAVL
SEQ ID NO.119
   APPRLICDSRVLERYLLEAKEAENITTGCAEHCSLNENITVPDTKVNFYAWKRMEVGQQAVEVWQGLALLSEAV
SEQ ID NO.120
   APPRLICDSRVLERYLLEAKEAENITTGCAEHCSLNENITVPDTKVNFYAWKRMEVGQQAVEVWQGLALLSEA
SEQ ID NO.121
   APPRLICDSRVLERYLLEAKEAENITTGCAEHCSLNENITVPDTKVNFYAWKRMEVGQQAVEVWQGLALLSE
SEQ ID NO.122
   APPRLICDSRVLERYLLEAKEAENITTGCAEHCSLNENITVPDTKVNFYAWXRMEVGQQAVEVWQGLALLS
SEQ ID NO.123
   APPRLICDSRVLERYLLEAKEAENITTGCAEHCSLNENITVPDTKVNFYAWKRMEVGQQAVEVWQGLALL
SEQ ID NO.124
   APPRLICDSRVLERYLLEAKEAENITTGCAEHCSLNENITVPDTKVNFYAWKRMEVGQQAVEVWQGLAL
SEQ ID NO.125
   APPRLICDSRVLERYLLEAKEAENITTGCAEHCSLNENITVPDTKVNFYAWKRMEVGQQAVEVWQGLA
SEQ ID NO.126
   APPRLICDSRVLERYLLEAKEAENITTGCAEHCSLNENITVPDTKVNFYAWKRMEVGQQAVEVWQGL
SEQ ID NO.127
   APPRLICDSRVLERYLLEAKEAENITTGCAEHCSLNENITVPDTKVNFYAWKRMEVGQQAVEVWQG
SEQ ID NO.128
   APPRLICDSRVLERYLLEAKEAENITTGCAEHCSLNENITVPDTKVNFYAWKRMEVGQQAVEVWQ
SEQ ID NO.129
   APPRLICDSRVLERYLLEAKEAENITTGCAEHCSLNENITVPDTKVNFYAWKRMEVGQQAVEVW
SEQ ID NO.130
   APPRLICDSRVLERYLLEAKEAENITTGCAEHCSLNENITVPDTKVNFYAWKRMEVGQQAVEV
SEQ ID NO.131
   APPRLICDSRVLERYLLEAKEAENITTGCAEHCSLNENITVPDTKVNFYAWKRMEVGQQAVE
SEQ ID NO.132
   APPRLICDSRVLERYLLEAKEAENITTGCAEHCSLNENITVPDTKVNFYAWKRMEVGQQAV
SEQ ID NO.133
   APPRLICDSRVLERYLLEAKEAENITTGCAEHCSLNENITVPDTKVNFYAWKRMEVGQQA
SEQ ID NO.134
   APPRLICDSRVLERYLLEAKEAENITTGCAEHCSLNENITVPDTKVNFYAWKRMEVGQQ
SEQ ID NO.135
   APPRLICDSRVLERYLLEAKEAENITTGCAEHCSLNENITVPDTKVNFYAWKRMEVGQ
SEQ ID NO.136
   APPRLICDSRVLERYLLEAKEAENITTGCAEHCSLNENITVPDTICVNFYAWKRMEVG
SEQ ID NO.137
   APPRLICDSRVLERYLLEAKEAENITTGCAEHCSLNENITVPDTKVNFYAWKRMEV
SEQ ID NO.138
   APPRLICDSRVLERYLLEAKEAENITTGCAEHCSLNENITVPDTKVNFYAWKRME
SEQ ID NO.139
   APPRLICDSRVLERYLLEAKEAENITTGCAEHCSLNENITVPDTKVNFYAWKRM
SEQ ID NO.140
   APPRLICDSRVLERYLLEAKEAENITTGCAEHCSLNENITVPDTKVNFYAWKR
SEQ ID NO.141
   APPRLICDSRVLERYLLEAKEAENITTGCAEHCSLNENITVPDTKVNFYAWK
SEQ ID NO.142
   APPRLICDSRVLERYLLEAKEAENITTGCAEHCSLNENITVPDTKVNFYAW
SEQ ID NO.143
   APPRLICDSRVLERYLLEAKEAENITTGCAEHCSLNENITVPDTKVNFYA
SEQ ID NO.144
   APPRLICDSRVLERYLLEAKEAENITTGCAEHCSLNENITVPDTKVNFY
SEQ ID NO.145
   APPRLICDSRVLERYLLEAKEAENITTGCAEHCSLNENITVPDTKVNF
SEQ ID NO.146
   APPRLICDSRVLERYLLEAKEAENITTC-CAEHCSLNENITVPDTKVN
SEQ ID NO.147
   APPRLICDSRVLERYLLEAKEAENITTGCAEHCSLNENITVPDTKV
SEQ ID NO.148
   APPRLICDSRVLERYLLEAKEAENITTGCAEHCSLNENITVPDTK
SEQ ID NO.149
   APPRLICDSRVLERYLLEAKEAENITTGCAEHCSLNENITVPDT
SEQ ID NO.150
   APPRLICDSRVLERYLLEAKEAENITTGCAEHCSLNENITVPD
SEQ ID NO.151
   APPRLICDSRVLERYLLEAKEAENITTGCAEHCSLNENITVP
SEQ ID NO.152
   APPRLICDSRVLERYLLEAKEAENITTGCAEHCSLNENITV
SEQ ID NO.153
   APPRLICDSRVLERYLLEAKEAENITTGCAEHCSLNENIT
SEQ ID NO.154
   APPRLICDSRVLERYLLEAKEAENITTGCAEHCSLNENI
SEQ ID NO.155
   APPRLICDSRVLERYLLEAKEAENITTGCAEHCSLNEN
SEQ ID NO.156
   APPRLICDSRVLERYLLEAKEAENITTGCAEHCSLNE
SEQ ID NO.157
   APPRLICDSRVLERYLLEAKEAENITTGCAEHCSLN
SEQ ID NO.158
   APPRLICDSRVLERYLLEAKEAENITTGCAEHCSL
SEQ ID NO.159
   APPRLICDSRVLERYLLEAKEAENITTGCAEHCS
SEQ ID NO.160
   APPRLICDSRVLERYLLEAKEAENITTGCAEHC
SEQ ID NO.161
   APPRLICDSRVLERYLLEAKEAENITTGCAEH
SEQ ID NO.162
   APPRLICDSRVLERYLLEAKEAENITTGCAE
SEQ ID NO.163
   APPRLICDSRVLERYLLEAKEAENITTGCA
SEQ ID NO.164
   APPRLICDSRVLERYLLEAKEAENITTGC
SEQ ID NO.165
   APPRLICDSRVLERYLLEAKEAENITTG
SEQ ID NO.166
   APPRLICDSRVLERYLLEAKEAENITT
SEQ ID NO.167
   APPRLICDSRVLERYLLEAKEAENIT
SEQ ID NO.168
   APPRLICDSRVLERYLLEAKEAENI
SEQ ID NO.169
   APPRLICDSRVLERYLLEAKEAEN
SEQ ID NO.170
   APPRLICDSRVLERYLLEAKEAE
SEQ ID NO.171
   AP PRLICDSRVLERYLLEAKEA
SEQ ID NO.172
   APPRLICDSRVLERYLLEAKE
SEQ ID NO.173
   APPRLICDSRVLERYLLEAK
SEQ ID NO.174
   APPRLICDSRVLERYLLEA
SEQ ID NO.175
   APPRLICDSRVLERYLLE
SEQ ID NO.176
   APPRLICDSRVLERYLL
SEQ ID NO.177
   APPRLICDSRVLERYL
SEQ ID NO.178
   APPRLICDSRVLERY
SEQ ID NO.179
   APPRLICDSRVLER
SEQ ID NO.180
   APPRLICDSRVLE
SEQ ID NO.181
   APPRLICDSRVL
SEQ ID NO.182
   APPRLICDSRV
SEQ ID NO.183
   APPRLICDSR
SEQ ID NO.184
   APPRLICDS
SEQ ID NO.185
   APPRLICD
SEQ ID NO.186
   APPRLIC

Single Amino Acid Mutations (Ala / Conversions) and all combinations/permutations thereof and all glycosylated versions of same. All possible combinations/permutations of mutations contained in Single mutations of SEQ ID NOs. 187-208 and glycosylated versions thereof.
SEQ ID NO.187
SEQ ID NO.188
SEQ ID NO.189
SEQ ID NO.190
SEQ ID NO.191
SEQ ID NO.192
SEQ ID NO.193
SEQ ID NO.194
SEQ ID NO.195
SEQ ID NO.196
SEQ ID NO.197
SEQ ID NO.198
SEQ ID NO.199
SEQ ID NO.200
SEQ ID NO.201
SEQ ID NO.202
SEQ ID NO.203
SEQ ID NO.204
SEQ ID NO.205
SEQ ID NO.206
SEQ ID NO.207
SEQ ID NO.208

EPO peptides overlapping interaction regions
SEQ ID NO.209
   APPRLICDSRVLERYLLEAKEAENITT
SEQ ID NO.210
   NENITVPDTKVNFYAWKRMEV
SEQ ID NO.211
   NSSQPWEPLQLHVDKAVSGLRSLTTLL
SEQ ID NO.212
   FRKLFRVYSNFLRGKLKL

d) NEPOR-targeting EPO chimera's. Such mutants bind and initiate/enhance the mediation of NEPOR's cytoprotective activity. For example, in a scenario where NEPOR constitutes an Ephrin A1 molecule (either as a homodimer or in heterodimeric association with EPOR), then chimeric proteins involving fusions of part of EPH-B4's Ephrin-ligand-binding domain and part of the EPO molecule may be developed as optimised binding partners. This implies fusing an N-terminal portion of EPO (derived from SEQ IDNO. 213) to a C-terminal portion of EPH-B4's Ephrin ligand binding domain (SEQ IDNO. 214), giving a sequence similar to SEQ IDNO. 215, or fusing an N-terminal portion of EPH-B4's Ephrin ligand binding domain (derived from SEQ ID NO. 214) to a C-terminal portion of EPO (SEQ ID NO. 213), giving a sequence similar to SEQ ID NO. 216.
e) high-affinity peptides which specifically target NEPOR to initiate/enhance the mediation of EPO's cytoprotective activity.
f) Small molecules targeting and enhancing the activity of downstream components of NEPOR.
g) Combination therapies involving one or more of approaches a-f.

SEQ ID NO.213
   >P01588|EPO_HUMAN Erythropoietin - Homo sapiens (Human).
SEQ ID NO.214
   >EPH-B4_ephrin_ligand_binding_domain
SEQ ID NO.215
   >NtermEPO_CtermEPHB4LBD
SEQ ID NO.216
   >NtermEPHB4LBD_CtermEPO

### Compounds in Combination with EPO

Such compounds, in combination with EPO, inhibit EPH-B4's tyrosine kinase activity while permitting EPOR mediated signalling/haematopoiesis. The following 101 compounds, either alone or in combination, inhibit the tyrosine kinase activity of EPH-B4 containing NEPOR dimers. Therefore, the present disclosure provides a combination therapeutic agent of a tyrosine kinase inhibitor in combination with EPO to provide the hematopoietic properties of EPO along with the prevention of NEPOR signalling so as to block the potentially fatal side effect of EPO to promote tumour survival and angiogenesis.
1: CID: 1095868, AKI-STT-00166305; ZINC00818264; BAS 09636496 IUPAC: N-[5-[(3-chlorophenyl)methyl]-1,3-thiazol-2-yl]-2-(4,6-dimethylpyrimidin-2-yl)sulfanylacetamide. MW: 404.93678 | MF: C18H17ClN4OS2. (MW is molecular weight and MF is molecular fonnula).
2: CID: 1465558, IUPAC: 2-[(3-chlorobenzoyl)amino]-4-methyl-N-pyridin-3-yl-1,3-thiazole-5-carboxamide, MW: 372.82872 | MF: C17H13ClN4O2S.
3: CID: 1468201, IUPAC: N-[5-[(2-chlorophenyl)carbamoyl]-4-methyl-1,3-thiazol-2-yl]pyridine-4-carboxamide, MW: 372.82872 | MF: C17H13ClN4O2S.
4: CID: 3062316, Dasatinib; Sprycel; BMS Dasatinib, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 488.00554 | MF: C22H26ClN7O2S.
5: CID: 3072360, 142287-40-9; Pyrimido(4,5-d)pyrimidin-4(1H)-one, 7-methyl-1-phenyl-2-((3-(4-(2-thiazolyl)-1-piperazinyl)propyl)thio)- IUPAC: 2-methyl-8-phenyl-7-[3-[4-(1,3-thiazol-2-yl)piperazin-1-yl]propylsulfanyl]pyrimido[6,5-d]pyrimidin-5-one, MW: 479.6209 | MF: C23H25N7OS2.
6: CID: 5041467, STK154706; ZINC04687922, IUPAC: [2-[(2-methylphenyl)amino]-1,3-thiazol-4-yl]-(4-pyrimidin-2-ylpiperazin-1-yl)methanone, MW: 380.4667 | MF: C19H20N6OS.
7: CID: 9822929, IUPAC: N-(2-chloro-6-methylphenyl)-2-[(6-imidazol-1-ylpyridazin-3-yl)amino]-1,3-thiazole-5-carboxamide, MW: 411.869 | MF: C18H14C1N7OS.
8: CID: 9927718, IUPAC: N-(2-chloro-6-methylphenyl)-2-(cyclopropanecarbonylamino)-1,3-thiazole-5-carboxamide, MW: 335.809 | MF: C15H14ClN3O2S.
9: CID: 10006113, IUPAC: N-[4-chloro-2-[(5-chloropyridin-2-yl)carbamoyl]phenyl]-5-methyl-6,7-dihydro-4H-[1,3]thiazolo[5,4-c]pyridine-2-carboxamide hydrochloride, MW: 498.81322 | MF: C20H18Cl3N5O2S.
10: CID: 10006114, IUPAC: N-[4-chloro-2-[(5-chloropyridin-2-yl)carbamoyl]phenyl]-5-methyl-6,7-dihydro-4H-[1,3]thiazolo[5,4-c]pyridine-2-carboxamide, MW: 462.35228 | MF: C20H17Cl2N5O2S.
11: CID: 10052635, IUPAC: 2-[[2-methyl-5-[[6-[(4-methylpiperazin-1-yl)methyl]pyridin-2-yl]amino]phenyl]amino]-N-(2-methylphenyl)-1,3-thiazole-5-carboxamide, MW: 527.68362 | MF: C29H33N7OS.
12: CID: 10195898, IUPAC: N-[(4-chlorophenyl)methyl]-2-[[[(2S)-2-hydroxy-2-pyrimidin-2-ylethyl]-methylamino]methyl]-4-methyl-7-oxothieno[2,3-e]pyridine-6-carboxamide, MW: 497.99706 | MF: C24H24ClN5O3S.
13: CID: 10206276, IUPAC: N-[4-[(5-chloropyridin-2-yl)carbamoyl]-2-phenyl-1,3-thiazol-5-yl]-1-propan-2-ylpiperidine-4-carboxamide, MW: 484.01354 | MF: C24H26ClN5O2S.
14: CID: 10252208, IUPAC: 2-[4-(5-amino-1,3-thiazol-2-yl)phenyl)-3-(5-chloropyridin-2-yl)quinazolin-4-one, MW: 431.89746 | MF: C22H14ClN5OS.
15: CID: 10253695, IUPAC: 2-[4-[3-(5-chloropyridin-2-yl)-4-oxoquinazolin-2-yl]phenyl]-1,3-thiazole-5-carboxamide, MW: 459.90756 | MF: C23H14ClN5O2S.
16: CID: 10301604, IUPAC: N-[4-[(5-chloropyridin-2-yl)carbamoyl]-2-(3,4-difluorophenyl)-1,3-thiazol-5-yl]-1-propan-2-ylpiperidine-4-carboxamide, MW: 519.994466 | MF: C24H24ClF2N5O2S.
17: CID: 10344807, IUPAC: N-[2-[4-[3-(5-chloropyridin-2-yl)-4-oxoquinazolin-2-yl]phenyl]-1,3-thiazol-4-yl]acetamide, MW: 473.93414 | MF: C24H16ClN5O2S.
18: CID: 10368624, IUPAC: N-[(4-chlorophenyl)methyl]-2-[[(2-hydroxy-2-pyrimidin-2-ylethyl)-methylamino]methyl]-7-methyl-4-oxothieno[3,2-e]pyridine-5-carboxamide, MW: 497.99706 | MF: C24H24ClN5O3S.
19: CID: 10370949, IUPAC: (3Z)-4-[[(2S)-2-(3-chlorophenyl)-2-hydroxyethyl]amino]-3-[6-methyl-2-[4-(1,3-thiazol-2-ylmethyl)piperazin-1-yl]-7,9-dihydropurin-8-ylidene]pyridin-2-one, MW: 578.08832 | MF: C27H28ClN9O2S.
20: CID: 10412586, IUPAC: N-[2-[4-[3-(5-chloropyridin-2-yl)-4-oxoquinazolin-2-yl]phenyl]-1,3-thiazol-5-yl]acetamide, MW: 473.93414 | MF: C24H16ClN5O2S.
21: CID: 10413555, IUPAC: N-[(4-chlorophenyl)methyl]-2-[[[(2R)-2-hydroxy-2-pyrimidin-2-ylethyl]-methylamino]methyl]-7-methyl-4-oxothieno[3,2-e]pyridine-5-carboxamide, MW: 497.99706 | MF: C24H24ClN5O3S.
22: CID: 10456156, IUPAC: 4-[(3-chlorothiophen-2-yl)methylamino]-2-[(4-morpholin-4-ylphenyl)amino]pyrimidine-5-carboxamide, MW: 444.93774 | MF: C20H21ClN6O2S.
23: CID: 10458706, IUPAC: N-[5-[2-[(4-chlorophenyl)amino]pyrimidin-4-yl]-4-methyl-1,3-thiazol-2-yl]-3-(2-morpholin-4-ylethylamino)propanamide, MW: 502.03212 | MF: C23H28ClN7O2S.
24: CID: 11153014, IUPAC: N-(2-chloro-6-methylphenyl)-2-[(2,6-dimethylpyrimidin-4-yl)amino]-1,3-thiazole-5-carboxamide, MW: 373.85984 | MF: C17H16ClN5OS.
25: CID: 11167695, IUPAC: N-(2-chloro-6-methylphenyl)-2-[[2-methyl-6-(2-morpholin-4-ylethylamino)pyrimidin-4-yl]amino]-1,3-thiazole-5-carboxamide, MW: 488.00554 | MF: C22H26ClN7O2S.
26: CID: 11168231, IUPAC: N-(2-chloro-6-methylphenyl)-2-[(6-chloro-2-methylpyrimidin-4-yl)amino]-N-[(4-methoxyphenyl)methyl]-1,3-thiazole-5-carboxamide, MW: 514.42684 | MF: C24H21Cl2N5O2S.
27: CID: 11200510, IUPAC: N-(2-chloro-6-methylphenyl)-2-[[6-(2-hydroxyethylamino)pyridin-2-yl]amino]-1,3-thiazole-5-carboxamide, MW: 403.88582 | MF: C18H18ClN5O2S.
28: CID: 11247793, IUPAC: N-(2-chloro-6-methylphenyl)-2-[[6-(methyl-(3-methylaminopropyl)amino)pyridin-2-yl]amino]-1,3-thiazole-5-carboxamide, MW: 444.9808 | MF: C21H25ClN6OS.
29: CID: 11260009, IUPAC: N-(2-chloro-6-methylphenyl)-2-[[6-[4-(hydroxymethyl)piperidin-1-yl]-2-methylpyrimidin-4-yl]amino]-1,3-thiazole-5-carboxamide, MW: 472.9909 | MF: C22H25ClN6O2S.
30: CID: 11269410, IUPAC: N-(2-chloro-6-methylphenyl)-2-[(6-chloro-2-methylpyrimidin-4-yl)amino]-1,3-thiazole-5-carboxamide, MW: 394.27832 | MF: C16H13Cl2N5OS.
31: CID: 11282881, IUPAC: N-(2-chloro-6-methylphenyl)-2-[[6-(2-morpholin-4-ylethylamino)pyrimidin-4-yl)amino]-1,3-thiazole-5-carboxamide, MW: 473.97896 | MF: C21H24CN7O2S.
32: CID: 11283174, IUPAC: N-(2-chloro-6-methylphenyl)-2-[[6-(3-morpholin-4-ylpropylamino)pyridin-2-yl]amino]-1,3-thiazole-5-carboxainide, MW: 487.01748 | MF: C23H27ClN6O2S.
33: CID: 11328827, IUPAC: N-(2-chloro-6-methylphenyl)-2-[[6-(3-imidazol-1-ylpropylamino)pyridin-2-yl]amino]-1,3-thiazole-5-carboxamide, MW: 467.97438 | MF: C22H22ClN7OS.
34: CID: 11407465, IUPAC: N-(2-chloro-6-methylphenyl)-2-[[6-(2-hydroxyethylamino)-2-methylpyrimidin-4-yl]amino]-1,3-thiazole-5-carboxamide, MW: 418.90046 | MF: C18H19ClN6O2S.
35: CID: 11466196, IUPAC: N-(2-chloro-6-methylphenyl)-2-[[2-methyl-6-(3-morpholin-4-ylpropylamino)pyrimidin-4-yl]amino]-1,3-thiazole-5-carboxamide. MW: 502.03212 | MF: C23H28ClN7O2S.
36: CID: 11466607, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrochloride, MW: 524.46648 | MF: C22H27Cl2N7O2S.
37: CID: 11487256, IUPAC: N-(2-chloro-6-methylphenyl)-2-[(6-morpholin-4-ylpyrimidin-4-yl)amino]-1,3-thiazole-5-carboxamide, MW: 430.91116 | MF: C19H19ClN6O2S.
38: CID: 11505502, IUPAC: 2-[[6-[4-(2-hydroxyethyl)piperazin-1-yl]pyrimidin-4-yl]amino]-N-[2-methyl-5-[[3-(trifluorometbyl)benzoyl]amino]phenyl]-1,3-thiazole-5-carboxamide. MW: 626.65257 | MF: C29H29F3N8O3S.
39: CID: 11512538, IUPAC: 2-[4-[6-[[5-[(2-chloro-6-methylphenyl)carbamoyl]-1,3-thiazol-2-yl]amino]-2-methylpyrimidin-4-yl]piperazin-1-yl]ethyl 2,2-dimethylpropanoate, MW: 572.12196 | MF: C27H34ClN7O3S.
40: CID: 11539665, IUPAC: (3-chloro-2-fluorophenyl)-[4-[[6-[(5-fluoro-1,3-thiazol-2-yl)amino]pyridin-2-yl]methyl]piperazin-1-yl]methanone, MW: 449.904626 | MF: C20H18ClF2N5OS.
41: CID: 11540687, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrate, MW: 506.02082 | MF: C22H28ClN7O3S.
42: CID: 11569328, IUPAC: N-(2-chloro-6-methylphenyl)-2-[[3-[4-(2-hydroxyethyl)piperazin-1-yl]-5-methylphenyl]amino]-1,3-thiazole-5-carboxamide, MW: 486.02942 | MF: C24H28ClN5O2S.
43: CID: 11570976, IUPAC: 2-[[6-[4-(2-hydroxyethyl)piperazin-1-yl]-2-methylpyrimidin-4-yl]amino]-N-[2-methyl-5-[[3-(trifluoromethyl)phenyl]carbamoyl]phenyl]-1,3-thiazole-5-carboxamide, MW: 640.67915 | MF: C30H31F3N8O3S.
44: CID: 11577776, IUPAC: 2-[[6-(2-hydroxyethylamino)-2-methylpyrimidin-4-yl]amino]-N-[2-methyl-5-[[3-(trifluoromethyl)benzoyl]amino]phenyl]-1,3-thiazole-5-carboxamide, MW: 571.57407 | MF: C26H24F3N7O3S.
45: CID: 11590089, IUPAC: (3-chloro-2-fluorophenyl)-[4-[5-methyl-6-(1,3-thiazol-2-ylamino)pyridin-2-yl]piperazin-1-yl]methanone, MW: 431.914163 | MF: C20H19ClFN5OS.
46: CID: 11606973, IUPAC: N-[5-[[3-[4-(2-hydroxyethyl)piperazin-1-yl]-5-(trifluoromethyl)benzoyl]amino]-2-methylphenyl]-2-(pyridin-2-ylamino)-1,3-thiazole-5-carboxamide, MW: 625.66451 | MF: C30H30F3N7O3S.
47: CID: 11650098, IUPAC: 2-[[6-(4-methylpiperazin-1-yl)pyrimidin-4-yl]amino]-N-[2-methyl-5-[[3-(trifluoromethyl)benzoyl]amino]pheny1]-1,3-thiazole-5-carboxamide,MW: 596.62659 | MF: C28H27F3N8O2S.
48: CID: 11650132, IUPAC: pentyl N-[5-[(2-chloro-6-methylphenyl)carbamoyl]-1,3-thiazol-2-yl]-N-[6-[4-(2-hydroxyethyl)piperazin-1-yl]-2-methylpyrimidin-4-yl]carbamate, MW: 602.14794 | MF: C28H36ClN7O4S.
49: CID: 11650511, IUPAC: N-[5-[[3-(4-ethylpiperazin-1-yl)-5-(trifluoromethyl)benzoyl]amino]-2-methylphenyl]-2-[[6-(2-hydroxyethylamino)-2-methylpyrimidin-4-yl]amino]-1,3-thiazole-5-carboxamide, MW: 683.74695 | MF: C32H36F3N9O3S.
50: CID: 11664355, IUPAC: 2-[(2-methyl-6-morpholin-4-ylpyrimidin-4-yl)amino]-N-[2-methyl-5-[[3-(trifluoromethyl)benzoyl]amino]phenyl]-1,3-thiazole-5-carboxamide, MW: 597.61135 | MF: C28H26F3N7O3S.
51: CID: 11664511, IUPAC: 2-[[4-[4-(2-hydroxyethyl)piperazin-1-yl]pyridin-2-yl]amino]-N-[2-methyl-5-[[3-(trifluoromethyl)benzoyl]amino]phenyl]-1,3-thiazole-5-carboxamide, MW: 625.66451 | MF: C30H30F3N7O3S.
52: CID: 11669430, IUPAC: N-(2-chloro-6-methylphenyl)-2-[(2-methyl-6-piperazin-1-ylpyrimidin-4-yl)amino]-1,3-thiazole-5-carboxamide, MW: 443.95298 | MF: C20H22ClN7OS.
53: CID: 11676373, IUPAC: (3-chloro-2-fluorophenyl)-[4-[[6-(1,3-thiazol-2-ylamino)pyridin-2-yl]methyl]piperazin-1-yl]methanone, MW: 431.914163 | MF: C20H19ClFN5OS.
54: CID: 11684148, IUPAC: (3-chloro-2-fluorophenyl)-[4-[[6-[(5-chloro-1,3-thiazol-2-yl)amino]pyridin-2-yl]methyl]piperazin-1-yl]methanone, MW: 466.359223 | MF: C20H18Cl2FN5OS.
55: CID: 11700117, IUPAC: 2-[[6-(4-ethylpiperazin-1-yl)-2-methylpyrimidin-4-yl]amino]-N-[2-methyl-5-[[3-(trifluoromethyl)benzoyl]amino]phenyl]-1,3-thiazole-5-carboxamide, MW: 624.67975 | MF: C30H31F3N8O2S.
56: CID: 11707091, IUPAC: 2-[[2-methyl-6-(4-methylpiperazin-1-yl)pyrimidin-4-yl]amino]-N-[2-methyl-5-[[3-(trifluoromethyl)benzoyl]amino]phenyl]-1,3-thiazole-5-carboxamide, MW: 610.65317 | MF: C29H29F3N8O2S.
57: CID: 11714286, IUPAC: 2-[[5-[4-(2-hydroxyethyl)piperazin-1-yl]pyridin-2-yl]amino]-N-[2-methyl-5-[[3-(trifluoromethyl)benzoyl]amino]phenyl]-1,3-thiazole-5-carboxamide, MW: 625.66451 | MF: C30H30F3N7O3S.
58: CID: 11714353, IUPAC: 2-[[6-[4-(2-hydroxyethyl)piperazin-1-yl]-2-methylpyrimidin-4-yl]amino]-N-[2-methyl-5-[[3-(trifluoromethyl)benzoyl]amino]phenyl]-1,3-thiazole-5-carboxamide, MW: 640.67915 | MF: C30H31F3N8O3S.
59: CID: 11752136, IUPAC: N-(2-chloro-6-methylphenyl)-2-[[5-[4-(2-hydroxyethyl)piperazin-1-yl]-2-methylpyrimidin-4-yl]amino]-1,3-thiazole-5-carboxamide, MW: 488.00554 | MF: C22H26ClN7O2S.
60: CID: 11772766, IUPAC: 4-[2-(3-chlorophenyl)ethylamino]-2-pyridin4-yl-1,3-thiazole-5-carboxamide, MW: 358.8452 | MF: C17H15ClN4OS.
61: CID: 11775143, IUPAC: N-(2-chloro-6-methylphenyl)-2-[(2-methyl-6-morpholin-4-ylpyrimidin-4-yl)amino]-1,3-thiazole-5-carboxamide, MW: 444.93774 | MF: C20H21ClN6O2S.
62: CID: 11854012, IUPAC: 2-[4-[6-[[5-[(2-chloro-6-methylphenyl)carbamoyl]-1,3-thiazol-2-yl]amino]-2-methylpyrimidin-4-yl]piperazin-1-yl]acetic acid, MW: 501.98906 | MF: C22H24ClN7O3 S.
63: CID: 11854269, IUPAC: 2-[4-[6-[[5-[(2-chloro-6-methylphenyl)carbamoyl]-1,3-thiazol-2-yl]amino]-2-methylpyrimidin-4-yl]piperazin-1-yl]ethyl hydrogen sulfate, MW: 568.06874 | MF: C22H26ClN7O5S2.
64: CID: 11854270, IUPAC: N-(2-chloro-6-methylphenyl)-2-[[6-[2-(2-hydroxyethylamino)ethylamino]-2-methylpyrimidin-4-yl]amino]-1,3-thiazole-5-carboxamide, MW: 461.96826 | MF: C20H24ClN7O2S
65: CID: 11854271, IUPAC: 2-[[6-(2-aminoethylamino)-2-methylpyrimidin-4-yl]amino]-N-(2-chloro-6-methylphenyl)-1,3-thiazole-5-carboxamide, MW: 417.9157 | MF: C18H20ClN7OS.
66: CID: 11854272, IUPAC: 2-[[2-[4-[6-[[5-[(2-chloro-6-methylphenyl)carbamoyl]-1,3-thiazol-2-yl]amino]-2-methylpyrimidin-4-yl]piperazin-1-yl]acetyl]amino]ethanesulfonic acid, MW: 609.12066 | MF: C24H29ClN8O5S2.
67: CID: 11854533, IUPAC: N-(2-chloro-4-hydroxy-6-methylphenyl)-2-[[6-[4-(2-hydroxyethyl)piperazin-1-yl]-2-methylpyrimidin-4-yl]amino]-1,3-thiazole-5-carboxamide, MW: 504.00494 | MF: C22H26ClN7O3S.
68: CID: 11854534, IUPAC: N-[2-chloro-6-(hydroxymethyl)phenyl]-2-[[6-[4-(2-hydroxyethyl)piperazin-1-yl]-2-methylpyrimidin-4-yl]amino]-1,3-thiazole-5-carboxamide, MW: 504.00494 | MF: C22H26ClN7O3S.
69: CID: 11854535, IUPAC: N-(2-chloro-6-methylphenyl)-2-[[6-[4-(2-hydroxyethyl)-4-oxidopiperazin-4-ium-1-yl]-2-methylpyrimidin-4-yl]amino]-1,3-thiazole-5-carboxamide, MW: 504.00494 | MF: C22H26ClN7O3S.
70: CID: 11854536, IUPAC: 2-[4-[6-[[5-[(2-chloro-6-methylphenyl)carbamoyl]-1,3-thiazol-2-yl]amino]-2-methylpyrimidin-4-yl]-1-oxidopiperazin-1-ium-1-yl]acetic acid, MW: 517.98846 | MF: C22H24ClN7O4S.
71: CID: 11949914, IUPAC: 4-[[2-(5-chloro-2-fluorophenyl)-5-dimethylaminopyrimidin-4-yl]amino]-N-[2-(2-hydroxyethylamino)ethyl]pyridine-3-carboxamide, MW: 473.931003 | MF: C22H25ClFN7O2.
72: CID: 11951866, IUPAC: 4-[[2-(5-chloro-2-fluorophenyl)-5-pyrrolidin-1-ylpyrimidin-4-yl]amino]-N-(2-hydroxyethyl)pyridine-3-carboxamide, MW: 456.900483 | MF: C22H22ClFN6O2.
73: CID: 11952045, IUPAC: 4-[[2-(5-chloro-2-fluorophenyl)-5-pyrrolidin-1-ylpyrimidin-4-yl]amino]-N-[(2S)-2-hydroxypropyl]pyridine-3-carboxamide, MW: 470.927063 | MF: C23H24ClFN6O2.
74: CID: 15979866, IUPAC: 5-[2-[[4-(4-acetylpiperazin-1-yl)pyridin-2-yl]amino]-1,3-thiazol-5-yl]-N-methylpyridine-3-carboxamide, MW: 437.51802 | MF: C21H23N7O2S.
75: CID: 15980109, IUPAC: N-(2-aminoethyl)-5-[2-[(4-morpholin-4-ylpyridin-2-yl)amino]-1,3-thiazol-5-yl]pyridine-3-carboxamide, MW: 425.50732 | MF: C20H23N7O2S
76: CID: 15980233, IUPAC: N-(2-hydroxyethyl)-5-[2-[(4-morpholin-4-ylpyridin-2-yl)amino]-1,3-thiazol-5-yl]pyridine-3-carboxamide, MW: 426.49208 | MF: C20H22N6O3S.
77: CID: 15980347, IUPAC: N-(2-methylaminoethyl)-5-[2-[(4-morpholin-4-ylpyridin-2-yl)amino]-1,3-thiazol-5-yl]pyridine-3-carboxamide, MW: 439.5339 | MF: C21H25N7O2S.
78: CID: 15980351, IUPAC: 5-[2-[[4-[4-(2-hydroxyacetyl)piperazin-1-yl]pyridin-2-yl]amino]-1,3-thiazol-5-yl]-N-(2,2,2-trifluoroethyl)pyridine-3-carboxamide, MW: 521.51539 | MF: C22H22F3N7O3S.
79: CID: 15982537, IUPAC: (3-chloro-2-fluorophenyl)-[4-[6-[(5-fluoro-1,3-thiazol-2-yl)amino]-5-methylpyridin-2-yl]piperazin-1-yl]methanone, MW: 449.904626 | MF: C20H18ClF2N5OS.
80: CID: 16034848, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 2,3-dihydroxybutanedioic acid, MW: 638.09238 | MF: C26H32ClN7O8S.
81: CID: 16037977, IUPAC: N-(2-chloro-6-methylphenyl)-2-[[6-[4-(2-hydroxyethyl)piperazin-1-yl]-5-methy]pyrimidin-4-yl]amino]-1,3-thiazole-5-carboxamide, MW: 488.00554 | MF: C22H26ClN7O2S.
82: CID: 16061431, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 4-[(4-methylpiperazin-1-yl)methyl]-N-[4-methyl-3-[(4-pyridin-3-ylpyrimidin-2-yl)amino]phenyl]benzamide, MW: 981.60828 | MF: C51H57ClN14O3S.
83: CID: 16223227, IUPAC: but-2-enedioic acid; N-(2-chloro-6-methylphenyl)-2-[[6-[4-(2-hydroxyethyl)piperazin-1-yl)-2-methylpyrimidin-4-yl)amino]-1,3-thiazole-5-carboxamide, MW: 604.0777 | MF: C26H30ClN7O6S.
84: CID: 16223228, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide hydrobromide, MW: 568.91748 | MF: C22H27BrClN7O2S.
85: CID: 16223229, IUPAC: but-2-enedioic acid; N-(2-chloro-6-methylphenyl)-2-[[6-[4-(2-hydroxyethyl)piperazin-1-yl]-2-methylpyrimidin-4-yl]amino]-1,3-thiazole-5-carboxamide, MW: 604.0777 | MF: C26H30ClN7O6S.
86: CID: 16223316, IUPAC: N-(2-chloro-6-methylphenyl)-2-[[6-[4-(2-hydroxyetbyl)piperazin-1-yl]-2-methylpyrimidin-4-yl]amino]-1,3-thiazole-5-carboxamide; methanesulfonic acid, MW: 584.1112 | MF: C23H30ClN7O5S2.
87: CID: 16223317, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; phosphoric acid, MW: 586.000721 | MF: C22H29ClN7O6PS.
88: CID: 16223318, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 2-hydroxybenzoic acid, MW: 626.12628 | MF: C29H32ClN7O5S.
89: CID: 16223319, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; sulfuric acid, MW: 586.08402 | MF: C22H28ClN7O6S2.
90: CID: 16223320, IUPAC: N-(2-chloro-6-methylphenyl)-2-[[6-[4-(2-hydroxyethyl)piperazin-1-yl]-2-methylpyrimidin-4-yl]amino]-1,3-thiazole-5-carboxamide; 4-methylbenzenesulfonic acid, MW: 660.20716 | MF: C29H34ClN7O5S2.
91: CID: 16584134, AKE-PB223730486, IUPAC: N-(4-chlorophenyl)-2-[(4,5-dimethyl-1,3-thiazol-2-yl)amino]-4-methylpyrimidine-5-carboxamide, MW: 373.85984 | MF: C17H16ClN5OS.
92: CID: 16584137, AKE-PB223730492, IUPAC: N-(3-chlorophenyl)-2-[(4,5-dimethyl-1,3-thiazol-2-yl)amino]-4-methylpyrimidine-5-carboxamide, MW: 373.85984 | MF: C17H16ClN5OS.
93: CID: 16584139, AKE-PB223730496, IUPAC: 2-[(4,5-dimethyl-1,3-thiazol-2-yl)amino]-4-methyl-N-(2-methylphenyl)pyrimidine-5-carboxamide, MW: 353.44136 | MF: C18H19N5OS.
94: CID: 16655683, IUPAC: 2-[(6-chloro-2-methylpyrimidin-4-yl)amino]-N-(2,6-dichlorophenyl)-1,3-thiazole-5-carboxamide, MW: 414.6968 MF: C15H10Cl3N5OS.
95: CID: 16655839, IUPAC: N-(2,6-dichlorophenyl)-2-[[6-[4-(2-hydroxyethyl)piperazin-1-yl]-2-methylpyrimidin-4-yl]amino]-1,3-thiazole-5-carboxamide, MW: 508.42402 | MF: C21H23Cl2N7O2S.
96: CID: 16660745, IUPAC: N-(4-fluorophenyl)-4-(2-hydroxyethylamino)-6-methylsulfanyl-2-pyridin-4-ylpyrimidine-5-carboxamide, MW: 399.441923 | MF: C19H18FN5O2S.
97: CID: 16660747, IUPAC: N-(4-ethylphenyl)-4-(2-hydroxyethylamino)-6-methylsulfanyl-2-pyridin-4-ylpyrimidine-5-carboxamide, MW: 409.50462 | MF: C21H23N5O2S.
98: CID: 16660907, IUPAC: 4-(2-hydroxyethylamino)-N-(4-methylphenyl)-6-methylsulfanyl-2-pyridin-4-ylpyrimidine-5-carboxamide, MW: 395.47804 MF: C20H21N5O2S.
99: CID: 16661063, IUPAC: N-(4-chlorophenyl)-4-(2-hydroxyethylamino)-6-methylsulfanyl-2-pyridin-4-ylpyrimidine-5-carboxamide, MW: 415.89652 | MF: C19H18ClN5O2S.
100: CID: 16661212, IUPAC: N-(2,4-dimethylphenyl)-4-(2-hydroxyethylamino)-6-methylsulfanyl-2-pyridin-4-ylpyrimidine-5-carboxamide, MW: 409.50462 | MF: C21H23N5O2S.
101: CID: 16661214, IUPAC: 4-(1-hydroxybutan-2-ylamino)-N-(4-methylphenyl)-6-methylsulfanyl-2-pyridin-4-ylpyrimidine-5-carboxamide, MW: 423.5312 | MF: C22H25N5O2S.

### NEPOR: Combined Prognostic and Therapeutic Value in Cancer Treatment.

Without being bound by theory, the observation that EPO treated patients often have poorer survival outcomes (at least in some cancers) means that treatment of these patients with a NEPOR targeted therapy provides a pharmacogenetic approach to targeted cancer treatment providing tumour tissue can be assessed for expression of NEPOR. Such a therapeutic perspective changes the balance in favour of performing biopsies under all suitable circumstances - meaning for cancers where EPOR, EPH-B4 and/or EphrinA1 are typically expressed.

The present disclosure further provides a method for imaging tumour tissue that is susceptible to enhanced survival in response to EPO treatment, comprising administering an anti-NEPOR antibody or NEPOR binding peptide linked to a radio-ligand or other imaging agent, and measuring for tissue distribution and location of the radio-ligand or other imaging agent.

If a tumour is NEPOR positive, then EPO is contraindicated and a NEPOR targeted therapy is administered. If NEPOR is not present, then it is safe to administer EPO. Both outcomes stand to benefit patient outcome, regardless of whether a patient is NEPOR positive or negative. Again, this shifts the balance in favour of performing routine biopsies.

In one embodiment the disclosure relates to an siRNA molecule specific to EPH-B4 and/or Ephrin A1 for use in treating a cancer patient that is or will receive EPO. EPH-B4 siRNAs and Antisense Oligodeoxynucleotides

Various EphB4-specific anti-sense phosphorothioate-modified oligodeoxynucleotides (ODNs) and siRNA may be synthesized from (e.g. by Qiagen. The most active antisense ODN and siRNA that knocks down EphB4 expression in the transiently transfected 293T cell line is chosen. The antisense ODN that may be used is AS-10 which spans nucleotides 1980 to 1999 with a sequence 5'-ATG GAG GCC TCG CTC AGA AA-3' (SEQ ID NO. 217). To eliminate cytokine responses, the cytosine at the CpG site may be methylated (AS-10M) without any loss in EphB4 knockdown efficiency (data not shown). Scrambled ODNs containing random nucleotide sequence and a similar CpG site, 5'-TAC CTG AAG GTC AGG CGA AC-3' (SEQ ID NO. 218), may be used as control. siRNA 465 corresponding to the sequences 5'-GGU GAA UGU CAA GAC GCU GUU-3' (SEQ ID NO. 219) and 3'-UUC CAC UUA CAG UUC UGC GAC-5' (SEQ ID NO. 220) may be used for RNA interference. Control siRNA may be generated by mutating three bases in this sequence to effectively abrogate EphB4 knockdown. This mutated siRNA (siRNAΔ) had the sequences 5'-AGU UAA UAU CAA GAC GCU GUU-3' (SEQ ID NO. 221) and 3'-UUU CAA UUA UAG UUC UGC GAC-5' (SEQ ID NO. 222). Additionally, siRNA directed against green fluorescent protein with sequences 5'-CGC UGA CCC UGA AGU UCA TUU-3' (SEQ ID NO. 223) and 3'-UUG CGA CUG GGA CUU CAA GUA-5' (SEQ ID NO. 224) may be used as a negative control.

siRNA may be obtained from numerous companies in particular Sigma Aldrich:
The siRNA's from the sigma catalogue predicted using Rosetta siRNA design algorithm

**EPHRIN A1**

| siRNA_ID | entrezgene_ID | approx_start_nucleotide |
|---|---|---|
| SASI_Hs01_00211016 | NM_004428 | 247 |
| SASI_Hs01_00211017 | NM_004428 | 223 |
| SASI_Hs01_00211018 | NM_004428 | 248 |
| SASI_Hs01_00211019 | NM_004428 | 1071 |
| SASI_Hs01_00211020 | NM_004428 | 256 |
| SASI_Hs01_00211021 | NM_004428 | 208 |
| SASI_Hs01_00211022 | NM_004428 | 829 |
| SASI_Hs01_00211023 | NM_004428 | 1015 |
| SASI_Hs01_00211024 | NM_004428 | 846 |
| SASI_Hs01_00211025 | NM_004428 | 225 |
| SASI_Hs01_00071683 | NM_182685 | 248 |
| SASI_Hs01_00071684 | NM_182685 | 214 |
| SASI_Hs01_00071685 | NM_182685 | 242 |
| SASI_Hs01_00071686 | NM_182685 | 1000 |
| SASI_Hs01_00071687 | NM_182685 | 263 |
| SASI_Hs01_00071688 | NM_182685 | 203 |
| SASI_Hs01_00071689 | NM_182685 | 769 |
| SASI_Hs01_00071690 | NM_182685 | 948 |
| SASI_Hs01_00071691 | NM_182685 | 778 |
| SASI_Hs01_00071692 | NM_182685 | 227 |

**EPHB4**

| siRNA_ID | entrezgene_ID | approx_start_nucleotide |
|---|---|---|
| SASI_Hs01_00039855 | NM_004444 | 1756 |
| SASI_Hs01_00039856 | NM_004444 | 577 |
| SASI_Hs01_00039857 | NM_004444 | 1373 |
| SASI_Hs01_00039858 | NM_004444 | 2290 |
| SASI_Hs01_00039859 | NM_004444 | 2318 |
| SASI_Hs01_00039860 | NM_004444 | 2353 |
| SASI_Hs01_00039861 | NM_004444 | 2898 |
| SASI_Hs01_00039862 | NM_004444 | 2245 |
| SASI_Hs01_00039863 | NM_004444 | 1679 |
| SASI_Hs01_00039864 | NM_004444 | 1416 |

### Antibodies to NEPOR

The present disclosure includes several antibodies that bind to NEPOR components. The following Table 6 provides a list of such antibodies and their availability.

**Table 6**

| ***Company*** | ***Item*** | ***Antigen*** | ***Catalog Number*** | ***Applications*** | ***Type*** |
|---|---|---|---|---|---|
| | ***EPOR*** | | | | |
| Abcam | Goat Anti-EPO Receptor Polyclonal Antibody, Unconjugated | EPOR | ab10653 | ELISA, WB | polyclonal |
| ABR-Affinity BioReagents | Mouse Anti-Human EPOR Monoclonal Antibody, Unconjugated, Clone 3D10 | EPOR | MA1-51823 | WB, ELISA | Monoclonal |
| Abnova Corporation | Mouse Anti-Human EPOR Monoclonal Antibody, Unconjugated, Clone 3D10 | EPOR | H00002057-M01 | WB, Capture ELISA | Monoclonal |
| Abcam | Goat Anti-Human EPO Receptor Polyclonal Antibody, Unconjugated | EPOR | ab27497 | ELISA, WB | Polyclonal |
| Abcam | Mouse Anti-Human EPO Receptor Monoclonal Antibody, Unconjugated, Clone MM-0031-6G7 | EPOR | ab56310 | WB | Monoclonal |
| ABR-Affinity BioReagents | Mouse Anti-Human EPOR Polyclonal Antibody, Unconjugated | EPOR | PA1-51822 | WB | Polyclonal |
| IMGENEX | Goat Anti-Human EPOR Polyclonal Antibody, Unconjugated | EPOR | IMG-3771 | WB, ELISA | Polyclonal |
| Lifespan Biosciences | Rabbit Anti-Human Erythropoietin Receptor (EPOR) Polyclonal Antibody, Unconjugated | EPOR | LS-C6720 | ELISA | Polyclonal |
| GeneTex | Mouse Anti-Human EPOR Monoclonal Antibody, Unconjugated, Clone 3D10 | EPOR | GTX91710 | ELISA, WB | Monoclonal |
| Lifespan Biosciences | Rabbit Anti-Human EPOR Polyclonal Antibody, Unconjugated | EPOR | LS-C6719-100 | ELISA | Polyclonal |
| Novus Biologicals | Mouse Anti-Human EPOR Polyclonal Antibody, Unconjugated | EPOR | H00002057-A01 | | Polyclonal |
| Novus Biologicals | Mouse Anti-Human EPOR Monoclonal Antibody, Unconjugated, Clone 3D10 | EPOR | H00002057-M01 | ELISA, WB | Monoclonal |
| Lifespan Biosciences | Sheep Anti-Human Erythropoietin Receptor (EPOR) Polyclonal Antibody, Unconjugated | EPOR | LS-C6718 | ELISA, WB | Polyclonal |
| Lifespan Biosciences | Sheep Anti-Human Erythropoietin Receptor (EPOR) Polyclonal Antibody, Unconjugated | EPOR | LS-C6716 | | Polyclonal |
| Lifespan Biosciences | Sheep Anti-Human EPOR Polyclonal Antibody, Unconjugated | EPOR | LS-C6717-50 | ELISA | Polyclonal |
| Santa Cruz Biotechnology, Inc. | Rabbit Anti-Human EpoR (C-20) Polyclonal Antibody, Unconjugated | EpoR (C-20) | sc-695 | WB, IP, IF, ICH. | Polyclonal |
| Santa Cruz Biotechnology, Inc. | Rabbit Anti-EpoR (M-20) Polyclonal Antibody, Unconjugated | EpoR (M-20) | sc-697 | WB, IP, IF, ICH. | Polyclonal |
| Santa Cruz Biotechnology, Inc. | Rabbit Anti-EpoR Polyclonal Antibody, Unconjugated | EpoR | sc-5624 | WB, IP, IF, ICH. | Polyclonal |

| | ***EPH-B4*** | | | | |
|---|---|---|---|---|---|
| Abgent | Rabbit Anti-EPH-B4 C-term RB1659-1660 Polyclonal Antibody, Unconjugated | EPH-B4 C-term | AP7625a | ELISA; IHC. | Polyclonal |
| ABR-Affinity BioReagents | Rabbit Anti-Human EPH-B4 Polyclonal Antibody, Unconjugated | EPH-B4 | PA1-24241 | WB | Polyclonal |
| ABR-Affinity BioReagents | Mouse Anti-Human EPH-B4 Monoclonal Antibody, Unconjugated, Clone 1D1 | EPH-B4 | MA1-51815 | ELISA | Monoclonal |
| AbD Serotec | Human Anti-Human EPH-B4 Monoclonal Antibody, Unconjugated, Clone 1327 | EPH-B4 | HCA001 | IHC, WB, ELISA | Monoclonal |
| AbD Serotec | Human Anti-Human EPH-B4 Monoclonal Antibody, Unconjugated, Clone 3934 | EPH-B4 | HCA025 | IHC, WB, ELISA | Monoclonal |
| Invitrogen | Anti-EPH-B4 receptor Monoclonal Antibody, Unconjugated, Clone 3D7F8 | EPH-B4 | 35-2900 | WB, ELISA | Monoclonal |
| GeneTex | Rabbit Anti-EPH-B4 Polyclonal Antibody, Unconjugated | EPH-B4 | GTX77656 | WB | Polyclonal |
| Invitrogen | Mouse Anti-EPH-B4 Receptor Monoclonal Antibody,, Clone 3D7G8 | EPH-B4 | 182394 | IHC(FFPE) | Monoclonal |
| Invitrogen | Anti-Eph Receptor Sampler Pack Antibody, | EPH-B4 | 901100 | | |
| GeneTex | Mouse Anti-Human EPH-B4 Monoclonal Antibody, Unconjugated, Clone 1D1 | EPH-B4 | GTX91629 | ELISA | Monoclonal |
| Invitrogen | Mouse Anti-Human EPH-B4 Receptor Monoclonal Antibody,, Clone 3D7G8 | EPH-B4 | 371800 | WB ELISA IP, IHC | Monoclonal |
| Novus Biologicals | Mouse Anti-Human EPH-B4 Monoclonal Antibody, Unconjugated, Clone 1D1 | EPH-B4 | H00002050-M01 | | Monoclonal |
| R&D Systems | Goat Anti-Human EPH-B4 Polyclonal Antibody, Unconjugated | EPH-B4 | AF3038 | FC, IHC, WB | Polyclonal |
| Raybiotech, Inc. | Human Anti-Human EPH-B4, (packaged with HRP-Conjugated Secondary Antibody); Monoclonal Antibody, Unconjugated | EPH-B4 | DS-MB-01224 | | Monoclonal |
| R&D Systems | Rat Anti-Human EPH-B4 Monoclonal Antibody, Unconjugated, Clone 395810 | EPH-B4 | MAB3038 | FC, IHC, WB | Monoclonal |
| Santa Cruz Biotechnology, Inc. | Goat Anti-EPH-B4 Polyclonal Antibody, Unconjugated | EPH-B4 | sc-7284 | WB, IF | Polyclonal |
| Santa Cruz | Goat Anti-EPH-B4 Polyclonal Antibody, | EPH-B4 | sc-7285 | WB, IF | Polyclonal |
| Biotechnology, Inc. | Unconjugated | | | | |
| Santa Cruz Biotechnology, Inc. | Rabbit Anti-EPH-B4 Polyclonal Antibody, Unconjugated | EPH-B4 | sc-5536 | WB, IF | Polyclonal |
| Raybiotech, Inc. | Human Anti-Human EPH-B4, (packaged with HRP-Conjugated Secondary Antibody); Monoclonal Antibody, Unconjugated | EPH-B4 | DS-MB-01225 | | Monoclonal |

| | ***EFNA1*** | | | | |
|---|---|---|---|---|---|
| Invitrogen | Anti-Ephrin A1 Polyclonal Antibody, Unconjugated, Clone ZMD.39 | EFNA1 | 34-3300 | | Polyclonal |
| Novus Biologicals | Mouse Anti-Human EFNA1 Monoclonal Antibody, Unconjugated, Clone 3C6 | EFNA1 | H00001942-M01 | | Monoclonal |
| Santa Cruz Biotechnology, Inc. | Rabbit Anti-ephrin-A1 (V-18) Polyclonal Antibody, Unconjugated | EFNA1 (V-18) | sc-911 | WB, IF | Polyclonal |
| Santa Cruz Biotechnology, Inc. | Rabbit Anti-ephrin-A1 Polyclonal Antibody, Unconjugated | EFNA1 | sc-20719 | WB, IP, IF | Polyclonal |
| Abcam | Rabbit Anti-Human Ephrin A1 Receptor Polyclonal Antibody, Unconjugated | EFNA1 | ab37857 | ELISA, IHC, WB | Polyclonal |
| GeneTex | Mouse Anti-Human EFNA1 Monoclonal Antibody, Unconjugated, Clone 3C7 | EFNA1 | GTX91614 | | Monoclonal |

| | ***EFNB2*** | | | | |
|---|---|---|---|---|---|
| Novus Biologicals | Mouse Anti-Human EFNB2 Polyclonal Antibody, Unconjugated | EFNB2 | H00001948-A01 | | Polyclonal |
| Santa Cruz Biotechnology, Inc. | Rabbit Anti-ephrin-B2 (P-20) Polyclonal Antibody, Unconjugated | EFNB2 (P-20) | sc-1010 | WB, IF | Polyclonal |
| Santa Cruz Biotechnology, Inc. | Goat Anti-ephrin-B2 Polyclonal Antibody, Unconjugated | EFNB2 | sc-19227 | WB, IF, IP | Polyclonal |
| Santa Cruz Biotechnology, Inc. | Rabbit Anti-ephrin-B2 Polyclonal Antibody, Unconjugated | EFNB2 | sc-15397 | WB, IF, IP | Polyclonal |

### EXAMPLES

### Example 1

A variety of sequence analysis approaches were pursued, including the search for homologues of the EPO binding domain from EPOR, a domain analysis based method combined with text-mining, and EPO homology analysis followed by text-mining of resultant hits. Only that part of the human proteome exposed to the extracellular environment was investigated. This allowed a focus on homologies that were significant, though possibly overlooked within the context of a complete proteome analysis. This formed the XtraCell database. The XtraCell database performed a signal peptide and transmembrane prediction for the entire human proteome. All proteins possessing at least one of these features were stored in a first version of the extracellular database. Given that not all extracellular proteins actually possess either of these features, there was extracted a list of protein domains specific to the extracellular environment from a SMART (Simple Modular Architecture Research Tool -.SMART is a well-known protein domain database with a strong bias towards domains contained in signalling proteins.) These were then screened against the human proteome using the HMMER algorithm. HMMER is a freely distributable implementation of profile HMM software for protein sequence analysis - Profile hidden Markov models (profile HMMs) can be used to do sensitive database searching using statistical descriptions of a sequence family's consensus. All hits were added to the XtraCell database and the dataset made non-redundant. A final version of the XtraCell database was established for the purpose of these EPO specific analyses (Figure 3).

### Example 2

This example illustrates a domain-based approach coupled with a text-mining and genome-wide analysis. The operating theory was that any novel EPO receptor involved in mediating EPO's neuroprotective effect might also possess the two membrane proximal fibronectin 3 (FN3) domains (as found in EPOR), whilst at the same time being hypoxia inducible. Such conserved domain architecture is compatible with both a heterodimeric complex containing EPOR and/or an independent hypoxia inducible homodimeric receptor. All proteins containing two membrane proximal FN3 domains from the human proteome (84 in all) were extracted and asked whether there was any evidence for their role in response to low oxygen conditions/ischaemia. The latter analysis was performed using a text-mining approach that encompasses the use of comprehensive protein synonyms, and concepts such as hypoxia and ischaemia. Of the 84 proteins containing the 2FN3-TM domain composition, only four showed evidence for mediating response to low oxygen conditions: EPH-B4, IL6RB, TIE1 and GM-CSF. Apart from EPH-B4, the cellular role of each of these proteins has been studied and an important role in response to hypoxia established.

Direct examination of the EPH-B4 locus revealed that it directly juxtaposes the EPO locus, albeit on the opposite strand. This close genomic association was conserved in all vertebrate genomes examined. The need for immediate response of cells to low oxygen conditions and thus the need to co-transcribe/-translate key effector molecules was seen. Moreover, such genomic co-localisation of functionally associated molecules is seen for other receptor:ligand partners (*e.g.* MST1 and its receptor MST1R: see http://www.ensembl,org/Homo_sapiens/contigview?gene=OTTHUMG00000136237:db=vega ); (see also Figure 4).

To examine this possibility in greater detail, we analysed the promoter, 5' UTR and 3' UTR regions of EPO, EPHB4 and EPOR in search of hypoxia inducible factor binding sites. Here we utilised the 'match' algorithm from Genomatix, searching for strict conservation of the core binding site residues and at least 90% conservation of non-core residues. We found that the EPO and EPH-B4 loci possessed numerous hypoxia-inducible transcription factor binding sites. In contrast, the EPOR gene regulatory regions were found to be complete devoid of such HIF-1 binding sites, again hinting at a possible role for EPHB4 as a hypoxia inducible EPO receptor (see Figure 4).

### Example 3

This example shows the homology-based approach using human extra-cellular database. Here we sought to directly identify regions of EPO binding activity in other proteins, by direct comparison to the EPO binding domain of EPOR. The region of EPOR responsible for EPO binding was thus extracted and used to identify homologies with proteins of the XtraCellDB. This specially developed database holds distinct advantages in that all homologies identified are to human extracellular proteins, thus avoiding the need to assess spurious homologies to irrelevant intracellular species. Analysis of resultant homologues revealed a striking homology to the Ephrin A1 protein, within the top four hits. Given what we had learned about EPH-B4's possible role in EPO signalling we decided to assess this homology in greater detail using the Swiss-model protein structure package. Here we employed information derived from the co-crystal structure of Ephrin A5 in association with EphB2 and compared it to EPO:EPOR co-crystal information. Conservation of key residues in structurally aligned positions allowed us to conclude a firm structural basis for association between Ephrin A1 and EPO. Moreover, the realisation that both EphrinA1 and EPHB4 possess a putative affinity for EPO, suggests a more exciting functional context for eprhin biology than heretofore recognised (See Figure 6).

### Example 4

This example provides wet lab or *in vivo* data that validates the bioinformatics analysis provide in Examples 1-3 herein. *In vivo* validation of EPH-B4's role in EPO signalling has focussed on the neuroprotective aspect of EPO's function, with a bias towards the hypothesis that EPH-B4 and EPOR are heterodimeric partners. The following table lists the validation experiments for which data are available (see Table 7).

**LAB-BASED validation experiments**

| Method | Goal | Result |
|---|---|---|
| Immunohistochemistry | To assess the expression of EPHB4 protein in brain and how it relates to EPOR expression. | Precipitation stainings on adult rodent brain showed that EPHB4 was expressed in adult neurons in the same pattern as EPO receptor. Staining in hippocampus showed co-expression of EPOR and EPHB4 (see figure 6). Strikingly, the staining was restricted to particular cells within the field of tissue. |
| Co-IP | Exogenous expression of EPOR/EPHB4 in COS cells. Co-ip with EpoR-and EphB4-antibodies => WB analysis. | Positive. Use of EPOR antibody successfully Co-IP's EPHB4 protein. |

Immunohistochemistry. For immunofluorescence, sections of paraffin-embedded rat brain tissues (2 µm) were deparaffinated and microwaved (citrate buffer at 600W for 15 min). Afterwards, sections were incubated simultaneously with the EpoR antiserum (1:200; sc-697, Santa Cruz Biotechnology) and the EphB4 antibody (1:100; AF446, R&D Systems) at 4°C over night. After adding a biotinylated anti-goat secondary antibody (1:200; Dianova), sections were incubated with Streptavidin-coupled Alexa Fluor 555 (1:200; Invitrogen, Karlsruhe, Germany) and a FITC-coupled anti-rabbit secondary antibody (1:200; Dianova). The nuclei were counterstained with Hoechst 33342 (1:10,000; Molecular Probes). Controls for the stainings included omission of primary antibodies, fluorophor swapping, and single-fluorescence stainings. Images were obtained with an Olympus IX-81 microscope with narrow-bandwidth monochromator excitation (Polychrome IV, Till Photonics, Gräfelfing, Germany) and appropriate filters.

Both EPHB4 and EPOR displayed a striking co-localisation when assessed in rat brain tissue sections. Without being bound by theory, this co-expression suggests functional coupling of both receptors.

Co-immunoprecipitation. The principle of an immunoprecipitation is an antibody (monoclonal or polyclonal) against a specific target antigen is allowed to form an immune complex with that target in a sample, such as a cell lysate. The immune complex is then captured on a solid support to which either Protein A or Protein G has been immobilized (Protein A or G binds to the antibody, which is bound to its antigen). The process of capturing this complex from the solution is referred to as precipitation. Any proteins not "precipitated" by the immobilized Protein A or G support are washed away. Finally, components of the bound immune complex (both antigen and antibody) are eluted from the support and analyzed by SDS-PAGE (gel electrophoresis), often followed by Western blot detection to verify the identity of the antigen.

Traditional immunoprecipitation involves the following steps:
1. Form the antigen-antibody complex (immune complex) by incubating specific antibody with the antigen-containing sample for 1 hour to several hours.
2. Capture the immune complex on an immobilized Protein A or Protein G agarose gel support by incubation for 0.5-2 hours.
3. Remove any non-bound protein (non-immune complex sample components) from the precipitated complex by washing gel support with additional sample buffer.
4. Boil gel support in reducing SDS-PAGE sample loading buffer.
5. Recover sample eluted in loading buffer from gel support and analyze by SDS-PAGE.
6. Perform Western blot analysis, probing with antigen-specific antibody.

In a co-immunoprecipitation the target antigen precipitated by the antibody "co-precipitates" a binding partner/protein complex from a lysate, that is, the interacting protein is bound to the target antigen, which becomes bound by the antibody that becomes captured on the Protein A or G gel support. The assumption that is usually made when associated proteins are coprecipitated is that these proteins are related to the function of the target antigen at the cellular level.

Assessment of a putative EPHB4:EPOR association using co-immmunoprecipitation showed that both proteins were physically associated. Here, FLAG-tagged EPOR was co-expressed with EPH-B4 in COS cells and then immunoprecipitated using an a-FLAG antibody. As can be seen from Figure 8, EPHB4 was shown to co-immunoprecipitate in these experiments.

Human Fc Antibody Constructs. The Fc conjugate approach is most appropriate when dealing with dimeric cell surface receptors. Here the extracellular portion of EPHB4/EPOR was fused to an Fc fragment. This method has advantages due to its *in vivo* (therapeutic) viability and the fact that it optimally mimics the dimerised receptor state. Figure 9 highlights the Human constructs that are used to show EPHB4's/EphrinA1's affinity for EPO.

One of two alternatives can assay the interaction of the Fc constructs with EPO, including, for example, a protein array approach or a surface plasmon resonance analysis.

### Example 5

### Further in vitro and in vivo validation of NEPORs role in mediating EPO function.

In these experiments we sought to determine the response to erythropoietin (EPO) treatment in a panel of ovarian cancer cell lines. This would be mediated by the expression of erythropoietin receptor (EPO as well as two receptors that potentially may be able to activate signaling pathways in response to EPO binding, EPH-B4 and Ephrin A1. It was first necessary to characterize the expression of these receptors in a panel of ovarian cancer cell lines. First we collected RNA from each cell line and reverse transcribed them into cDNA. Using specific primers for each receptor we analyzed their RNA expression. As evident in the figures the expression of EPOR and EPH-B4 RNA is different in different cell lines suggesting changes in transcriptional regulation during tumorigenesis no significant changes were seen in the EphrinA1. It was then necessary to determine protein expression of these receptors in the panel. Again we see significant differences in the expression of the EPOR and EPH-B4 receptors though they do not coincide with the RNA expression suggesting there is changes in post transcriptional regulation of these receptors in the cell lines. We then categorized these expression changes particularly with regard to the EPOR and EPH-B4 to then analyze the response to EPO treatment. We analyzed their response to chemotherapy in conjunction with EPO. We found that particularly in the HeyA8 ovarian cancer cell line that EPO was able to abrogate the apoptosis induced by docetaxel. It was then necessary to analyze the activation of signaling pathways known to be activated by these receptors in response to EPO treatment. Three cell lines were starved for two hours to isolate their response to EPO. Cell lines with higher expression (HeyA8 and HeyA8 MDR) of the EPOR demonstrated activation of the MAPK/ERK pathway while cell lines that expressed higher EPH-B4 (SKOV3ip1) demonstrated increased activation of the AKT and STAT5b signaling pathways. We then sought to determine a EPO dose that optimized its tumor promoting effect *in vivo.* Female nude mice were injected i.p. with HeyA8 MDR (positive for both EPOR and EPH-B4). At day eight the mice were treated with increasing doses of EPO (10, 50, 100 U) every two days. Treatment continued until tumors became evident, the mice were then sacrificed and the tumor weight was determined. We saw an increase in tumor weight as compared to control in the mice treated with 10 and 50 U EPO. The differential expression of EphB4 in cell lines as well as the activation of particular signaling pathways suggested that it would also mediate the tumor promoting effect *in vivo.* To determine this we again injected mice with HeyA8 MDR cell lines i.p. At day eight we began treatment with EPO (50 U 3x week) in conjunction with siRNA specific to EPH-B4. As previously described EphB4 siRNA was able decrease tumor growth alone. Moreover, EPH-B4 siRNA also completely abrogated the EPO induced tumor growth.

### SEQUENCE LISTING

<110> Molecular Health GmbH
<120> Novel Tissue Protective Erythropoietin Receptor (NEPOR) and Methods of use
<130> F66500PCEPT2
<140> Divisional of EP 11004689.3
   <141> 2008-11-28
<150> US 60/991,042
   <151> 2007-11-29
<160> 224
<170> PatentIn version 3.4
<210> 1
   <211> 508
   <212> PRT
   <213> Homo sapiens
<400> 1
   Met Asp His Leu Gly Ala Ser Leu Trp Pro Gln Val Gly Ser Leu Cys
   1               5                   10                  15
   Leu Leu Leu Ala Gly Ala Ala Trp Ala Pro Pro Pro Asn Leu Pro Asp
               20                  25                  30
   Pro Lys Phe Glu Ser Lys Ala Ala Leu Leu Ala Ala Arg Gly Pro Glu
           35                  40                  45
   Glu Leu Leu Cys Phe Thr Glu Arg Leu Glu Asp Leu Val Cys Phe Trp
       50                  55                  60
   Glu Glu Ala Ala Ser Ala Gly Val Gly Pro Gly Asn Tyr Ser Phe Ser
   65                  70                  75                  80
   Tyr Gln Leu Glu Asp Glu Pro Trp Lys Leu Cys Arg Leu His Gln Ala
                   85                  90                  95
   Pro Thr Ala Arg Gly Ala Val Arg Phe Trp Cys Ser Leu Pro Thr Ala
               100                 105                 110
   Asp Thr Ser Ser Phe Val Pro Leu Glu Leu Arg Val Thr Ala Ala Ser
           115                 120                 125
   Gly Ala Pro Arg Tyr His Arg Val Ile His Ile Asn Glu Val Val Leu
       130                 135                 140
   Leu Asp Ala Pro Val Gly Leu Val Ala Arg Leu Ala Asp Glu Ser Gly
   145                 150                 155                 160
   His Val Val Leu Arg Trp Leu Pro Pro Pro Glu Thr Pro Met Thr Ser
                   165                 170                 175
   His Ile Arg Tyr Glu Val Asp Val Ser Ala Gly Asn Gly Ala Gly Ser
   180 185 190
   Val Gln Arg Val Glu Ile Leu Glu Gly Arg Thr Glu Cys Val Leu Ser
   195 200 205
   Asn Leu Arg Gly Arg Thr Arg Tyr Thr Phe Ala Val Arg Ala Arg Met
   210 215 220
   Ala Glu Pro Ser Phe Gly Gly Phe Trp Ser Ala Trp Ser Glu Pro Val
   225 230 235 240
   Ser Leu Leu Thr Pro Ser Asp Leu Asp Pro Leu Ile Leu Thr Leu Ser
   245 250 255
   Leu Ile Leu Val Val Ile Leu Val Leu Leu Thr Val Leu Ala Leu Leu
   260 265 270
   Ser His Arg Arg Ala Leu Lys Gln Lys Ile Trp Pro Gly Ile Pro Ser
   275 280 285
   Pro Glu Ser Glu Phe Glu Gly Leu Phe Thr Thr His Lys Gly Asn Phe
   290 295 300
   Gln Leu Trp Leu Tyr Gln Asn Asp Gly Cys Leu Trp Trp Ser Pro Cys
   305 310 315 320
   Thr Pro Phe Thr Glu Asp Pro Pro Ala Ser Leu Glu Val Leu Ser Glu
   325 330 335
   Arg Cys Trp Gly Thr Met Gln Ala Val Glu Pro Gly Thr Asp Asp Glu
   340 345 350
   Gly Pro Leu Leu Glu Pro Val Gly Ser Glu His Ala Gln Asp Thr Tyr
   355 360 365
   Leu Val Leu Asp Lys Trp Leu Leu Pro Arg Asn Pro Pro Ser Glu Asp
   370 375 380
   Leu Pro Gly Pro Gly Gly Ser Val Asp Ile Val Ala Met Asp Glu Gly
   385 390 395 400
   Ser Glu Ala Ser Ser Cys Ser Ser Ala Leu Ala Ser Lys Pro Ser Pro
   405 410 415
   Glu Gly Ala Ser Ala Ala Ser Phe Glu Tyr Thr Ile Leu Asp Pro Ser
   420 425 430
   Ser Gln Leu Leu Arg Pro Trp Thr Leu Cys Pro Glu Leu Pro Pro Thr
   435 440 445
   Pro Pro His Leu Lys Tyr Leu Tyr Leu Val Val Ser Asp Ser Gly Ile
       450                 455                 460
   Ser Thr Asp Tyr Ser Ser Gly Asp Ser Gln Gly Ala Gln Gly Gly Leu
   465                 470                 475                 480
   Ser Asp Gly Pro Tyr Ser Asn Pro Tyr Glu Asn Ser Leu Ile Pro Ala
                   485                 490                 495
   Ala Glu Pro Leu Pro Pro Ser Tyr Val Ala Cys Ser
               500                 505
<210> 2
   <211> 987
   <212> PRT
   <213> Homo sapiens
<400> 2
<210> 3
   <211> 205
   <212> PRT
   <213> Homo sapiens
<400> 3
   Met Glu Phe Leu Trp Ala Pro Leu Leu Gly Leu Cys Cys Ser Leu Ala
   1               5                   10                  15
   Ala Ala Asp Arg His Thr Val Phe Trp Asn Ser Ser Asn Pro Lys Phe
               20                  25                  30
   Arg Asn Glu Asp Tyr Thr Ile His Val Gln Leu Asn Asp Tyr Val Asp
           35                  40                  45
   Ile Ile Cys Pro His Tyr Glu Asp His Ser Val Ala Asp Ala Ala Met
       50                  55                  60
   Glu Gln Tyr Ile Leu Tyr Leu Val Glu His Glu Glu Tyr Gln Leu Cys
   65                  70                  75                  80
   Gln Pro Gln Ser Lys Asp Gln Val Arg Trp Gln Cys Asn Arg Pro Ser
                   85                  90                  95
   Ala Lys His Gly Pro Glu Lys Leu Ser Glu Lys Phe Gln Arg Phe Thr
               100                 105                 110
   Pro Phe Thr Leu Gly Lys Glu Phe Lys Glu Gly His Ser Tyr Tyr Tyr
           115                 120                 125
   Ile Ser Lys Pro Ile His Gln His Glu Asp Arg Cys Leu Arg Leu Lys
       130                 135                 140
   Val Thr Val Ser Gly Lys Ile Thr His Ser Pro Gln Ala His Asp Asn
   145                 150                 155                 160
   Pro Gln Glu Lys Arg Leu Ala Ala Asp Asp Pro Glu Val Arg Val Leu
                   165                 170                 175
   His Ser Ile Gly His Ser Ala Ala Pro Arg Leu Phe Pro Leu Ala Trp
               180                 185                 190
   Thr Val Leu Leu Leu Pro Leu Leu Leu Leu Gln Thr Pro
           195                 200                 205
<210> 4
   <211> 333
   <212> PRT
   <213> Homo sapiens
<400> 4
   Met Ala Val Arg Arg Asp Ser Val Trp Lys Tyr Cys Trp Gly Val Leu
   1 5 10 15
   Met Val Leu Cys Arg Thr Ala Ile Ser Lys Ser Ile Val Leu Glu Pro
   20 25 30
   Ile Tyr Trp Asn Ser Ser Asn Ser Lys Phe Leu Pro Gly Gln Gly Leu
   35 40 45
   Val Leu Tyr Pro Gln Ile Gly Asp Lys Leu Asp Ile Ile Cys Pro Lys
   50 55 60
   Val Asp Ser Lys Thr Val Gly Gln Tyr Glu Tyr Tyr Lys Val Tyr Met
   65 70 75 80
   Val Asp Lys Asp Gln Ala Asp Arg Cys Thr Ile Lys Lys Glu Asn Thr
   85 90 95
   Pro Leu Leu Asn Cys Ala Lys Pro Asp Gln Asp Ile Lys Phe Thr Ile
   100 105 110
   Lys Phe Gln Glu Phe Ser Pro Asn Leu Trp Gly Leu Glu Phe Gln Lys
   115 120 125
   Asn Lys Asp Tyr Tyr Ile Ile Ser Thr Ser Asn Gly Ser Leu Glu Gly
   130 135 140
   Leu Asp Asn Gln Glu Gly Gly Val Cys Gln Thr Arg Ala Met Lys Ile
   145 150 155 160
   Leu Met Lys Val Gly Gln Asp Ala Ser Ser Ala Gly Ser Thr Arg Asn
   165 170 175
   Lys Asp Pro Thr Arg Arg Pro Glu Leu Glu Ala Gly Thr Asn Gly Arg
   180 185 190
   Ser Ser Thr Thr Ser Pro Phe Val Lys Pro Asn Pro Gly Ser Ser Thr
   195 200 205
   Asp Gly Asn Ser Ala Gly His Ser Gly Asn Asn Ile Leu Gly Ser Glu
   210 215 220
   Val Ala Leu Phe Ala Gly Ile Ala Ser Gly Cys Ile Ile Phe Ile Val
   225 230 235 240
   Ile Ile Ile Thr Leu Val Val Leu Leu Leu Lys Tyr Arg Arg Arg His
   245 250 255
   Arg Lys His Ser Pro Gln His Thr Thr Thr Leu Ser Leu Ser Thr Leu
   260 265 270
   Ala Thr Pro Lys Arg Ser Gly Asn Asn Asn Gly Ser Glu Pro Ser Asp
   275 280 285
   Ile Ile Ile Pro Leu Arg Thr Ala Asp Ser Val Phe Cys Pro His Tyr
   290 295 300
   Glu Lys Val Ser Gly Asp Tyr Gly His Pro Val Tyr Ile Val Gln Glu
   305 310 315 320
   Met Pro Pro Gln Ser Pro Ala Asn Ile Tyr Tyr Lys Val
   325 330
<210> 5
   <211> 1849
   <212> DNA
   <213> Homo sapiens
<400> 5
   acttagaggc gcctggtcgg gaagggcctg gtcagctgcg tccggcggag gcagctgctg 60
   acccagctgt ggactgtgcc gggggcgggg gacggagggg caggagccct gggctccccg 120
   tggcgggggc tgtatcatgg accacctcgg ggcgtccctc tggccccagg tcggctccct 180
   ttgtctcctg ctcgctgggg ccgcctgggc gcccccgcct aacctcccgg accccaagtt 240
   cgagagcaaa gcggccttgc tggcggcccg ggggcccgaa gagcttctgt gcttcaccga 300
   gcggttggag gacttggtgt gtttctggga ggaagcggcg agcgctgggg tgggcccggg 360
   caactacagc ttctcctacc agctcgagga tgagccatgg aagctgtgtc gcctgcacca 420
   ggctcccacg gctcgtggtg cggtgcgctt ctggtgttcg ctgcctacag ccgacacgtc 480
   gagcttcgtg cccctagagt tgcgcgtcac agcagcctcc ggcgctccgc gatatcaccg 540
   tgtcatccac atcaatgaag tagtgctcct agacgccccc gtggggctgg tggcgcggtt 600
   ggctgacgag agcggccacg tagtgttgcg ctggctcccg ccgcctgaga cacccatgac 660
   gtctcacatc cgctacgagg tggacgtctc ggccggcaac ggcgcaggga gcgtacagag 720
   ggtggagatc ctggagggcc gcaccgagtg tgtgctgagc aacctgcggg gccggacgcg 780
   ctacaccttc gccgtccgcg cgcgtatggc tgagccgagc ttcggcggct tctggagcgc 840
   ctggtcggag cctgtgtcgc tgctgacgcc tagcgacctg gaccccctca tcctgacgct 900
   ctccctcatc ctcgtggtca tcctggtgct gctgaccgtg ctcgcgctgc tctcccaccg 960
   ccgggctctg aagcagaaga tctggcctgg catcccgagc ccagagagcg agtttgaagg 1020
   cctcttcacc acccacaagg gtaacttcca gctgtggctg taccagaatg atggctgcct 1080
   gtggtggagc ccctgcaccc ccttcacgga ggacccacct gcttccctgg aagtcctctc 1140
   agagcgctgc tgggggacga tgcaggcagt ggagccgggg acagatgatg agggccccct 1200
   gctggagcca gtgggcagtg agcatgccca ggatacctat ctggtgctgg acaaatggtt 1260
   gctgccccgg aacccgccca gtgaggacct cccagggcct ggtggcagtg tggacatagt 1320
   ggccatggat gaaggctcag aagcatcctc ctgctcatct gctttggcct cgaagcccag 1380
   cccagaggga gcctctgctg ccagctttga gtacactatc ctggacccca gctcccagct 1440
   cttgcgtcca tggacactgt gccctgagct gccccctacc ccaccccacc taaagtacct 1500
   gtaccttgtg gtatctgact ctggcatctc aactgactac agctcagggg actcccaggg 1560
   agcccaaggg ggcttatccg atggccccta ctccaaccct tatgagaaca gccttatccc 1620
   agccgctgag cctctgcccc ccagctatgt ggcttgctct taggacacca ggctgcagat 1680
   gatcagggat ccaatatgac tcagagaacc agtgcagact caagacttat ggaacaggga 1740
   tggcgaggcc tctctcagga gcaggggcat tgctgatttt gtctgcccaa tccatcctgc 1800
   tcaggaaacc acaaccttgc agtattttta aatatgtata gtttttttg 1849
<210> 6
   <211> 4369
   <212> DNA
   <213> Homo sapiens
<400> 6
   ttccagcgca gctcagcccc tgcccggccc ggcccgcccg gctccgcgcc gcagtctccc      60
   tccctcccgc tccgtccccg ctcgggctcc caccatcccc gcccgcgagg agagcactcg     120
   gcccggcggc gcgagcagag ccactccagg gaggggggga gaccgcgagc ggccggctca      180
   gcccccgcca cccggggcgg gaccccgagg ccccggaggg accccaactc cagccacgtc     240
   ttgctgcgcg cccgcccggc gcggccactg ccagcacgct ccgggcccgc cgcccgcgcg     300
   cgcggcacag acgcggggcc acacttggcg ccgccgcccg gtgccccgca cgctcgcatg     360
   ggcccgcgct gagggccccg acgaggagtc ccgcgcggag tatcggcgtc cacccgccca     420
   gggagagtca gacctggggg ggcgagggcc ccccaaactc agttcggatc ctacccgagt     480
   gaggcggcgc catggagctc cgggtgctgc tctgctgggc ttcgttggcc gcagctttgg     540
   aagagaccct gctgaacaca aaattggaaa ctgctgatct gaagtgggtg acattccctc     600
   aggtggacgg gcagtgggag gaactgagcg gcctggatga ggaacagcac agcgtgcgca     660
   cctacgaagt gtgtgacgtg cagcgtgccc cgggccaggc ccactggctt cgcacaggtt     720
   gggtcccacg gcggggcgcc gtccacgtgt acgccacgct gcgcttcacc atgctcgagt     780
   gcctgtccct gcctcgggct gggcgctcct gcaaggagac cttcaccgtc ttctactatg     840
   agagcgatgc ggacacggcc acggccctca cgccagcctg gatggagaac ccctacatca     900
   aggtggacac ggtggccgcg gagcatctca cccggaagcg ccctggggcc gaggccaccg     960
   ggaaggtgaa tgtcaagacg ctgcgtctgg gaccgctcag caaggctggc ttctacctgg    1020
   ccttccagga ccagggtgcc tgcatggccc tgctatccct gcacctcttc tacaaaaagt    1080
   gcgcccagct gactgtgaac ctgactcgat tcccggagac tgtgcctcgg gagctggttg    1140
   tgcccgtggc cggtagctgc gtggtggatg ccgtccccgc ccctggcccc agccccagcc    1200
   tctactgccg tgaggatggc cagtgggccg aacagccggt cacgggctgc agctgtgctc    1260
   cggggttcga ggcagctgag gggaacacca agtgccgagc ctgtgcccag ggcaccttca    1320
   agcccctgtc aggagaaggg tcctgccagc catgcccagc caatagccac tctaacacca    1380
   ttggatcagc cgtctgccag tgccgcgtcg ggtacttccg ggcacgcaca gacccccggg 1440
   gtgcaccctg caccacccct ccttcggctc cgcggagcgt ggtttcccgc ctgaacggct 1500
   cctccctgca cctggaatgg agtgcccccc tggagtctgg tggccgagag gacctcacct 1560
   acgccctccg ctgccgggag tgccgacccg gaggctcctg tgcgccctgc gggggagacc 1620
   tgacttttga ccccggcccc cgggacctgg tggagccctg ggtggtggtt cgagggctac 1680
   gtcctgactt cacctatacc tttgaggtca ctgcattgaa cggggtatcc tccttagcca 1740
   cggggcccgt cccatttgag cctgtcaatg tcaccactga ccgagaggta cctcctgcag 1800
   tgtctgacat ccgggtgacg cggtcctcac ccagcagctt gagcctggcc tgggctgttc 1860
   cccgggcacc cagtggggct gtgctggact acgaggtcaa ataccatgag aagggcgccg 1920
   agggtcccag cagcgtgcgg ttcctgaaga cgtcagaaaa ccgggcagag ctgcgggggc 1980
   tgaagcgggg agccagctac ctggtgcagg tacgggcgcg ctctgaggcc ggctacgggc 2040
   ccttcggcca ggaacatcac agccagaccc aactggatga gagcgagggc tggcgggagc 2100
   agctggccct gattgcgggc acggcagtcg tgggtgtggt cctggtcctg gtggtcattg 2160
   tggtcgcagt tctctgcctc aggaagcaga gcaatgggag agaagcagaa tattcggaca 2220
   aacacggaca gtatctcatc ggacatggta ctaaggtcta catcgacccc ttcacttatg 2280
   aagaccctaa tgaggctgtg agggaatttg caaaagagat cgatgtctcc tacgtcaaga 2340
   ttgaagaggt gattggtgca ggtgagtttg gcgaggtgtg ccgggggcgg ctcaaggccc 2400
   cagggaagaa ggagagctgt gtggcaatca agaccctgaa gggtggctac acggagcggc 2460
   agcggcgtga gtttctgagc gaggcctcca tcatgggcca gttcgagcac cccaatatca 2520
   tccgcctgga gggcgtggtc accaacagca tgcccgtcat gattctcaca gagttcatgg 2580
   agaacggcgc cctggactcc ttcctgcggc taaacgacgg acagttcaca gtcatccagc 2640
   tcgtgggcat gctgcggggc atcgcctcgg gcatgcggta ccttgccgag atgagctacg 2700
   tccaccgaga cctggctgct cgcaacatcc tagtcaacag caacctcgtc tgcaaagtgt 2760
   ctgactttgg cctttcccga ttcctggagg agaactcttc cgatcccacc tacacgagct 2820
   ccctgggagg aaagattccc atccgatgga ctgccccgga ggccattgcc ttccggaagt 2880
   tcacttccgc cagtgatgcc tggagttacg ggattgtgat gtgggaggtg atgtcatttg 2940
   gggagaggcc gtactgggac atgagcaatc aggacgtgat caatgccatt gaacaggact 3000
   accggctgcc cccgccccca gactgtccca cctccctcca ccagctcatg ctggactgtt 3060
   ggcagaaaga ccggaatgcc cggccccgct tcccccaggt ggtcagcgcc ctggacaaga 3120
   tgatccggaa ccccgccagc ctcaaaatcg tggcccggga gaatggcggg gcctcacacc 3180
   ctctcctgga ccagcggcag cctcactact cagcttttgg ctctgtgggc gagtggcttc 3240
   gggccatcaa aatgggaaga tacgaagaaa gtttcgcagc cgctggcttt ggctccttcg 3300
   agctggtcag ccagatctct gctgaggacc tgctccgaat cggagtcact ctggcgggac 3360
   accagaagaa aatcttggcc agtgtccagc acatgaagtc ccaggccaag ccgggaaccc 3420
   cgggtgggac aggaggaccg gccccgcagt actgacctgc aggaactccc caccccaggg 3480
   acaccgcctc cccattttcc ggggcagagt ggggactcac agaggccccc agccctgtgc 3540
   cccgctggat tgcactttga gcccgtgggg tgaggagttg gcaatttgga gagacaggat 3600
   ttgggggttc tgccataata ggaggggaaa atcacccccc agccacctcg gggaactcca 3660
   gaccaagggt gagggcgcct ttccctcagg actgggtgtg accagaggaa aaggaagtgc 3720
   ccaacatctc ccagcctccc caggtgcccc cctcaccttg atgggtgcgt tcccgcagac 3780
   caaagagagt gtgactccct tgccagctcc agagtggggg ggctgtccca gggggcaaga 3840
   aggggtgtca gggcccagtg acaaaatcat tggggtttgt agtcccaact tgctgctgtc 3900
   accaccaaac tcaatcattt ttttcccttg taaatgcccc tcccccagct gctgccttca 3960
   tattgaaggt ttttgagttt tgtttttggt cttaattttt ctccccgttc cctttttgtt 4020
   tcttcgtttt gtttttctac cgtccttgtc ataactttgt gttggaggga acctgtttca 4080
   ctatggcctc ctttgcccaa gttgaaacag gggcccatca tcatgtctgt ttccagaaca 4140
   gtgccttggt catcccacat ccccggaccc cgcctgggac ccccaagctg tgtcctatga 4200
   aggggtgtgg ggtgaggtag tgaaaagggc ggtagttggt ggtggaaccc agaaacggac 4260
   gccggtgctt ggaggggttc ttaaattata tttaaaaaag taactttttg tataaataaa 4320
   agaaaatggg acgtgtccca gctccagggg taaaaaaaaa aaaaaaaaa 4369
<210> 7
   <211> 1590
   <212> DNA
   <213> Homo sapiens
<400> 7
   gccagatctg tgagcccagc gctgactgcg ccgcggagaa agccagtggg aacccagacc      60
   cataggagac ccgcgtcccc gctcggcctg gccaggcccc gcgctatgga gttcctctgg     120
   gcccctctct tgggtctgtg ctgcagtctg gccgctgctg atcgccacac cgtcttctgg     180
   aacagttcaa atcccaagtt ccggaatgag gactacacca tacatgtgca gctgaatgac     240
   tacgtggaca tcatctgtcc gcactatgaa gatcactctg tggcagacgc tgccatggag     300
   cagtacatac tgtacctggt ggagcatgag gagtaccagc tgtgccagcc ccagtccaag     360
   gaccaagtcc gctggcagtg caaccggccc agtgccaagc atggcccgga gaagctgtct     420
   gagaagttcc agcgcttcac acctttcacc ctgggcaagg agttcaaaga aggacacagc     480
   tactactaca tctccaaacc catccaccag catgaagacc gctgcttgag gttgaaggtg     540
   actgtcagtg gcaaaatcac tcacagtcct caggcccatg acaatccaca ggagaagaga     600
   cttgcagcag atgacccaga ggtgcgggtt ctacatagca tcggtcacag tgctgcccca     660
   cgcctcttcc cacttgcctg gactgtgctg ctccttccac ttctgctgct gcaaaccccg     720
   tgaaggtgta tgccacacct ggccttaaag agggacaggc tgaagagagg gacaggcact     780
   ccaaacctgt cttggggcca ctttcagagc ccccagccct gggaaccact cccaccacag     840
   gcataagcta tcacctagca gcctcaaaac gggtcagtat taaggttttc aaccggaagg     900
   aggccaacca gcccgacagt gccatcccca ccttcacctc ggagggatgg agaaagaagt     960
   ggagacagtc ctttcccacc attcctgcct ttaagccaaa gaaacaagct gtgcaggcat    1020
   ggtcccttaa ggcacagtgg gagctgagct ggaaggggcc acgtggatgg gcaaagcttg    1080
   tcaaagatgc cccctccagg agagagccag gatgcccaga tgaactgact gaaggaaaag    1140
   caagaaacag tttcttgctt ggaagccagg tacaggagag gcagcatgct tgggctgacc    1200
   cagcatctcc cagcaagacc tcatctgtgg agctgccaca gagaagtttg tagccaggta    1260
   ctgcattctc tcccatcctg gggcagcact ccccagagct gtgccagcag gggggctgtg    1320
   ccaacctgtt cttagagtgt agctgtaagg gcagtgccca tgtgtacatt ctgcctagag    1380
   tgtagcctaa agggcagggc ccacgtgtat agtatctgta tataagttgc tgtgtgtctg    1440
   tcctgatttc tacaactgga gtttttttat acaatgttct ttgtctcaaa ataaagcaat    1500
   gtgttttttc ggacatgctt ttctgccact ccatattaaa acatatgacc attgagtccc    1560
   tgctaaaaaa aaaaaaaaaa aaaaaaaaaa                                     1590
<210> 8
   <211> 4335
   <212> DNA
   <213> Homo sapiens
<400> 8
   gcgcggagct gggagtggct tcgccatggc tgtgagaagg gactccgtgt ggaagtactg     60
   ctggggtgtt ttgatggttt tatgcagaac tgcgatttcc aaatcgatag ttttagagcc    120
   tatctattgg aattcctcga actccaaatt tctacctgga caaggactgg tactataccc    180
   acagatagga gacaaattgg atattatttg ccccaaagtg gactctaaaa ctgttggcca    240
   gtatgaatat tataaagttt atatggttga taaagaccaa gcagacagat gcactattaa    300
   gaaggaaaat acccctctcc tcaactgtgc caaaccagac caagatatca aattcaccat    360
   caagtttcaa gaattcagcc ctaacctctg gggtctagaa tttcagaaga acaaagatta    420
   ttacattata tctacatcaa atgggtcttt ggagggcctg gataaccagg agggaggggt    480
   gtgccagaca agagccatga agatcctcat gaaagttgga caagatgcaa gttctgctgg    540
   atcaaccagg aataaagatc caacaagacg tccagaacta gaagctggta caaatggaag    600
   aagttcgaca acaagtccct ttgtaaaacc aaatccaggt tctagcacag acggcaacag    660
   cgccggacat tcggggaaca acatcctcgg ttccgaagtg gccttatttg cagggattgc    720
   ttcaggatgc atcatcttca tcgtcatcat catcacgctg gtggtcctct tgctgaagta    780
   ccggaggaga cacaggaagc actcgccgca gcacacgacc acgctgtcgc tcagcacact    840
   ggccacaccc aagcgcagcg gcaacaacaa cggctcagag cccagtgaca ttatcatccc    900
   gctaaggact gcggacagcg tcttctgccc tcactacgag aaggtcagcg gggactacgg    960
   gcacccggtg tacatcgtcc aggagatgcc cccgcagagc ccggcgaaca tttactacaa   1020
   ggtctgagag ggaccctggt ggtacctgtg ctttcccaga ggacacctaa tgtcccgatg   1080
   cctcccttga gggtttgaga gcccgcgtgc tggagaattg actgaagcac agcaccgggg 1140
   gagagggaca ctcctcctcg gaagagcccg tcgcgctgga cagcttacct agtcttgtag 1200
   cattcggcct tggtgaacac acacgctccc tggaagctgg aagactgtgc agaagacgcc 1260
   cattcggact gctgtgccgc gtcccacgtc tcctcctcga agccatgtgc tgcggtcact 1320
   caggcctctg cagaagccaa gggaagacag tggtttgtgg acgagagggc tgtgagcatc 1380
   ctggcaggtg ccccaggatg ccacgcctgg aagggccggc ttctgcctgg ggtgcatttc 1440
   ccccgcagtg cataccggac ttgtcacacg gacctcgggc tagttaaggt gtgcaaagat 1500
   ctctagagtt tagtccttac tgtctcactc gttctgttac ccagggctct gcagcacctc 1560
   acctgagacc tccactccac atctgcatca ctcatggaac actcatgtct ggagtcccct 1620
   cctccagccg ctggcaacaa cagcttcagt ccatgggtaa tccgttcata gaaattgtgt 1680
   ttgctaacaa ggtgcccttt agccagatgc taggctgtct gcgaagaagg ctaggagttc 1740
   atagaaggga gtggggctgg ggaaagggct ggctgcaatt gcagctcact gctgctgcct 1800
   ctgaaacaga aagttggaaa ggaaaaaaga aaaaagcaat taggtagcac agcactttgg 1860
   ttttgctgag atcgaagagg ccagtaggag acacgacagc acacacagtg gattccagtg 1920
   catggggagg cactcgctgt tatcaaatag cgatgtgcag gaagaaaagc ccctcttcat 1980
   tccggggaac aaagacgggt attgttggga aaggaacagg cttggaggga agggagaaag 2040
   taggccgctg atgatatatt cgggcaggac tgttgtggta ctggcaataa gatacacagc 2100
   tccgagctgt aggagagtcg gtctgctttg gatgattttt taagcagact cagctgctat 2160
   acttatcaca ttttattaaa cacagggaaa gcatttagga gaatagcaga gagccaaatc 2220
   tgacctaaaa gttgaaaagc caaaggtcaa acaggctgta attccatcat catcgttgtt 2280
   attaaagaat ccttatctat aaaaggtagg tcagatcccc ctccccccag gttcctcctt 2340
   cccctcccga ttgagcctta cgacactttg gtttatgcgg tgctgtccgg gtgccagggc 2400
   tgcagggtcg gtactgatgg aggctgcagc gcccggtgct ctgtgtcaag gtgaagcaca 2460
   tacggcagac ctcttagagt ccttaagacg gaagtaaatt atgatgtcca gggggagaag 2520
   gaagatagga cgtatttata ataggtatat agaacacaag ggatataaaa tgaaagattt 2580
   ttactaatat atattttaag gttgcacaca gtacacacca gaagatgtga aattcatttg 2640
   tggcaattaa gtggtcccaa tgctcagcgc ttaaaaaaac aaattggaca gctacttctg 2700
   ggaaaaacaa catcattcca aaaagaacaa taatgagagc aaatgcaaaa ataaccaagt 2760
   cctccgaagg catctcacgg aaccgtagac taggaagtac gagccccaca gagcaggaag 2820
   ccgatgtgac tgcatcatat atttaacaat gacaagatgt tccggcgttt atttctgcgt 2880
   tgggttttcc cttgccttat gggctgaagt gttctctaga atccagcagg tcacactggg 2940
   ggcttcaggt gacgatttag ctgtggctcc ctcctcctgt cctcccccgc accccctccc 3000
   ttctgggaaa caagaagagt aaacaggaaa cctacttttt atgtgctatg caaaatagac 3060
   atctttaaca tagtcctgtt actatggtaa cactttgctt tctgaattgg aagggaaaaa 3120
   aaatgtagcg acagcatttt aaggttctca gacctccagt gagtacctgc aaaaatgagt 3180
   tgtcacagaa attatgatcc tctatttcct gaacctggaa atgatgttgg tccaaagtgc 3240
   gtgtgtgtat gtgtgagtgg gtgcgtggta tacatgtgta catatatgta taatatatat 3300
   ctacaatata tattatatat atctatatca tatttctgtg gagggttgcc atggtaacca 3360
   gccacagtac atatgtaatt ctttccatca ccccaacctc tcctttctgt gcattcatgc 3420
   aagagtttct tgtaagccat cagaagttac ttttaggatg ggggagaggg gcgagaaggg 3480
   gaaaaatggg aaatagtctg attttaatga aatcaaatgt atgtatcatc agttggctac 3540
   gttttggttc tatgctaaac tgtgaaaaat cagatgaatt gataaaagag ttccctgcaa 3600
   ccaattgaaa agtgttctgt gcgtctgttt tgtgtctggt gcagaatatg acaatctacc 3660
   aactgtccct ttgtttgaag ttggtttagc tttggaaagt tactgtaaat gccttgcttg 3720
   tatgatcgtc cctggtcacc cgactttgga atttgcacca tcatgtttca gtgaagatgc 3780
   tgtaaatagg ttcagatttt actgtctatg gatttggggt gttacagtag ccttattcac 3840
   ctttttaata aaaatacaca tgaaaacaag aaagaaatgg cttttcttac ccagattgtg 3900
   tacatagagc aatgttggtt ttttataaag tctaagcaag atgttttgta taaaatctga 3960
   attttgcaat gtatttagct acagcttgtt taacggcagt gtcattcccc tttgcactgt 4020
   aatgaggaaa aaatggtata aaaggttgcc aaattgctgc atatttgtgc cgtaattatg 4080
   taccatgaat atttatttaa aatttcgttg tccaatttgt aagtaacaca gtattatgcc 4140
   tgagttataa atattttttt ctttctttgt tttattttaa tagcctgtca taggttttaa 4200
   atctgcttta gtttcacatt gcagttagcc ccagaaaatg aaatccgtga agtcacattc 4260
   cacatctgtt tcaaactgaa tttgttctta aaaaaataaa atattttttt cctatggaaa 4320
   aaaaaaaaaa aaaaa 4335
<210> 9
   <211> 642
   <212> DNA
   <213> Homo sapiens
<400> 9
   agcaaagcgg ccttgctggc ggcccggggg cccgaagagc ttctgtgctt caccgagcgg 60
   ttggaggact tggtgtgttt ctgggaggaa gcggcgagcg ctggggtggg cccgggcaac 120
   tacagcttct cctaccagct cgaggatgag ccatggaagc tgtgtcgcct gcaccaggct 180
   cccacggctc gtggtgcggt gcgcttctgg tgttcgctgc ctacagccga cacgtcgagc 240
   ttcgtgcccc tagagttgcg cgtcacagca gcctccggcg ctccgcgata tcaccgtgtc 300
   atccacatca atgaagtagt gctcctagac gcccccgtgg ggctggtggc gcggttggct 360
   gacgagagcg gccacgtagt gttgcgctgg ctcccgccgc ctgagacacc catgacgtct 420
   cacatccgct acgaggtgga cgtctcggcc ggcaacggcg cagggagcgt acagagggtg 480
   gagatcctgg agggccgcac cgagtgtgtg ctgagcaacc tgcggggccg gacgcgctac 540
   accttcgccg tccgcgcgcg tatggctgag ccgagcttcg gcggcttctg gagcgcctgg 600
   tcggagcctg tgtcgctgct gacgcctagc gacctggacc cc 642
<210> 10
   <211> 651
   <212> DNA
   <213> Homo sapiens
<400> 10
   ccttcggctc cgcggagcgt ggtttcccgc ctgaacggct cctccctgca cctggaatgg 60
   agtgcccccc tggagtctgg tggccgagag gacctcacct acgccctccg ctgccgggag 120
   tgccgacccg gaggctcctg tgcgccctgc gggggagacc tgacttttga ccccggcccc 180
   cgggacctgg tggagccctg ggtggtggtt cgagggctac gtcctgactt cacctatacc 240
   tttgaggtca ctgcattgaa cggggtatcc tccttagcca cggggcccgt cccatttgag 300
   cctgtcaatg tcaccactga ccgagaggta cctcctgcag tgtctgacat ccgggtgacg 360
   cggtcctcac ccagcagctt gagcctggcc tgggctgttc cccgggcacc cagtggggct 420
   gtgctggact acgaggtcaa ataccatgag aagggcgccg agggtcccag cagcgtgcgg 480
   ttcctgaaga cgtcagaaaa ccgggcagag ctgcgggggc tgaagcgggg agccagctac 540
   ctggtgcagg tacgggcgcg ctctgaggcc ggctacgggc ccttcggcca ggaacatcac 600
   agccagaccc aactggatga gagcgagggc tggcgggagc agctggccct g 651
<210> 11
   <211> 417
   <212> DNA
   <213> Homo sapiens
<400> 11
   ctggccgctg ctgatcgcca caccgtcttc tggaacagtt caaatcccaa gttccggaat 60
   gaggactaca ccatacatgt gcagctgaat gactacgtgg acatcatctg tccgcactat 120
   gaagatcact ctgtggcaga cgctgccatg gagcagtaca tactgtacct ggtggagcat 180
   gaggagtacc agctgtgcca gccccagtcc aaggaccaag tccgctggca gtgcaaccgg 240
   cccagtgcca agcatggccc ggagaagctg tctgagaagt tccagcgctt cacacctttc 300
   accctgggca aggagttcaa agaaggacac agctactact acatctccaa acccatccac 360
   cagcatgaag accgctgctt gaggttgaag gtgactgtca gtggcaaaat cactcac 417
<210> 12
   <211> 426
   <212> DNA
   <213> Homo sapiens
<400> 12
   tccaaatcga tagttttaga gcctatctat tggaattcct cgaactccaa atttctacct 60
   ggacaaggac tggtactata cccacagata ggagacaaat tggatattat ttgccccaaa 120
   gtggactcta aaactgttgg ccagtatgaa tattataaag tttatatggt tgataaagac 180
   caagcagaca gatgcactat taagaaggaa aatacccctc tcctcaactg tgccaaacca 240
   gaccaagata tcaaattcac catcaagttt caagaattca gccctaacct ctggggtcta 300
   gaatttcaga agaacaaaga ttattacatt atatctacat caaatgggtc tttggagggc 360
   ctggataacc aggagggagg ggtgtgccag acaagagcca tgaagatcct catgaaagtt 420
   ggacaa 426
<210> 13
   <211> 214
   <212> PRT
   <213> Homo sapiens
<400> 13
   Ser Lys Ala Ala Leu Leu Ala Ala Arg Gly Pro Glu Glu Leu Leu Cys
   1 5 10 15
   Phe Thr Glu Arg Leu Glu Asp Leu Val Cys Phe Trp Glu Glu Ala Ala
   20 25 30
   Ser Ala Gly Val Gly Pro Gly Asn Tyr Ser Phe Ser Tyr Gln Leu Glu
   35 40 45
   Asp Glu Pro Trp Lys Leu Cys Arg Leu His Gln Ala Pro Thr Ala Arg
   50 55 60
   Gly Ala Val Arg Phe Trp Cys Ser Leu Pro Thr Ala Asp Thr Ser Ser
   65 70 75 80
   Phe Val Pro Leu Glu Leu Arg Val Thr Ala Ala Ser Gly Ala Pro Arg
   85 90 95
   Tyr His Arg Val Ile His Ile Asn Glu Val Val Leu Leu Asp Ala Pro
   100 105 110
   Val Gly Leu Val Ala Arg Leu Ala Asp Glu Ser Gly His Val Val Leu
   115 120 125
   Arg Trp Leu Pro Pro Pro Glu Thr Pro Met Thr Ser His Ile Arg Tyr
   130 135 140
   Glu Val Asp Val Ser Ala Gly Asn Gly Ala Gly Ser Val Gln Arg Val
   145 150 155 160
   Glu Ile Leu Glu Gly Arg Thr Glu Cys Val Leu Ser Asn Leu Arg Gly
   165 170 175
   Arg Thr Arg Tyr Thr Phe Ala Val Arg Ala Arg Met Ala Glu Pro Ser
   180 185 190
   Phe Gly Gly Phe Trp Ser Ala Trp Ser Glu Pro Val Ser Leu Leu Thr
   195 200 205
   Pro Ser Asp Leu Asp Pro
   210
<210> 14
   <211> 217
   <212> PRT
   <213> Homo sapiens
<400> 14
   Pro Ser Ala Pro Arg Ser Val Val Ser Arg Leu Asn Gly Ser Ser Leu
   1               5                   10                  15
   His Leu Glu Trp Ser Ala Pro Leu Glu Ser Gly Gly Arg Glu Asp Leu
               20                  25                  30
   Thr Tyr Ala Leu Arg Cys Arg Glu Cys Arg Pro Gly Gly Ser Cys Ala
           35                  40                  45
   Pro Cys Gly Gly Asp Leu Thr Phe Asp Pro Gly Pro Arg Asp Leu Val
       50                  55                  60
   Glu Pro Trp Val Val Val Arg Gly Leu Arg Pro Asp Phe Thr Tyr Thr
   65                  70                  75                  80
   Phe Glu Val Thr Ala Leu Asn Gly Val Ser Ser Leu Ala Thr Gly Pro
                   85                  90                  95
   Val Pro Phe Glu Pro Val Asn Val Thr Thr Asp Arg Glu Val Pro Pro
               100                 105                 110
   Ala Val Ser Asp Ile Arg Val Thr Arg Ser Ser Pro Ser Ser Leu Ser
           115                 120                 125
   Leu Ala Trp Ala Val Pro Arg Ala Pro Ser Gly Ala Val Leu Asp Tyr
       130                 135                 140
   Glu Val Lys Tyr His Glu Lys Gly Ala Glu Gly Pro Ser Ser Val Arg
   145                 150                 155                 160
   Phe Leu Lys Thr Ser Glu Asn Arg Ala Glu Leu Arg Gly Leu Lys Arg
                   165                 170                 175
   Gly Ala Ser Tyr Leu Val Gln Val Arg Ala Arg Ser Glu Ala Gly Tyr
               180                 185                 190
   Gly Pro Phe Gly Gln Glu His His Ser Gln Thr Gln Leu Asp Glu Ser
           195                 200                 205
   Glu Gly Trp Arg Glu Gln Leu Ala Leu
       210                 215
<210> 15
   <211> 139
   <212> PRT
   <213> Homo sapiens
<400> 15
   Leu Ala Ala Ala Asp Arg His Thr Val Phe Trp Asn Ser Ser Asn Pro
   1               5                   10                  15
   Lys Phe Arg Asn Glu Asp Tyr Thr Ile His Val Gln Leu Asn Asp Tyr
               20                  25                  30
   Val Asp Ile Ile Cys Pro His Tyr Glu Asp His Ser Val Ala Asp Ala
           35                  40                  45
   Ala Met Glu Gln Tyr Ile Leu Tyr Leu Val Glu His Glu Glu Tyr Gln
       50                  55                  60
   Leu Cys Gln Pro Gln Ser Lys Asp Gln Val Arg Trp Gln Cys Asn Arg
   65                  70                  75                  80
   Pro Ser Ala Lys His Gly Pro Glu Lys Leu Ser Glu Lys Phe Gln Arg
                   85                  90                  95
   Phe Thr Pro Phe Thr Leu Gly Lys Glu Phe Lys Glu Gly His Ser Tyr
               100                 105                 110
   Tyr Tyr Ile Ser Lys Pro Ile His Gln His Glu Asp Arg Cys Leu Arg
           115                 120                 125
   Leu Lys Val Thr Val Ser Gly Lys Ile Thr His
       130                 135
<210> 16
   <211> 142
   <212> PRT
   <213> Homo sapiens
<400> 16
   Ser Lys Ser Ile Val Leu Glu Pro Ile Tyr Trp Asn Ser Ser Asn Ser
   1               5                   10                  15
   Lys Phe Leu Pro Gly Gln Gly Leu Val Leu Tyr Pro Gln Ile Gly Asp
               20                  25                  30
   Lys Leu Asp Ile Ile Cys Pro Lys Val Asp Ser Lys Thr Val Gly Gln
           35                  40                  45
   Tyr Glu Tyr Tyr Lys Val Tyr Met Val Asp Lys Asp Gln Ala Asp Arg
       50                  55                  60
   Cys Thr Ile Lys Lys Glu Asn Thr Pro Leu Leu Asn Cys Ala Lys Pro
   65                  70                  75                  80
   Asp Gln Asp Ile Lys Phe Thr Ile Lys Phe Gln Glu Phe Ser Pro Asn
   85 90 95
   Leu Trp Gly Leu Glu Phe Gln Lys Asn Lys Asp Tyr Tyr Ile Ile Ser
   100 105 110
   Thr Ser Asn Gly Ser Leu Glu Gly Leu Asp Asn Gln Glu Gly Gly Val
   115 120 125
   Cys Gln Thr Arg Ala Met Lys Ile Leu Met Lys Val Gly Gln
   130 135 140
<210> 17
   <211> 138
   <212> PRT
   <213> Homo sapiens
<400> 17
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Arg Val Gly Gln Gln Ala
   20 25 30
   Val Glu Val Trp Gln Gly Leu Ala Leu Leu Ser Glu Ala Val Leu Arg
   35 40 45
   Gly Gln Ala Leu Leu Val Asn Ser Ser Gln Pro Trp Glu Pro Leu Gln
   50 55 60
   Leu His Val Asp Lys Ala Val Ser Gly Leu Arg Ser Leu Thr Thr Leu
   65 70 75 80
   Leu Arg Ala Leu Gly Ala Gln Lys Glu Ala Ile Ser Pro Pro Asp Ala
   85 90 95
   Ala Ser Ala Ala Pro Leu Arg Thr Ile Thr Ala Asp Thr Phe Arg Lys
   100 105 110
   Leu Phe Arg Val Tyr Ser Asn Phe Leu Arg Gly Lys Leu Lys Leu Tyr
   115 120 125
   Thr Gly Glu Ala Cys Arg Thr Gly Asp Arg
   130 135
<210> 18
   <211> 147
   <212> PRT
   <213> Homo sapiens
<400> 18
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Leu Leu Val Asn
   50 55 60
   Ser Ser Gln Pro Trp Glu Pro Leu Gln Leu His Val Asp Lys Ala Val
   65 70 75 80
   Ser Gly Leu Arg Ser Leu Thr Thr Leu Leu Arg Ala Leu Gly Ala Gln
   85 90 95
   Lys Glu Ala Ile Ser Pro Pro Asp Ala Ala Ser Ala Ala Pro Leu Arg
   100 105 110
   Thr Ile Thr Ala Asp Thr Phe Arg Lys Leu Phe Arg Val Tyr Ser Asn
   115 120 125
   Phe Leu Arg Gly Lys Leu Lys Leu Tyr Thr Gly Glu Ala Cys Arg Thr
   130 135 140
   Gly Asp Arg
   145
<210> 19
   <211> 137
   <212> PRT
   <213> Homo sapiens
<400> 19
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Leu Leu Val Asn Ser Ser Gln Pro Trp Glu Pro Leu Gln Leu
       50                  55                  60
   His Val Asp Lys Ala Val Ser Gly Leu Arg Ser Leu Thr Thr Leu Leu
   65                  70                  75                  80
   Arg Ala Leu Gly Ala Gln Lys Glu Ala Ile Ser Pro Pro Asp Ala Ala
                   85                  90                  95
   Ser Ala Ala Pro Leu Arg Thr Ile Thr Ala Asp Thr Phe Arg Lys Leu
               100                 105                 110
   Phe Arg Val Tyr Ser Asn Phe Leu Arg Gly Lys Leu Lys Leu Tyr Thr
           115                 120                 125
   Gly Glu Ala Cys Arg Thr Gly Asp Arg
       130                 135
<210> 20
   <211> 135
   <212> PRT
   <213> Homo sapiens
<400> 20
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Pro Trp Glu Pro Leu Gln Leu His Val
   50 55 60
   Asp Lys Ala Val Ser Gly Leu Arg Ser Leu Thr Thr Leu Leu Arg Ala
   65 70 75 80
   Leu Gly Ala Gln Lys Glu Ala Ile Ser Pro Pro Asp Ala Ala Ser Ala
   85 90 95
   Ala Pro Leu Arg Thr Ile Thr Ala Asp Thr Phe Arg Lys Leu Phe Arg
   100 105 110
   Val Tyr Ser Asn Phe Leu Arg Gly Lys Leu Lys Leu Tyr Thr Gly Glu
   115 120 125
   Ala Cys Arg Thr Gly Asp Arg
   130 135
<210> 21
   <211> 60
   <212> PRT
   <213> Homo sapiens
<400> 21
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Pro Gly Val Gly Gln Leu
   35 40 45
   Phe Pro Ala Val Gly Ala Pro Ala Ala Ala Cys Gly
   50 55 60
<210> 22
   <211> 41
   <212> PRT
   <213> Homo sapiens
<400> 22
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Asn His Cys
   35 40
<210> 23
   <211> 26
   <212> PRT
   <213> Homo sapiens
<400> 23
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr
   20 25
<210> 24
   <211> 26
   <212> PRT
   <213> Homo sapiens
<400> 24
   Ala Pro Pro Arg Leu Ile Cys Asp Ser Arg Val Leu Glu Ala Tyr Leu
   1 5 10 15
   Leu Glu Ala Lys Glu Ala Glu Asn Ile Thr
   20 25
<210> 25
   <211> 26
   <212> PRT
   <213> Homo sapiens
<400> 25
   Ala Pro Pro Arg Leu Ile Cys Asp Ser Arg Val Leu Glu Glu Tyr Leu
   1 5 10 15
   Leu Glu Ala Lys Glu Ala Glu Asn Ile Thr
   20 25
<210> 26
   <211> 16
   <212> PRT
   <213> Homo sapiens
<400> 26
   Ala Pro Pro Arg Leu Ile Cys Asp Ser Arg Val Leu Glu Arg Tyr Leu
   1               5                   10                  15
<210> 27
   <211> 6
   <212> PRT
   <213> Homo sapiens
<400> 27
   Ala Pro Pro Arg Leu Ile
   1               5
<210> 28
   <211> 106
   <212> PRT
   <213> Homo sapiens
<400> 28
   Ala Pro Pro Arg Leu Ile Cys Asp Ser Arg Val Leu Glu Arg Tyr Ile
   1               5                   10                  15
   Leu Glu Ala Lys Glu Ala Glu Asn Val Thr Met Gly Cys Ala Glu Gly
               20                  25                  30
   Pro Arg Leu Ser Glu Asn Ile Thr Val Pro Asp Thr Lys Val Asn Phe
           35                  40                  45
   Tyr Ala Trp Lys Arg Met Glu Lys Glu Leu Met Ser Pro Pro Asp Thr
       50                  55                  60
   Thr Pro Pro Ala Pro Leu Arg Thr Leu Thr Val Asp Thr Phe Cys Lys
   65                  70                  75                  80
   Leu Phe Arg Val Tyr Ala Asn Phe Leu Arg Gly Lys Leu Lys Leu Tyr
                   85                  90                  95
   Thr Gly Glu Val Cys Arg Arg Gly Asp Arg
               100                 105
<210> 29
   <211> 153
   <212> PRT
   <213> Homo sapiens
<400> 29
   Ala Pro Pro Arg Leu Ile Cys Glu Ala Glu Asn Ile Thr Thr Gly Cys
   1               5                   10                  15
   Ala Glu His Cys Ser Leu Asn Glu Asn Ile Thr Val Pro Asp Thr Lys
               20                  25                  30
   Val Asn Phe Tyr Ala Trp Lys Arg Met Glu Val Gly Gln Gln Ala Val
           35                  40                  45
   Glu Val Trp Gln Gly Leu Ala Leu Leu Ser Glu Ala Val Leu Arg Gly
       50                  55                  60
   Gln Ala Leu Leu Val Asn Ser Ser Gln Pro Trp Glu Pro Leu Gln Leu
   65                  70                  75                  80
   His Val Asp Lys Ala Val Ser Gly Leu Arg Ser Leu Thr Thr Leu Leu
                   85                  90                  95
   Arg Ala Leu Gly Ala Gln Lys Glu Ala Ile Ser Pro Pro Asp Ala Ala
               100                 105                 110
   Ser Ala Ala Pro Leu Arg Thr Ile Thr Ala Asp Thr Phe Arg Lys Leu
           115                 120                 125
   Phe Arg Val Tyr Ser Asn Phe Leu Arg Gly Lys Leu Lys Leu Tyr Thr
       130                 135                 140
   Gly Glu Ala Cys Arg Thr Gly Asp Arg
   145                 150
<210> 30
   <211> 162
   <212> PRT
   <213> Homo sapiens
<400> 30
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Phe Tyr Ala Trp Lys
           35                  40                  45
   Arg Met Glu Val Gly Gln Gln Ala Val Glu Val Trp Gln Gly Leu Ala
       50                  55                  60
   Leu Leu Ser Glu Ala Val Leu Arg Gly Gln Ala Leu Leu Val Asn Ser
   65 70 75 80
   Ser Gln Pro Trp Glu Pro Leu Gln Leu His Val Asp Lys Ala Val Ser
   85 90 95
   Gly Leu Arg Ser Leu Thr Thr Leu Leu Arg Ala Leu Gly Ala Gln Lys
   100 105 110
   Glu Ala Ile Ser Pro Pro Asp Ala Ala Ser Ala Ala Pro Leu Arg Thr
   115 120 125
   Ile Thr Ala Asp Thr Phe Arg Lys Leu Phe Arg Val Tyr Ser Asn Phe
   130 135 140
   Leu Arg Gly Lys Leu Lys Leu Tyr Thr Gly Glu Ala Cys Arg Thr Gly
   145 150 155 160
   Asp Arg
<210> 31
   <211> 158
   <212> PRT
   <213> Homo sapiens
<400> 31
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Leu Thr Thr Leu Leu Arg Ala Leu Gly Ala Gln Lys Glu Ala Ile Ser
   100 105 110
   Pro Pro Asp Ala Ala Ser Ala Ala Pro Leu Arg Thr Ile Thr Ala Asp
   115 120 125
   Thr Phe Arg Lys Leu Phe Arg Val Tyr Ser Asn Phe Leu Arg Gly Lys
   130 135 140
   Leu Lys Leu Tyr Thr Gly Glu Ala Cys Arg Thr Gly Asp Arg
   145                 150                 155
<210> 32
   <211> 158
   <212> PRT
   <213> Homo sapiens
<400> 32
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Gly Lys
       130                 135                 140
   Leu Lys Leu Tyr Thr Gly Glu Ala Cys Arg Thr Gly Asp Arg
   145                 150                 155
<210> 33
   <211> 160
   <212> PRT
   <213> Homo sapiens
<400> 33
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr Thr Gly Glu Ala
   145 150 155 160
<210> 34
   <211> 159
   <212> PRT
   <213> Homo sapiens
<400> 34
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu
   145                 150                 155
<210> 35
   <211> 158
   <212> PRT
   <213> Homo sapiens
<400> 35
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly
   145                 150                 155
<210> 36
   <211> 157
   <212> PRT
   <213> Homo sapiens
<400> 36
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr Thr
   145 150 155
<210> 37
   <211> 156
   <212> PRT
   <213> Homo sapiens
<400> 37
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr
   145 150 155
<210> 38
   <211> 155
   <212> PRT
   <213> Homo sapiens
<400> 38
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu
   145 150 155
<210> 39
   <211> 154
   <212> PRT
   <213> Homo sapiens
<400> 39
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys
   145                 150
<210> 40
   <211> 153
   <212> PRT
   <213> Homo sapiens
<400> 40
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu
   145                 150
<210> 41
   <211> 152
   <212> PRT
   <213> Homo sapiens
<400> 41
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys
   145                 150
<210> 42
   <211> 151
   <212> PRT
   <213> Homo sapiens
<400> 42
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly
   145                 150
<210> 43
   <211> 150
   <212> PRT
   <213> Homo sapiens
<400> 43
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg
   145 150
<210> 44
   <211> 149
   <212> PRT
   <213> Homo sapiens
<400> 44
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu
   145
<210> 45
   <211> 148
   <212> PRT
   <213> Homo sapiens
<400> 45
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe
   145
<210> 46
   <211> 147
   <212> PRT
   <213> Homo sapiens
<400> 46
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn
   145
<210> 47
   <211> 146
   <212> PRT
   <213> Homo sapiens
<400> 47
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser
   145
<210> 48
   <211> 145
   <212> PRT
   <213> Homo sapiens
<400> 48
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr
   145
<210> 49
   <211> 144
   <212> PRT
   <213> Homo sapiens
<400> 49
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
<210> 50
   <211> 143
   <212> PRT
   <213> Homo sapiens
<400> 50
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg
       130                 135                 140
<210> 51
   <211> 142
   <212> PRT
   <213> Homo sapiens
<400> 51
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe
       130                 135                 140
<210> 52
   <211> 141
   <212> PRT
   <213> Homo sapiens
<400> 52
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu
       130                 135                 140
<210> 53
   <211> 140
   <212> PRT
   <213> Homo sapiens
<400> 53
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys
       130                 135                 140
<210> 54
   <211> 139
   <212> PRT
   <213> Homo sapiens
<400> 54
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg
       130                 135
<210> 55
   <211> 138
   <212> PRT
   <213> Homo sapiens
<400> 55
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe
       130                 135
<210> 56
   <211> 137
   <212> PRT
   <213> Homo sapiens
<400> 56
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr
       130                 135
<210> 57
   <211> 136
   <212> PRT
   <213> Homo sapiens
<400> 57
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp
   130 135
<210> 58
   <211> 135
   <212> PRT
   <213> Homo sapiens
<400> 58
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala
       130                 135
<210> 59
   <211> 134
   <212> PRT
   <213> Homo sapiens
<400> 59
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr
       130
<210> 60
   <211> 133
   <212> PRT
   <213> Homo sapiens
<400> 60
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile
       130
<210> 61
   <211> 132
   <212> PRT
   <213> Homo sapiens
<400> 61
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr
   130
<210> 62
   <211> 131
   <212> PRT
   <213> Homo sapiens
<400> 62
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg
       130
<210> 63
   <211> 130
   <212> PRT
   <213> Homo sapiens
<400> 63
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu
   130
<210> 64
   <211> 129
   <212> PRT
   <213> Homo sapiens
<400> 64
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro
<210> 65
   <211> 128
   <212> PRT
   <213> Homo sapiens
<400> 65
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
<210> 66
   <211> 127
   <212> PRT
   <213> Homo sapiens
<400> 66
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala
   115 120 125
<210> 67
   <211> 126
   <212> PRT
   <213> Homo sapiens
<400> 67
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser
   115 120 125
<210> 68
   <211> 125
   <212> PRT
   <213> Homo sapiens
<400> 68
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala
           115                 120                 125
<210> 69
   <211> 124
   <212> PRT
   <213> Homo sapiens
<400> 69
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala
   115 120
<210> 70
   <211> 123
   <212> PRT
   <213> Homo sapiens
<400> 70
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
                   20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp
           115                 120
<210> 71
   <211> 122
   <212> PRT
   <213> Homo sapiens
<400> 71
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro
           115                 120
<210> 72
   <211> 121
   <212> PRT
   <213> Homo sapiens
<400> 72
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro
           115                 120
<210> 73
   <211> 120
   <212> PRT
   <213> Homo sapiens
<400> 73
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser
           115                 120
<210> 74
   <211> 119
   <212> PRT
   <213> Homo sapiens
<400> 74
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile
   115
<210> 75
   <211> 118
   <212> PRT
   <213> Homo sapiens
<400> 75
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala
   115
<210> 76
   <211> 117
   <212> PRT
   <213> Homo sapiens
<400> 76
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu
   115
<210> 77
   <211> 116
   <212> PRT
   <213> Homo sapiens
<400> 77
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys
           115
<210> 78
   <211> 115
   <212> PRT
   <213> Homo sapiens
<400> 78
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln
           115
<210> 79
   <211> 114
   <212> PRT
   <213> Homo sapiens
<400> 79
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala
<210> 80
   <211> 113
   <212> PRT
   <213> Homo sapiens
<400> 80
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly
<210> 81
   <211> 112
   <212> PRT
   <213> Homo sapiens
<400> 81
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
<210> 82
   <211> 111
   <212> PRT
   <213> Homo sapiens
<400> 82
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala
               100                 105                 110
<210> 83
   <211> 110
   <212> PRT
   <213> Homo sapiens
<400> 83
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg
   100 105 110
<210> 84
   <211> 109
   <212> PRT
   <213> Homo sapiens
<400> 84
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu
   100 105
<210> 85
   <211> 108
   <212> PRT
   <213> Homo sapiens
<400> 85
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu
               100                 105
<210> 86
   <211> 107
   <212> PRT
   <213> Homo sapiens
<400> 86
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr
   100 105
<210> 87
   <211> 106
   <212> PRT
   <213> Homo sapiens
<400> 87
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr
   100 105
<210> 88
   <211> 105
   <212> **PRT**
   <213> Homo sapiens
<400> 88
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu
   100 105
<210> 89
   <211> 104
   <212> PRT
   <213> Homo sapiens
<400> 89
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser
   100
<210> 90
   <211> 103
   <212> PRT
   <213> Homo sapiens
<400> 90
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg
               100
<210> 91
   <211> 102
   <212> PRT
   <213> Homo sapiens
<400> 91
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu
   100
<210> 92
   <211> 101
   <212> PRT
   <213> Homo sapiens
<400> 92
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly
   100
<210> 93
   <211> 100
   <212> PRT
   <213> Homo sapiens
<400> 93
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser
               100
<210> 94
   <211> 99
   <212> PRT
   <213> Homo sapiens
<400> 94
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val
<210> 95
   <211> 98
   <212> PRT
   <213> Homo sapiens
<400> 95
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala
<210> 96
   <211> 97
   <212> PRT
   <213> Homo sapiens
<400> 96
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys
<210> 97
   <211> 96
   <212> PRT
   <213> Homo sapiens
<400> 97
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
<210> 98
   <211> 95
   <212> PRT
   <213> Homo sapiens
<400> 98
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val
                   85                  90                  95
<210> 99
   <211> 94
   <212> PRT
   <213> Homo sapiens
<400> 99
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His
                   85                  90
<210> 100
   <211> 93
   <212> PRT
   <213> Homo sapiens
<400> 100
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu
   85 90
<210> 101
   <211> 92
   <212> PRT
   <213> Homo sapiens
<400> 101
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln
   85 90
<210> 102
   <211> 91
   <212> PRT
   <213> Homo sapiens
<400> 102
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu
                   85                  90
<210> 103
   <211> 90
   <212> **PRT**
   <213> Homo sapiens
<400> 103
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro
                   85                  90
<210> 104
   <211> 89
   <212> PRT
   <213> Homo sapiens
<400> 104
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu
                   85
<210> 105
   <211> 88
   <212> PRT
   <213> Homo sapiens
<400> 105
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp
                   85
<210> 106
   <211> 87
   <212> PRT
   <213> Homo sapiens
<400> 106
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro
   85
<210> 107
   <211> 86
   <212> PRT
   <213> Homo sapiens
<400> 107
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln
   85
<210> 108
   <211> 85
   <212> PRT
   <213> Homo sapiens
<400> 108
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser
                   85
<210> 109
   <211> 84
   <212> PRT
   <213> Homo sapiens
<400> 109
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser
<210> 110
   <211> 83
   <212> PRT
   <213> Homo sapiens
<400> 110
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn
<210> 111
   <211> 82
   <212> PRT
   <213> Homo sapiens
<400> 111
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val
<210> 112
   <211> 81
   <212> PRT
   <213> Homo sapiens
<400> 112
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu
<210> 113
   <211> 80
   <212> PRT
   <213> Homo sapiens
<400> 113
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
<210> 114
   <211> 79
   <212> PRT
   <213> Homo sapiens
<400> 114
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala
   65                  70                  75
<210> 115
   <211> 78
   <212> PRT
   <213> Homo sapiens
<400> 115
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln
   65                  70                  75
<210> 116
   <211> 77
   <212> PRT
   <213> Homo sapiens
<400> 116
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly
   65 70 75
<210> 117
   <211> 76
   <212> PRT
   <213> Homo sapiens
<400> 117
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg
   65 70 75
<210> 118
   <211> 75
   <212> PRT
   <213> Homo sapiens
<400> 118
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu
   65 70 75
<210> 119
   <211> 74
   <212> PRT
   <213> Homo sapiens
<400> 119
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val
   65                  70
<210> 120
   <211> 73
   <212> PRT
   <213> Homo sapiens
<400> 120
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala
   65 70
<210> 121
   <211> 72
   <212> **PRT**
   <213> Homo sapiens
<400> 121
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu
   65 70
<210> 122
   <211> 71
   <212> PRT
   <213> Homo sapiens
<400> 122
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser
   65 70
<210> 123
   <211> 70
   <212> PRT
   <213> Homo sapiens
<400> 123
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu
   65 70
<210> 124
   <211> 69
   <212> PRT
   <213> Homo sapiens
<400> 124
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu
   65
<210> 125
   <211> 68
   <212> **PRT**
   <213> Homo sapiens
<400> 125
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala
   65
<210> 126
   <211> 67
   <212> PRT
   <213> Homo sapiens
<400> 126
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu
   65
<210> 127
   <211> 67
   <212> PRT
   <213> Homo sapiens
<400> 127
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu
   65
<210> 128
   <211> 65
   <212> **PRT**
   <213> Homo sapiens
<400> 128
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln
   65
<210> 129
   <211> 64
   <212> PRT
   <213> Homo sapiens
<400> 129
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
<210> 130
   <211> 63
   <212> PRT
   <213> Homo sapiens
<400> 130
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val
   50 55 60
<210> 131
   <211> 62
   <212> PRT
   <213> Homo sapiens
<400> 131
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu
       50                  55                  60
<210> 132
   <211> 61
   <212> PRT
   <213> Homo sapiens
<400> 132
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val
       50                  55                  60
<210> 133
   <211> 60
   <212> PRT
   <213> Homo sapiens
<400> 133
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala
       50                  55                  60
<210> 134
   <211> 59
   <212> PRT
   <213> Homo sapiens
<400> 134
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln
   50 55
<210> 135
   <211> 58
   <212> PRT
   <213> Homo sapiens
<400> 135
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln
       50                  55
<210> 136
   <211> 57
   <212> PRT
   <213> Homo sapiens
<400> 136
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly
       50                  55
<210> 137
   <211> 56
   <212> PRT
   <213> Homo sapiens
<400> 137
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val
       50                  55
<210> 138
   <211> 55
   <212> PRT
   <213> Homo sapiens
<400> 138
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu
       50                  55
<210> 139
   <211> 54
   <212> PRT
   <213> Homo sapiens
<400> 139
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met
       50
<210> 140
   <211> 53
   <212> PRT
   <213> Homo sapiens
<400> 140
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg
       50
<210> 141
   <211> 52
   <212> PRT
   <213> Homo sapiens
<400> 141
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys
       50
<210> 142
   <211> 51
   <212> PRT
   <213> Homo sapiens
<400> 142
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp
       50
<210> 143
   <211> 50
   <212> PRT
   <213> Homo sapiens
<400> 143
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala
   50
<210> 144
   <211> 49
   <212> PRT
   <213> Homo sapiens
<400> 144
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr
<210> 145
   <211> 48
   <212> PRT
   <213> Homo sapiens
<400> 145
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
<210> 146
   <211> 47
   <212> PRT
   <213> Homo sapiens
<400> 146
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn
   35 40 45
<210> 147
   <211> 46
   <212> **PRT**
   <213> Homo sapiens
<400> 147
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val
           35                  40                  45
<210> 148
   <211> 45
   <212> PRT
   <213> Homo sapiens
<400> 148
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys
           35                  40                  45
<210> 149
   <211> 44
   <212> PRT
   <213> Homo sapiens
<400> 149
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr
           35                  40
<210> 150
   <211> 43
   <212> PRT
   <213> Homo sapiens
<400> 150
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp
           35                  40
<210> 151
   <211> 42
   <212> PRT
   <213> Homo sapiens
<400> 151
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro
           35                  40
<210> 152
   <211> 41
   <212> PRT
   <213> Homo sapiens
<400> 152
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val
           35                  40
<210> 153
   <211> 40
   <212> PRT
   <213> Homo sapiens
<400> 153
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr
   35 40
<210> 154
   <211> 39
   <212> PRT
   <213> Homo sapiens
<400> 154
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile
   35
<210> 155
   <211> 38
   <212> PRT
   <213> Homo sapiens
<400> 155
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn
   35
<210> 156
   <211> 37
   <212> PRT
   <213> Homo sapiens
<400> 156
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu
           35
<210> 157
   <211> 36
   <212> PRT
   <213> Homo sapiens
<400> 157
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn
           35
<210> 158
   <211> 35
   <212> PRT
   <213> Homo sapiens
<400> 158
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu
           35
<210> 159
   <211> 34
   <212> PRT
   <213> Homo sapiens
<400> 159
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser
<210> 160
   <211> 33
   <212> PRT
   <213> Homo sapiens
<400> 160
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys
<210> 161
   <211> 32
   <212> PRT
   <213> Homo sapiens
<400> 161
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
<210> 162
   <211> 31
   <212> PRT
   <213> Homo sapiens
<400> 162
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu
   20 25 30
<210> 163
   <211> 30
   <212> PRT
   <213> Homo sapiens
<400> 163
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala
               20                  25                  30
<210> 164
   <211> 29
   <212> PRT
   <213> Homo sapiens
<400> 164
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys
               20                  25
<210> 165
   <211> 28
   <212> PRT
   <213> Homo sapiens
<400> 165
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly
               20                  25
<210> 166
   <211> 27
   <212> PRT
   <213> Homo sapiens
<400> 166
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr
               20                  25
<210> 167
   <211> 26
   <212> PRT
   <213> Homo sapiens
<400> 167
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr
               20                  25
<210> 168
   <211> 25
   <212> PRT
   <213> Homo sapiens
<400> 168
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile
               20                  25
<210> 169
   <211> 24
   <212> PRT
   <213> Homo sapiens
<400> 169
   Ala Pro Pro Arg Leu Ile Cys Asp Ser Arg Val Leu Glu Arg Tyr Leu
   1               5                   10                  15
   Leu Glu Ala Lys Glu Ala Glu Asn
               20
<210> 170
   <211> 23
   <212> PRT
   <213> Homo sapiens
<400> 170
   Ala Pro Pro Arg Leu Ile Cys Asp Ser Arg Val Leu Glu Arg Tyr Leu
   1               5                   10                  15
   Leu Glu Ala Lys Glu Ala Glu
               20
<210> 171
   <211> 22
   <212> PRT
   <213> Homo sapiens
<400> 171
   Ala Pro Pro Arg Leu Ile Cys Asp Ser Arg Val Leu Glu Arg Tyr Leu
   1               5                   10                  15
   Leu Glu Ala Lys Glu Ala
               20
<210> 172
   <211> 21
   <212> PRT
   <213> Homo sapiens
<400> 172
   Ala Pro Pro Arg Leu Ile Cys Asp Ser Arg Val Leu Glu Arg Tyr Leu
   1               5                   10                  15
   Leu Glu Ala Lys Glu
               20
<210> 173
   <211> 20
   <212> PRT
   <213> Homo sapiens
<400> 173
   Ala Pro Pro Arg Leu Ile Cys Asp Ser Arg Val Leu Glu Arg Tyr Leu
   1               5                   10                  15
   Leu Glu Ala Lys
               20
<210> 174
   <211> 19
   <212> PRT
   <213> Homo sapiens
<400> 174
   Ala Pro Pro Arg Leu Ile Cys Asp Ser Arg Val Leu Glu Arg Tyr Leu
   1               5                   10                  15
   Leu Glu Ala
<210> 175
   <211> 18
   <212> PRT
   <213> Homo sapiens
<400> 175
   Ala Pro Pro Arg Leu Ile Cys Asp Ser Arg Val Leu Glu Arg Tyr Leu
   1 5 10 15
   Leu Glu
<210> 176
   <211> 17
   <212> PRT
   <213> Homo sapiens
<400> 176
   Ala Pro Pro Arg Leu Ile Cys Asp Ser Arg Val Leu Glu Arg Tyr Leu
   1 5 10 15
   Leu
<210> 177
   <211> 16
   <212> PRT
   <213> Homo sapiens
<400> 177
   Ala Pro Pro Arg Leu Ile Cys Asp Ser Arg Val Leu Glu Arg Tyr Leu
   1 5 10 15
<210> 178
   <211> 15
   <212> PRT
   <213> Homo sapiens
<400> 178
   Ala Pro Pro Arg Leu Ile Cys Asp Ser Arg Val Leu Glu Arg Tyr
   1 5 10 15
<210> 179
   <211> 14
   <212> PRT
   <213> Homo sapiens
<400> 179
   Ala Pro Pro Arg Leu Ile Cys Asp Ser Arg Val Leu Glu Arg
   1 5 10
<210> 180
   <211> 13
   <212> PRT
   <213> Homo sapiens
<400> 180
   Ala Pro Pro Arg Leu Ile Cys Asp Ser Arg Val Leu Glu
   1               5                   10
<210> 181
   <211> 12
   <212> PRT
   <213> Homo sapiens
<400> 181
   Ala Pro Pro Arg Leu Ile Cys Asp Ser Arg Val Leu
   1               5                   10
<210> 182
   <211> 11
   <212> PRT
   <213> Homo sapiens
<400> 182
   Ala Pro Pro Arg Leu Ile Cys Asp Ser Arg Val
   1               5                   10
<210> 183
   <211> 10
   <212> PRT
   <213> Homo sapiens
<400> 183
   Ala Pro Pro Arg Leu Ile Cys Asp Ser Arg
   1               5                   10
<210> 184
   <211> 9
   <212> PRT
   <213> Homo sapiens
<400> 184
   Ala Pro Pro Arg Leu Ile Cys Asp Ser
   1               5
<210> 185
   <211> 8
   <212> PRT
   <213> Homo sapiens
<400> 185
   Ala Pro Pro Arg Leu Ile Cys Asp
   1               5
<210> 186
   <211> 7
   <212> PRT
   <213> Homo sapiens
<400> 186
   Ala Pro Pro Arg Leu Ile Cys
   1 5
<210> 187
   <211> 166
   <212> PRT
   <213> Homo sapiens
<400> 187
   Ala Pro Pro Arg Leu Ile Cys Ala Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr Thr Gly Glu Ala
   145 150 155 160
   Cys Arg Thr Gly Asp Arg
   165
<210> 188
   <211> 166
   <212> PRT
   <213> Homo sapiens
<400> 188
   Ala Pro Pro Arg Leu Ile Cys Arg Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
                   165
<210> 189
   <211> 166
   <212> PRT
   <213> Homo sapiens
<400> 189
   Ala Pro Pro Arg Leu Ile Cys Asp Ser Arg Val Leu Glu Ala Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr Thr Gly Glu Ala
   145 150 155 160
   Cys Arg Thr Gly Asp Arg
   165
<210> 190
   <211> 166
   <212> PRT
   <213> Homo sapiens
<400> 190
   Ala Pro Pro Arg Leu Ile Cys Asp Ser Arg Val Leu Glu Glu Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
                       165
<210> 191
   <211> 166
   <212> PRT
   <213> Homo sapiens
<400> 191
   Ala Pro Pro Arg Leu Ile Cys Asp Ser Arg Val Leu Glu Arg Tyr Leu
   1 5 10 15
   Leu Glu Ala Ala Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr Thr Gly Glu Ala
   145 150 155 160
   Cys Arg Thr Gly Asp Arg
   165
<210> 192
   <211> 166
   <212> PRT
   <213> Homo sapiens
<400> 192
   Ala Pro Pro Arg Leu Ile Cys Asp Ser Arg Val Leu Glu Arg Tyr Leu
   1 5 10 15
   Leu Glu Ala Glu Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
                   165
<210> 193
   <211> 166
   <212> PRT
   <213> Homo sapiens
<400> 193
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Ala Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
                   165
<210> 194
   <211> 166
   <212> PRT
   <213> Homo sapiens
<400> 194
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Ala Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr Thr Gly Glu Ala
   145 150 155 160
   Cys Arg Thr Gly Asp Arg
   165
<210> 195
   <211> 166
   <212> PRT
   <213> Homo sapiens
<400> 195
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Glu Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
                   165
<210> 196
   <211> 166
   <212> PRT
   <213> Homo sapiens
<400> 196
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Ala Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr Thr Gly Glu Ala
   145 150 155 160
   Cys Arg Thr Gly Asp Arg
   165
<210> 197
   <211> 166
   <212> PRT
   <213> Homo sapiens
<400> 197
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Ala Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr Thr Gly Glu Ala
   145 150 155 160
   Cys Arg Thr Gly Asp Arg
   165
<210> 198
   <211> 166
   <212> PRT
   <213> Homo sapiens
<400> 198
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Ala Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
   165
<210> 199
   <211> 166
   <212> PRT
   <213> Homo sapiens
<400> 199
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Glu Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
                   165
<210> 200
   <211> 166
   <212> PRT
   <213> Homo sapiens
<400> 200
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ala Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr Thr Gly Glu Ala
   145 150 155 160
   Cys Arg Thr Gly Asp Arg
   165
<210> 201
   <211> 166
   <212> PRT
   <213> Homo sapiens
<400> 201
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Ala Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Arg Gly Lys Leu Lys Leu Tyr Thr Gly Glu Ala
   145 150 155 160
   Cys Arg Thr Gly Asp Arg
   165
<210> 202
   <211> 166
   <212> PRT
   <213> Homo sapiens
<400> 202
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Glu Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
                   165
<210> 203
   <211> 166
   <212> PRT
   <213> Homo sapiens
<400> 203
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ala Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp Arg
                   165
<210> 204
   <211> 166
   <212> PRT
   <213> Homo sapiens
<400> 204
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Ala Val
   130 135 140
   Tyr Ser Asn Phe Leu Arg Gly Lys Leu Lys Leu Tyr Thr Gly Glu Ala
   145 150 155 160
   Cys Arg Thr Gly Asp Arg
   165
<210> 205
   <211> 166
   <212> PRT
   <213> Homo sapiens
<400> 205
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Glu Val
   130 135 140
   Tyr Ser Asn Phe Leu Arg Gly Lys Leu Lys Leu Tyr Thr Gly Glu Ala
   145 150 155 160
   Cys Arg Thr Gly Asp Arg
   165
<210> 206
   <211> 166
   <212> PRT
   <213> Homo sapiens
<400> 206
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Ala Phe Leu Arg Gly Lys Leu Lys Leu Tyr Thr Gly Glu Ala
   145                 150                 155                 160
   Cys Arg Thr Gly Asp Arg
                   165
<210> 207
   <211> 166
   <212> PRT
   <213> Homo sapiens
<400> 207
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
   35 40 45
   Tyr Ala Trp Lys Arg Met Glu Val Gly Gln Gln Ala Val Glu Val Trp
   50 55 60
   Gln Gly Leu Ala Leu Leu Ser Glu Ala Val Leu Arg Gly Gln Ala Leu
   65 70 75 80
   Leu Val Asn Ser Ser Gln Pro Trp Glu Pro Leu Gln Leu His Val Asp
   85 90 95
   Lys Ala Val Ser Gly Leu Arg Ser Leu Thr Thr Leu Leu Arg Ala Leu
   100 105 110
   Gly Ala Gln Lys Glu Ala Ile Ser Pro Pro Asp Ala Ala Ser Ala Ala
   115 120 125
   Pro Leu Arg Thr Ile Thr Ala Asp Thr Phe Arg Lys Leu Phe Arg Val
   130 135 140
   Tyr Ser Asn Phe Leu Ala Gly Lys Leu Lys Leu Tyr Thr Gly Glu Ala
   145 150 155 160
   Cys Arg Thr Gly Asp Arg
   165
<210> 208
   <211> 166
   <212> PRT
   <213> Homo sapiens
<400> 208
   Ala Pro Pro Arg Leu Ile Cys Asp Ser Arg Val Leu Glu Arg Tyr Leu
   1 5 10 15
   Leu Glu Ala Lys Glu Ala Glu Asn Ile Thr Thr Gly Cys Ala Glu His
   20 25 30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Glu Gly Lys Leu Lys Leu Tyr Thr Gly Glu Ala
   145                 150                 155                 160
   Cys Arg Thr Gly Asp Arg
                   165
<210> 209
   <211> 27
   <212> PRT
   <213> Homo sapiens
<400> 209
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr
               20                  25
<210> 210
   <211> 21
   <212> PRT
   <213> Homo sapiens
<400> 210
   Asn Glu Asn Ile Thr Val Pro Asp Thr Lys Val Asn Phe Tyr Ala Trp
   1               5                   10                  15
   Lys Arg Met Glu Val
               20
<210> 211
   <211> 27
   <212> PRT
   <213> Homo sapiens
<400> 211
   Asn Ser Ser Gln Pro Trp Glu Pro Leu Gln Leu His Val Asp Lys Ala
   1               5                   10                  15
   Val Ser Gly Leu Arg Ser Leu Thr Thr Leu Leu
               20                  25
<210> 212
   <211> 18
   <212> PRT
   <213> Homo sapiens
<400> 212
   Phe Arg Lys Leu Phe Arg Val Tyr Ser Asn Phe Leu Arg Gly Lys Leu
   1               5                   10                  15
   Lys Leu
<210> 213
   <211> 193
   <212> PRT
   <213> Homo sapiens
<400> 213
   Met Gly Val His Glu Cys Pro Ala Trp Leu Trp Leu Leu Leu Ser Leu
   1 5 10 15
   Leu Ser Leu Pro Leu Gly Leu Pro Val Leu Gly Ala Pro Pro Arg Leu
   20 25 30
   Ile Cys Asp Ser Arg Val Leu Glu Arg Tyr Leu Leu Glu Ala Lys Glu
   35 40 45
   Ala Glu Asn Ile Thr Thr Gly Cys Ala Glu His Cys Ser Leu Asn Glu
   50 55 60
   Asn Ile Thr Val Pro Asp Thr Lys Val Asn Phe Tyr Ala Trp Lys Arg
   65 70 75 80
   Met Glu Val Gly Gln Gln Ala Val Glu Val Trp Gln Gly Leu Ala Leu
   85 90 95
   Leu Ser Glu Ala Val Leu Arg Gly Gln Ala Leu Leu Val Asn Ser Ser
   100 105 110
   Gln Pro Trp Glu Pro Leu Gln Leu His Val Asp Lys Ala Val Ser Gly
   115 120 125
   Leu Arg Ser Leu Thr Thr Leu Leu Arg Ala Leu Gly Ala Gln Lys Glu
   130 135 140
   Ala Ile Ser Pro Pro Asp Ala Ala Ser Ala Ala Pro Leu Arg Thr Ile
   145 150 155 160
   Thr Ala Asp Thr Phe Arg Lys Leu Phe Arg Val Tyr Ser Asn Phe Leu
   165 170 175
   Arg Gly Lys Leu Lys Leu Tyr Thr Gly Glu Ala Cys Arg Thr Gly Asp
   180 185 190
   Arg
<210> 214
   <211> 181
   <212> PRT
   <213> Homo sapiens
<400> 214
   Glu Glu Thr Leu Leu Asn Thr Lys Leu Glu Thr Ala Asp Leu Lys Trp
   1 5 10 15
   Val Thr Phe Pro Gln Val Asp Gly Gln Trp Glu Glu Leu Ser Gly Leu
   20 25 30
   Asp Glu Glu Gln His Ser Val Arg Thr Tyr Glu Val Cys Asp Val Gln
   35 40 45
   Arg Ala Pro Gly Gln Ala His Trp Leu Arg Thr Gly Trp Val Pro Arg
   50 55 60
   Arg Gly Ala Val His Val Tyr Ala Thr Leu Arg Phe Thr Met Leu Glu
   65 70 75 80
   Cys Leu Ser Leu Pro Arg Ala Gly Arg Ser Cys Lys Glu Thr Phe Thr
   85 90 95
   Val Phe Tyr Tyr Glu Ser Asp Ala Asp Thr Ala Thr Ala Leu Thr Pro
   100 105 110
   Ala Trp Met Glu Asn Pro Tyr Ile Lys Val Asp Thr Val Ala Ala Glu
   115 120 125
   His Leu Thr Arg Lys Arg Pro Gly Ala Glu Ala Thr Gly Lys Val Asn
   130 135 140
   Val Lys Thr Leu Arg Leu Gly Pro Leu Ser Lys Ala Gly Phe Tyr Leu
   145 150 155 160
   Ala Phe Gln Asp Gln Gly Ala Cys Met Ala Leu Leu Ser Leu His Leu
   165 170 175
   Phe Tyr Lys Lys Cys
   180
<210> 215
   <211> 199
   <212> PRT
   <213> Homo sapiens
<400> 215
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Leu
           115                 120                 125
   Thr Pro Ala Trp Met Glu Asn Pro Tyr Ile Lys Val Asp Thr Val Ala
       130                 135                 140
   Ala Glu His Leu Thr Arg Lys Arg Pro Gly Ala Glu Ala Thr Gly Lys
   145                 150                 155                 160
   Val Asn Val Lys Thr Leu Arg Leu Gly Pro Leu Ser Lys Ala Gly Phe
                   165                 170                 175
   Tyr Leu Ala Phe Gln Asp Gln Gly Ala Cys Met Ala Leu Leu Ser Leu
               180                 185                 190
   His Leu Phe Tyr Lys Lys Cys
           195
<210> 216
   <211> 206
   <212> PRT
   <213> Homo sapiens
<400> 216
   Glu Glu Thr Leu Leu Asn Thr Lys Leu Glu Thr Ala Asp Leu Lys Trp
   1               5                   10                  15
   Val Thr Phe Pro Gln Val Asp Gly Gln Trp Glu Glu Leu Ser Gly Leu
               20                  25                  30
   Asp Glu Glu Gln His Ser Val Arg Thr Tyr Glu Val Cys Asp Val Gln
           35                  40                  45
   Arg Ala Pro Gly Gln Ala His Trp Leu Arg Thr Gly Trp Val Pro Arg
       50                  55                  60
   Arg Gly Ala Val His Val Tyr Ala Thr Leu Arg Phe Thr Met Leu Glu
   65                  70                  75                  80
   Cys Leu Ser Leu Pro Arg Ala Gly Arg Ser Cys Lys Glu Thr Phe Thr
                   85                  90                  95
   Val Phe Tyr Tyr Glu Ser Asp Ala Asp Thr Ala Thr Ala Leu Ser Glu
               100                 105                 110
   Ala Val Leu Arg Gly Gln Ala Leu Leu Val Asn Ser Ser Gln Pro Trp
           115                 120                 125
   Glu Pro Leu Gln Leu His Val Asp Lys Ala Val Ser Gly Leu Arg Ser
       130                 135                 140
   Leu Thr Thr Leu Leu Arg Ala Leu Gly Ala Gln Lys Glu Ala Ile Ser
   145                 150                 155                 160
   Pro Pro Asp Ala Ala Ser Ala Ala Pro Leu Arg Thr Ile Thr Ala Asp
                   165                 170                 175
   Thr Phe Arg Lys Leu Phe Arg Val Tyr Ser Asn Phe Leu Arg Gly Lys
               180                 185                 190
   Leu Lys Leu Tyr Thr Gly Glu Ala Cys Arg Thr Gly Asp Arg
           195                 200                 205
<210> 217
   <211> 20
   <212> DNA
   <213> Homo sapiens
<400> 217
   atggaggcct cgctcagaaa
<210> 218
   <211> 20
   <212> DNA
   <213> Homo sapiens
<400> 218
   tacctgaagg tcaggcgaac 20
<210> 219
   <211> 21
   <212> RNA
   <213> Homo sapiens
<400> 219
   ggugaauguc aagacgcugu u 21
<210> 220
   <211> 21
   <212> RNA
   <213> Homo sapiens
<400> 220
   uuccacuuac aguucugcga c 21
<210> 221
   <211> 21
   <212> RNA
   <213> Homo sapiens
<400> 221
   aguuaauauc aagacgcugu u 21
<210> 222
   <211> 21
   <212> RNA
   <213> Homo sapiens
<400> 222
   uuucaauuau aguucugcga c 21
<210> 223
   <211> 21
   <212> RNA
   <213> Artificial
<220>
   <223> GF Protein siRNA
<400> 223
   cgcugacccu gaaguucauu u 21
<210> 224
   <211> 21
   <212> RNA
   <213> Artificial
<220>
   <223> siRNA GFP
<400> 224
   uugcgacugg gacuucaagu a 21

1. A method for assessing tumour tissue for expression of EPH-B4 and/or Ephrin Al, comprising:
   (a) isolating a tissue sample from an individual who is receiving or shall receive erythropoietin,
   (b) determining the level of expression of the EPH-B4 and/or Ephrin Al
   (c) correlating the presence of these component gene expression products to a negative physiological response to the treatment with erythropoietin.
2. The method according to 1, wherein the level of expression of the component genes (mRNA) is determined by a molecular biological technique selected from the group consisting of PCR, QPCR, R-PCR, gene expression microarray analysis, northern-blot analysis, reverse transcription and amplification, zymography, ligase-chain-reaction, NASBA, RNase Protection Assay (RPA), capillary electrophoresis with laser induced fluorescence (CE-LIF).
3. The method according to 1 or 2, wherein the individual is a cancer patient who is to be treated with erythropoietin or is being treated with erythropoietin.
4. The method according to any one of 1 to 3, wherein the presence of EPH-B4 and/or Ephrin A1 gene expression products is indicative of poorer loco-regional tumor control and poorer patient survival upon treatment with erythropoietin.
5. The method according to any one of 1 to 3, wherein the presence of a higher level of EPH-B4 and/or Ephrin A1 gene expression products is indicative of poorer loco-regional tumour control and poorer patient survival upon treatment with erythropoietin.
6. The method according to any one of 1 or 3 to 5, wherein the means for testing for the presence of the gene expression products are a protein array or binding to a mass microbalance instrument.
7. The method according to any one of 1 or 3 to 5, wherein the determination of the presence of the EPH-B4 and/or Ephrin A1 gene products is done by detecting the respective proteins with an immunoassay procedure, where the immunoassay procedure is selected from the group of immunoprecipitation, enzyme immunoassay (EIA), radioimmunoassay (RIA) or fluorescent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay.
8. The method according to 7, wherein the immunoassay is an ELISA.
9. The method according to any one of 1 to 8, wherein the tissue sample may be selected from the cancerous tissue or circulating cells derived from same or from a group of biological tissues and fluids such as blood, lymph, urine, cerebral fluid.
10. A prognostic method to stratify patients having a tumour as suitable (EPH-B4 and/or Ephrin Al) or non-suitable (EPH-B4 and/or Ephrin Al) for EPO treatment, comprising:
   (a) isolating a tissue sample from an individual who is receiving or shall receive erythropoietin;
   (b) determining the level of expression of the EPH-B4 and/or Ephrin A1 gene(s) component, a EPH-B4 and/or Ephrin A1 from the isolated tissue; and
   (c) correlating the presence of EPH-B4 and/or Ephrin A1 component gene expression products to a negative physiological response to the treatment with erythropoietin.
11. The prognostic method according to 10, wherein the level of expression of EPH-B4 and/or Ephrin A1 component genes is determined by a molecular biological technique selected from the group consisting of PCR, QPCR, R-PCR, gene expression microarray analysis, northern-blot analysis, reverse transcription and amplification, zymography, ligase-chain-reaction, NASBA, RNase Protection Assay (RPA), capillary electrophoresis with laser induced fluorescence (CE-LIF).
12. The prognostic method according to 10, wherein the determination of the presence of the EPH-B4 and/or Ephrin A1 gene products is done by detecting the respective protein with an immunoassay procedure, where the immunoassay procedure is selected from the group of ELISA, immunoprecipitation, enzyme immunoassay (EIA), radioimmunoassay (RIA) or fluorescent immunoassay, a chemiluminescent assay, an agglutination assay, nephelometric assay, turbidimetric assay, a Western blot, a competitive immunoassay, a noncompetitive immunoassay, a homogeneous immunoassay a heterogeneous immunoassay, a bioassay and a reporter-assay such as a luciferase-assay.
13. The prognostic method according to any one of 10 to 12, wherein the tissue sample is selected from the cancerous tissue or circulating cells derived from same, or from a group of biological tissues and fluids such as blood, lymph, urine, cerebral fluid.
14. A method for imaging tumour tissue that is susceptible to enhanced survival in response to EPO treatment, comprising administering an anti-EPH-B4 and/or anti-Ephrin A1 antibody or EPH-B4 and/or Ephrin A1 binding peptide linked to a radio-ligand or other imaging agent, and measuring for tissue distribution and location of the radio-ligand or other imaging agent.
15. The method according to 14, wherein the anti-EPH-B4 and/or anti-Ephrin A1 antibody is a monoclonal or polyclonal antibody selected from the group of antibodies listed in Table 6.
16. A method for designing a compound which interferes with NEPOR's survival promoting activity, comprising:
   (a) providing the molecular makeup of the NEPOR species and providing amino acid sequences of a component NEPOR polypeptides;
   (b) using software comprised by the digital computer to design a chemical compound/protein construct which is predicted to bind to NEPOR; and
   (c) optionally designing protein constructs which mimic NEPOR in its dimerised/multimerised state (e.g. Fc constructs).
17. A method for identifying compounds that modulate NEPOR's tissue protective signalling activity, comprising (a) contacting a test compound with the NEPOR receptor complex; (b) measuring the level of tissue protective activity initiated by NEPOR activation; (c) identifying a test compound which increases or decreases the level of tissue protective NEPOR complex activity; (d) assaying the identified therapeutics for tissue protective activity mediated via NEPOR; and (e) assaying the identified therapeutics for NEPOR inhibitory activity.
18. The method of 17 wherein the tissue protective NEPOR receptor complex activity is measured by measuring the binding of the test compound to the NEPOR receptor complex.
19. The method of 18, wherein the test compound is labelled and binding of the labelled test compound to the tissue protective NEPOR receptor complex is measured by detecting the label attached to the test compound.
20. The method of 18 wherein the tissue protective NEPOR receptor complex activity is measured by measuring the binding of the test compound to the tissue protective NEPOR receptor complex.
21. A method for identifying compounds that modulate NEPOR's tissue protective signalling activity, comprising
   (a) contacting a test compound with the NEPOR receptor complex expressing cell;
   (b) measuring the level of tissue protective activity initiated by NEPOR activation in the cell;
   (c) identifying a test compound which increases or decreases the level of tissue protective NEPOR complex activity in a cell;
   (d) assaying the identified compounds for tissue protective activity mediated via NEPOR; and
   (e) assaying the identified therapeutics for NEPOR inhibitory activity.
22. The method of 21, wherein assay in step (d) is a tissue protective NEPOR receptor complex activity is measured by a cell proliferation/differentiation assay.
23. The method of 22, wherein the cells in the cell proliferentiation/differentiation assay are recombinantly engineered to express EPH-B4, and/or EPOR, and/or Ephrin A1.
24. The method of 23, wherein the cells endogenously expresses an EPO receptor and are transformed with a nucleic acid comprising a nucleotide sequence that
   (i) is operably linked to a promoter, and
   (ii) encodes either EPH-B4 and/or Ephrin A1.
25. The method of 23, wherein the cells endogenously expresses EPH-B4 and/or Ephrin A1 and are transformed with a nucleic acid comprising a nucleotide sequence that (i) is operably linked to a promoter, and (ii) encodes an EPO receptor polypeptide.
26. A method for identifying a compound that modulates the interaction between a tissue protective NEPOR receptor complex and a tissue protective NEPOR receptor complex ligand, comprising:
   (a) contacting a tissue protective NEPOR receptor complex with one or more test compounds; and
   (b) measuring the tissue protective NEPOR receptor complex activity, whereby if the activity measured in (b) differs from the tissue protective NEPOR receptor complex activity in the absence of the one or more test compounds, then a compound that modulates the interaction between the tissue protective NEPOR receptor complex and the tissue protective NEPOR receptor complex ligand is identified.
27. The method of 17, 21 or 26, wherein the tissue protective NEPOR receptor complex activity is measured by cell proliferation or cell differentiation.
28. The method of 17, 21 or 26, wherein the tissue protective NEPOR receptor complex activity measured is the ability of the tissue protective NEPOR receptor complex to interact with a tissue protective NEPOR receptor complex ligand.
29. The method of 17, 21 or 26, wherein the step of assaying the identified compound for tissue protective activity comprises detecting the presence of nucleolin in the cell.
30. The method of 17, 21 or 26, wherein the step of assaying the identified compound for tissue protective activity comprises detecting or measuring an increased level of activity of neuroglobin or cytoglobin in a cell.
31. The method of 17, 21 or 26 wherein the tissue protective NEPOR receptor complex is in solution.
32. The method of claims 17, 21 or 26, wherein the tissue protective NEPOR receptor complex is in a cell.
33. The method of 17, 21 or 26, wherein the compound inhibits the binding of a tissue protective NEPOR receptor complex ligand to a tissue protective NEPOR receptor complex.
34. The method of 17, 21 or 26, wherein the compound enhances the binding of a tissue protective NEPOR receptor complex ligand to a tissue protective NEPOR receptor complex.
35. The method of 17, 21 or 26, wherein the tissue protective NEPOR receptor complex contacted in step (a) is on a cell surface.
36. The method of 17, 21 or 26, wherein the tissue protective NEPOR receptor complex is on an isolated cell membrane.
37. The method of 17, 21 or 26, wherein the tissue protective NEPOR receptor complex activity is compared to EPOR receptor activation to identify NEPOR specific compounds.
38. The method of 17, 21 or 26, wherein the tissue protective NEPOR receptor complex is immobilized to a solid surface.
39. The method of 38, wherein the solid surface is a microtiter dish.
40. The method of 38, wherein the solid surface is a chip.
41. A method for identifying a compound that binds a tissue protective NEPOR receptor complex, comprising:
   (a) contacting a test compound with a ligand-binding tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support; and
   (b) contacting a test compound with a ligand-binding EPOR receptor complex fragment comprising at least two EPO receptor extracellular domains fused to an Fc fragment attached to a solid support
   (c) removing unbound test compounds from the solid supports;
   (d) identifying the compound attached to the tissue protective NEPOR receptor complex fragment, but not the EPOR receptor complex (and vice versa), whereby a compound bound to the solid support is identified as a compound that binds specifically to a tissue protective NEPOR receptor complex or a compound that binds specifically to an EPOR receptor complex.
42. A method for identifying a compound that binds a tissue protective NEPOR receptor complex, comprising:
   (a) contacting a test compound with a ligand-binding tissue protective NEPOR receptor complex fragment comprising at least one EPO receptor or EPH-B4 receptor or Ephrin A1 receptor, extracellular domain fused to an Fc fragment attached to a solid support;
   (b) removing unbound test compounds from the solid supports;
   (c) identifying the compound attached to the tissue protective NEPOR receptor complex fragment, whereby a compound bound to the solid support is identified as a compound that binds specifically to a tissue protective NEPOR receptor complex.
43. A method for identifying a compound that binds to a tissue protective NEPOR receptor complex, comprising:
   (a) contacting a tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support with
      (i) a tissue protective NEPOR receptor complex ligand attached to a first label and
      (ii) an equivalent amount of a test compound attached to a second label under conditions conducive to binding;
   (b) removing unbound material from the tissue protective NEPOR receptor complex; and
   (c) detecting the level of the first and second labels wherein if the second label is present the compound binds the complex and if the level of the first label decreases relative to the level of the first label where the labelled ligand is contacted with a tissue protective NEPOR receptor complex under conditions conducive to binding in the absence of a test compound after removal of unbound material, then a compound that binds to a tissue protective NEPOR receptor complex is identified.
44. A method for identifying a compound that modulates the binding of a tissue protective NEPOR receptor complex ligand to a tissue protective NEPOR receptor complex, comprising:
   (a) contacting a tissue protective NEPOR receptor complex ligand with a tissue protective NEPOR receptor complex fragment comprising at least one EPO receptor or EPH-B4 receptor or Ephrin A1 receptor extracellular domain and at least one EPO receptor or EPH-B4 receptor or Ephrin A1 receptor, extracellular domain fused to an Fc fragment attached to a solid support; in the presence of one or more test compounds under conditions conducive to binding; and
   (b) measuring the amount of tissue protective NEPOR receptor complex ligated bound to the tissue protective NEPOR receptor complex; whereby if the amount of bound tissue protective NEPOR receptor complex ligand measured in (b) differs from the amount of bound tissue protective NEPOR receptor complex ligand measured in the absence of the one or more test compounds, then a compound that modulates the binding of a tissue protective NEPOR receptor complex ligand to the tissue protective NEPOR receptor complex is identified.
45. The method of 26 or 44, wherein the amount of bound tissue protective NEPOR receptor complex ligand is measured using a tissue protective NEPOR receptor complex ligand-specific antibody.
46. The method of 26 or 44 wherein the tissue protective NEPOR receptor complex ligand is labelled and binding of the tissue protective NEPOR receptor complex ligand to the tissue protective NEPOR receptor complex is measured by detecting the label attached to the tissue protective NEPOR receptor complex ligand.
47. The method of 26 or 44 wherein the tissue protective NEPOR receptor complex ligand is labelled and binding of the labelled ligand to the tissue protective NEPOR receptor complex is measured by detecting the label attached to the tissue protective NEPOR receptor complex ligand.
48. The method of 46, wherein the label is fluorescent.
49. The method of 16, 17, 21, 26, or 41 to 46 wherein the test compound is an antibody specific for the tissue protective NEPOR receptor complex.
50. The method of 16, 17, 21, 26, or 41 to 46 wherein the test compound is a small molecule.
51. The method of 16, 17, 21, 26, or 41 to 46 wherein the test compound is a peptide.
52. The method of 16, 17, 21, 26, or 41 to 46 wherein the test compound is a member of a library.
53. The method of 26 or 44, wherein the tissue protective NEPOR receptor complex ligand is EPO, or derivatives thereof.
54. The method of 16, 17, 21, 41, 42, or 43 wherein the compound binds the tissue protective NEPOR receptor complex.
55. The method of 26 or 44, wherein the compound binds the tissue protective NEPOR receptor complex ligand.
56. The method of 16, 17, 21, 26, or 41 to 46 wherein the tissue protective NEPOR receptor complex activity is compared to EPOR receptor activation to identify NEPOR specific compounds.
57. A method for identifying a compound that modulates a tissue protective activity in a mammal, comprising:
   (a) administering the compound to a first animal immediately following infliction of an injury, wherein the first animal endogenously expresses a tissue protective NEPOR receptor complex; and
   (b) administering the compound to a second animal immediately following infliction of the same injury as in step (a), wherein the second animal is deficient in expression of a tissue protective NEPOR receptor complex or components thereof; such that if recovery from the injury differs in the animal of step (a) as compared to the animal of step (b), a compound that modulates a tissue protective activity is identified.
58. A method for treating the negative patient outcomes associated with EPO stimulated NEPOR function, involving the co-administration of EPO with an inhibitor of NEPOR activity.
59. The method for treating EPO-induced tumour cell survival in NEPOR positive tumour tissue of 58, comprising administering an effective amount of anti-NEPOR antibody from claim 1, in combination with EPO, whereby such combinations permits haematopoietic signalling whilst switching off NEPOR signalling and thus EPO mediated cell survival signals on tumour cells.
60. The method for treating EPO-induced tumour cell survival in NEPOR positive tumour tissue of 58, further comprising administering an effective amount of EPHB4 tyrosine kinase inhibitor in combination with EPO, whereby such combinations permits haematopoietic signalling whilst switching off NEPOR signalling and thus EPO mediated cell survival signals on tumour cells.
61. The method for treating EPO-induced tumour cell survival in NEPOR positive tumour tissue of 58, further comprising administering an effective amount of anti-NEPOR siRNA's, in combination with EPO, whereby such combinations permits haematopoietic signalling whilst switching off NEPOR signalling and thus EPO mediated cell survival signals on tumour cells.
62. A method for decreasing the survival of tumour cells or tissues in a human comprising administering a therapeutically effective amount of a compound that modulates the activity of a tissue protective NEPOR receptor complex to a human in need thereof, wherein said decreased survival of cancer cells/tissues results in the decrease of tumour growth and/or an increase in patient survival, with the proviso that the compound is an EPO derivative and not a wild-type EPO.
63. The method of 58, 59, 60, 61, 62 wherein the compound is identified by the method of any one of claims 16, 17, 21, 26, or 41 to 46.
64. A method for modulating cell survival in NEPOR positive tissue comprising administering an EPO mutants and peptides selected from the group consisting of peptides from each of SEQ ID NO. 17 through SEQ ID NO. 212.
65. A method for modulating cell survival in NEPOR positive tissue comprising administering an effective amount of an EPO chimera's, comprising an ephrin receptor ligand binding domain selected from the group consisting of SEQ ID NO.215, and SEQ ID NO.216.
66. The method of 58, 59, 60, 61, 62 wherein the compound is an antibody specific for the tissue protective NEPOR receptor complex.
67. The method of 58, 59, 60, 61, 62 wherein the compound is an antibody is specific for a tissue protective NEPOR receptor complex ligand.
68. The method of 58, 59, 60, 61, 62 wherein the compound is a small molecule, peptide, or a member of a library.
69. The method of 58, 59, 60, 61, 62 wherein the compound binds to the tissue protective NEPOR receptor complex.
70. The method of 58, 59, 60, 61, 62 wherein the compound decreases the activity of the tissue protective NEPOR receptor complex.
71. The method of 58, 59, 60, 61, 62 wherein the compound is administered in conjunction with an EPO.
72. The method of 58, 59, 60, 61, 62 wherein the disease or disorder is a cancer including, head and neck cancer, breast cancer, liver cancer, colorectal cancer, small intestine cancer, leukemia, prostate cancer, lung cancer, ovarian cancer, pancreatic cancer, endometrial cancer, stomach cancer, non-Hodgkin lymphoma, kidney cancer, Renal cell carcinoma (RCC), malignant melanoma, gallbladder cancer, bladder cancer, vulvar cancer, Penile cancer, testicular cancer, thymus cancer, Kaposi's sarcoma, eye cancer, adrenal gland cancer, brain cancer, cervical cancer, appendix cancer, adenoid cancer, bile duct cancer, urethral cancer, spinal cancer, Ewing's family of tumors, extragonal germ cell cancer, extra hepatic bile duct cancer, fallopian tube cancer, soft tissue cancers, bone cancer, Hodgkin's lymphoma, anal cancer, malignant mesothelioma, vaginal cancer skin cancer, central nervous system cancer (craniopharyngioma), pleuropulmonary blastoma, nasal cavity and paranasal sinus cancer transitional cell cancer of renal pelvis and ureter, pituitary gland cancer, sqamous cell carcinoma of the head and neck (HNSCC), prostate cancer, colorectal cancer, lung cancer, brain cancer, bladder cancer, and salivary gland cancer.
73. The method of 58, 59, 60, 61, 62 wherein the cancer comprises cancer cells expressing the tissue protective NEPOR receptor complex.
74. The method of 58, 59, 60, 61, 62, wherein the cancer is metastatic cancer.
75. The method of 58, 59, 60, 61, 62 wherein the cancer is an angiogenesis-dependent cancer.
76. A method for treating a patient suffering from an angiogenesis-associated disease, comprising administering to the patient a compound identified by the method of any one of 16, 17, 21, 26, or 41 to 46.
77. siRNA which is specific for EPH-B4 for use in treating a cancer and/or tumor patient that is receiving or will receive Erythropoietin.

### Further Embodiments

1. A method of determining whether a cancer patient is suitable for erythropoietin (EPO) therapy, comprising
   (A) determining the level of expression of EPH-B4 in a tissue sample isolated from said patient; and
   (B) correlating a presence of EPH-B4 expression to a negative physiological response to EPO therapy.
2. The method of item 1, wherein the level of expression is determined by a technique selected from the group consisting of immunoassay, chemiluminescent assay, nephelometric assay, turbidimetric assay, bioassay and reporter-assay.
3. The method of item 1, wherein the level of expression is determined by an immunoassay selected from the group consisting of enzyme-linked immunosorbent assay (ELISA), immunoprecipitation, enzyme immunoassay (EIA), radioimmunoassay (RIA), fluorescent immunoassay, Western blot, competitive immunoassay, noncompetitive immunoassay, homogeneous immunoassay and heterogeneous immunoassay.
4. The method of item 1, wherein the level of expression is determined by ELISA.
5. The method of item 1, wherein said level of expression is determined by immunohistochemistry.
6. The method of item 1, wherein the level of expression is determined by measuring the amount of EPH-B4 mRNA in said sample, in particular wherein the level of expression is determined by a technique selected from the group consisting of PCR, QPCR, R-PCR, gene expression microarray analysis, northern-blot analysis, reverse transcription and amplification, zymography, ligase-chain-reaction, NASBA, RNase Protection Assay (RPA), capillary electrophoresis with laser induced fluorescence (CE-LIF), and RT-PCR.
7. The method of item 6, wherein the level of expression is determined by reverse transcription and amplification.
8. The method of item 1, wherein the tissue sample is selected from cancerous tissue or circulating cells derived from same, or wherein the tissue sample is selected from blood, lymph, urine or cerebral fluid.
9. The method of item 1, further comprising determining the level of expression in said sample of at least one of Ephrin A1 or EPOR.
10. The method of item 1, wherein said negative physiological response includes increased tumor progression, poorer patient survival or enhanced tumor cell survival.
11. A diagnostic comprising at least one biomarker specific for EPH-B4 and/or Ephrin A1 for use in a method of determining whether a cancer patient is suitable for EPO therapy.
12. The diagnostic of item 11, wherein said biomarker is a probe, primer, or antibody.

## Claims

1. A method of assessing a tissue sample from a cancer patient for expression of EPH-B4, comprising determining the level of expression of EPH-B4 in said tissue sample isolated from said patient; wherein the presence of a higher level of EPH-B4 expression is indicative of increased tumor progression and / or poorer patient survival upon treatment with erythropoietin.

2. The method of claim 1, wherein the level of expression is determined by a technique selected from the group consisting of immunoassay, chemiluminescent assay, nephelometric assay, turbidimetric assay, bioassay and reporter-assay.

3. The method of claim 1, wherein the level of expression is determined by an immunoassay selected from the group consisting of enzyme-linked immunosorbent assay (ELISA), immunoprecipitation, enzyme immunoassay (EIA), radioimmunoassay (RIA), fluorescent immunoassay, Western blot, competitive immunoassay, noncompetitive immunoassay, homogeneous immunoassay and heterogeneous immunoassay.

4. The method of claim 1, wherein the level of expression is determined by ELISA.

5. The method of claim 1, wherein said level of expression is determined by immunohistochemistry.

6. The method of claim 1, wherein the level of expression is determined by measuring the amount of EPH-B4 mRNA in said sample, in particular wherein the level of expression is determined by a technique selected from the group consisting of PCR, QPCR, R-PCR, gene expression microarray analysis, northern-blot analysis, reverse transcription and amplification, zymography, ligase-chain-reaction, NASBA, RNase Protection Assay (RPA), capillary electrophoresis with laser induced fluorescence (CE-LIF), and RT-PCR.

7. The method of claim 6, wherein the level of expression is determined by reverse transcription and amplification.

8. The method of any of claims 1 to 7, wherein the tissue sample is selected from cancerous tissue or circulating cells derived from same, or wherein the tissue sample is selected from blood, lymph, urine or cerebral fluid.

9. The method of any of claims 1 to 8, further comprising determining the level of expression in said sample of at least one of Ephrin A1 or EPOR.

10. The method of any of claims 1 to 9, wherein the cancer is selected from the group consisting of head and neck cancer, breast cancer, liver cancer, colorectal cancer, small intestine cancer, leukemia, prostate cancer, lung cancer, ovarian cancer, pancreatic cancer, endometrial cancer, stomach cancer, non-Hodgkin lymphoma, kidney cancer, Renal cell carcinoma (RCC), malignant melanoma, gallbladder cancer, bladder cancer, vulvar cancer, Penile cancer, testicular cancer, thymus cancer, Kaposi's sarcoma, eye cancer, adrenal gland cancer, brain cancer, cervical cancer, appendix cancer, adenoid cancer, bile duct cancer, urethral cancer, spinal cancer, Ewing's family of tumors, extragonal germ cell cancer, extra hepatic bile duct cancer, fallopian tube cancer, soft tissue cancers, bone cancer, Hodgkin's lymphoma, anal cancer, malignant mesothelioma, vaginal cancer skin cancer, central nervous system cancer (craniopharyngioma), pleuropulmonary blastoma, nasal cavity and paranasal sinus cancer transitional cell cancer of renal pelvis and ureter, pituitary gland cancer, sqamous cell carcinoma of the head and neck (HNSCC), prostate cancer, colorectal cancer, lung cancer, brain cancer, bladder cancer, and salivary gland cancer. It is particularly preferred that the cancer is selected from the group of squamous cell carcinoma of the head and neck (HNSCC), prostate cancer, colorectal cancer, lung cancer, kidney cancer, brain cancer, bladder cancer and breast cancer.

11. Use of a diagnostic test for determining whether a cancer patient bears the risk of increased tumor progression and / or poorer patient survival upon treatment with erythropoietin, wherein said diagnostic test includes means for determining the expression of EPH-B4.

12. The use of claim 11, wherein said means are suitable for performing a technique as defined in any of claims 2-7.

## Patentansprüche

1. Verfahren zum Bewerten einer Gewebeprobe von einem Krebs-Patienten auf Expression von EPH-B4, umfassend Bestimmen des Expressionslevels von EPH-B4 in der Gewebeprobe, isoliert von dem Patienten;
wobei das Vorhandensein eines höheren Levels von EPH-B4-Expression indikativ für erhöhte Tumorprogression und / oder schlechteres Patientenüberleben bei Behandlung mit Erythropoietin ist.

2. Verfahren nach Anspruch 1, wobei der Expressionslevel mittels einer Technik bestimmt wird, die ausgewählt ist aus der Gruppe, bestehend aus Immunoassay, Chemilumineszenz-Assay, nephelometrischem Assay, turbidimetrischem Assay, Bioassay und Reporter-Assay.

3. Verfahren nach Anspruch 1, wobei der Expressionslevel mittels eines Immunoassays bestimmt wird, der ausgewählt ist aus der Gruppe, bestehend aus enzymgekoppelter Immunadsorbtionsbestimmung (ELISA), Immunopräzipitation, Enzym-Immunoassay (EIA), Radioimmunoassay (RIA), Fluoreszenz-Immunoassay, Western-Blot, kompetitivem Immunoassay, nicht-kompetitivem Immunoassay, homogenem Immunoassay und heterogenem Immunoassay.

4. Verfahren nach Anspruch 1, wobei der Expressionslevel mittels ELISA bestimmt wird.

5. Verfahren nach Anspruch 1, wobei der Expressionslevel mittels Immunhistochemie bestimmt wird.

6. Verfahren nach Anspruch 1, wobei der Expressionslevel mittels Messen der Menge an EPH-B4-mRNA in der Probe bestimmt wird, insbesondere wobei der Expressionslevel mittels einer Technik bestimmt wird, die ausgewählt wird aus der Gruppe, bestehend aus PCR, QPCR, R-PCR, Microarray-Genexpressionsanalyse, Northern-Blot-Analyse, reverser Transkription und Amplifikation, Zymographie, Ligase-Kettenreaktion, NASBA, RNase-Schutz-Assay (RPA), Kapillarelektrophorese mit Laser-induzierter Fluoreszenz (CE-LIF) und RT-PCR.

7. Verfahren nach Anspruch 6, wobei der Expressionslevel mittels reverser Transkription und Amplifikation bestimmt wird.

8. Verfahren nach einem beliebigen der Ansprüche 1 bis 7, wobei die Gewebeprobe ausgewählt ist aus Krebsgewebe oder davon abgeleiteten zirkulierenden Zellen, oder wobei die Gewebeprobe ausgewählt ist aus Blut, Lymphe, Urin oder Zerebralflüssigkeit.

9. Verfahren nach einem beliebigen der Ansprüche 1 bis 8, weiterhin umfassend Bestimmen des Expressionslevels in der Probe von mindestens einem von Ephrin A1 oder EPOR.

10. Verfahren nach einem beliebigen der Ansprüche 1 bis 9, wobei der Krebs ausgewählt ist aus Kopf- und Halskrebs, Brustkrebs, Leberkrebs, kolorektalem Krebs, Dünndarmkrebs, Leukämie, Prostatakrebs, Lungenkrebs, Eierstockkrebs, Bauchspeicheidrüsenkrebs, endometrischem Krebs, Magenkrebs, Nicht-Hodgkin-Lymphom, Nierenkrebs, Nierenzellkarzinom (RCC), malignem Melanom, Gallenblasenkrebs, Blasenkrebs, Vulvakrebs, Peniskrebs, Hodenkrebs, Thymuskrebs, Kaposi-Sarkom, Augenkrebs, Nebennierenkrebs, Gehirnkrebs, Gebärmutterhalskrebs, Appendixkrebs, Drüsenkrebs, Gallengangkrebs, Harnröhrenkrebs, Spinalkrebs, der Ewing'schen Familie von Tumoren, extragonadalem Keimzellenkrebs, extrahepatischem Gallengangkrebs, Eileiterkrebs, Weichgewebekrebs, Knochenkrebs, Hodgkin-Lymphom, Analkrebs, malignem Mesotheliom, Vaginalkrebs Hautkrebs, Zentralnervensystemkrebs (Craniopharyngiom), pleuropulmonalem Blastom, Nasenhöhlen- und Nasennebenhöhlenkrebs Übergangszellkarzinom des Nierenbeckens und des Harnleiters, Hypophysenkrebs, Plattenepithelkrebs von Kopf und Nacken (HNSCC), Prostatakrebs, kolorektalem Krebs, Lungenkrebs, Gehirnkrebs, Blasenkrebs und Speicheldrüsenkrebs. Es ist insbesondere bevorzugt, dass der Krebs ausgewählt ist aus der Gruppe, bestehend aus Plattenepithelkrebs von Hals und Nacken (HNSCC), Prostatakrebs, kolorektalem Krebs, Lungenkrebs, Nierenkrebs, Gehirnkrebs, Blasenkrebs und Brustkrebs.

11. Verwendung eines diagnostischen Tests zum Bestimmen, ob ein Krebspatient das Risiko erhöhter Tumorprogression und / oder schlechterem Patientenüberleben bei Behandlung mit Erythropoietin trägt, wobei der diagnostische Test Mittel zum Bestimmen der Expression von EPH-B4 beinhaltet.

12. Verwendung nach Anspruch 11, wobei die Mittel geeignet sind, eine wie in einem beliebigen der Ansprüche 2-7 definierte Technik durchzuführen.

## Revendications

1. Une méthode d'évaluation d'un échantillon tissulaire provenant d'un patient cancéreux quant à l'expression de EPH-B4, comprenant déterminer le niveau d'expression de EPH-B4 dans ledit échantillon tissulaire isolé dudit patient ; dans laquelle la présence d'un niveau supérieur d'expression de EPH-B4 est indicatif d'une progression tumorale accrue et/ou d'une survie plus faible du patient lors d'un traitement à l'érythropoïétine.

2. La méthode selon la revendication **1,** dans laquelle le niveau d'expression est déterminé par une technique sélectionnée dans le groupe consistant en un test immunologique, un test de chimiluminescence, un test néphélométrique, un test turbidimétrique, un test biologique et un test utilisant un rapporteur.

3. La méthode selon la revendication **1,** dans laquelle le niveau d'expression est déterminé par un test immunologique sélectionné dans le groupe consistant en un dosage d'immunoabsorption par enzyme liée (ELISA), une immunoprécipitation, un dosage immuno-enzymatique (EIA), un dosage radio-immunologique (RIA), un test par immunofluorescence, un western blot, un test immunologique compétitif, un test immunologique non compétitif, un test immunologique homogène et un test immunologique hétérogène.

4. La méthode selon la revendication **1,** dans laquelle le niveau d'expression est déterminé par ELISA.

5. La méthode selon la revendication **1,** dans laquelle le niveau d'expression est déterminé par immunohistochimie.

6. La méthode selon la revendication **1,** dans laquelle le niveau d'expression est déterminé en mesurant la quantité d'ARNm de EPH-B4 dans ledit échantillon, en particulier dans laquelle le niveau d'expression est déterminé par une technique sélectionnée dans le groupe consistant en une PCR, une QPCR, une R-PCR, une analyse de l'expression génique par micropuce, une analyse par northern blot, une transcription inverse et amplification, une zymographie, une réaction de ligature en chaîne, une NASBA, une test de protection à la RNase (RPA), une électrophorèse capillaire avec fluorescence induite par laser (CE-LIF), et une RT-PCR.

7. La méthode selon la revendication **6,** dans laquelle le niveau d'expression est déterminé par transcription inverse et amplification.

8. La méthode selon l'une quelconque des revendications **1** à **7,** dans laquelle l'échantillon tissulaire est sélectionné parmi du tissu cancéreux ou des cellules circulantes dérivées de ce dernier, ou dans laquelle l'échantillon tissulaire est sélectionné parmi le sang, la lymphe, l'urine ou le liquide céphalo-rachidien.

9. La méthode selon l'une quelconque des revendications **1** à **8**, comprenant en outre de déterminer le niveau d'expression dans ledit échantillon d'au moins l'un de l'éphrine A1 ou l'EPOR.

10. La méthode selon l'une quelconque des revendications **1** à **9**, dans laquelle le cancer est sélectionné dans le groupe consistant en un cancer de la tête et du cou, un cancer du sein, un cancer du foie, un cancer colorectal, un cancer de l'intestin grêle, une leucémie, un cancer de la prostate, un cancer du poumon, un cancer ovarien, un cancer pancréatique, un cancer endométrial, un cancer de l'estomac, un lymphome non-hodgkinien, un cancer du rein, un carcinome à cellules rénales (CCR), un mélanome malin, un cancer de la vésicule biliaire, un cancer de la vessie, un cancer vulvaire, un cancer pénien, un cancer testiculaire, un cancer du thymus, un sarcome de Kaposi, un cancer de l'oeil, un cancer de la glande surrénale, un cancer du cerveau, un cancer du col de l'utérus, un cancer de l'appendice, un cancer adénoïde, un cancer des canaux biliaires, un cancer urétral, un cancer de la moelle épinière, la famille des tumeurs d'Ewing, un cancer d'une lignée germinale extragonadique, un cancer des canaux biliaires extra-hépatiques, un cancer des trompes de Fallope, des cancers des tissus mous, un cancer des os, un lymphome de Hodgkin, un cancer anal, un mésothéliome malin, un cancer vaginal, un cancer de la peau, un cancer du système nerveux central (craniopharyngiome), un blastome pleuropulmonaire, un cancer des cavités nasales et des sinus paranasaux, un cancer des cellules transitionnelles du bassinet et de l'uretère, un cancer de l'hypophyse, un carcinome à cellules squameuses de la tête et du cou (CCSTC), un cancer de la prostate, un cancer colorectal, un cancer du poumon, un cancer du cerveau, un cancer de la vessie, et un cancer des glandes salivaires. Il est particulièrement préféré que le cancer soit sélectionné dans le groupe d'un carcinome à cellules squameuses de la tête et du cou (CCSTC), un cancer de la prostate, un cancer colorectal, un cancer du poumon, un cancer du rein, un cancer du cerveau, un cancer de la vessie et un cancer du sein.

11. Utilisation d'un test diagnostic pour déterminer si un patient porte le risque d'une progression tumorale accrue et/ou d'une survie plus faible du patient lors d'un traitement à l'érythropoïétine, dans laquelle ledit test diagnostic comprend des moyens pour déterminer l'expression de EPH-B4.

12. L'utilisation selon la revendication **11,** dans laquelle lesdits moyens sont appropriés pour mettre en oeuvre une technique telle que définie dans l'une quelconque des revendications **2** à **7.**
